# EUROPEAN PATENT SPECIFICATION

(11) **EP 1 499 316 B1**
(45) Date of publication and mention of the grant of the patent: **19.03.2008**
(21) Application number: 03719766.2
(22) Date of filing: 16.04.2003
(51) Int. Cl.: A61K 31/4545, C07D 471/04, C07D 401/14, A61K 31/4184, A61P 37/08

(54) **(1-4-PIPERIDINYL)BENZIMIDAZOLE DERIVATIVES USEFUL AS HISTAMINE H3 ANTAGONISTS**
1-(4-PIPERIDINYL)BENZIMIDAZOLE ALS HISTAMIN H3 ANTAGONISTEN
DERIVES DE (1-4-PIPERIDINYL)BENZIMIDAZOLE SERVANT D'ANTAGONISTES DES RECEPTEURS H3 DE L'HISTAMINE

(30) Priority: 18.04.2002 US 373731 P
(43) Date of publication of application: 26.01.2005
(73) Proprietor: Schering Corporation, Kenilworth, New Jersey 07033-0530 (US)
(72) Inventor: ZENG, Qingbei, Edison, NJ 08820 (US); ASLANIAN, Robert, G., Rockaway, NJ 07866 (US); BERLIN, Michael, Y., Flemington, NJ 08822 (US); BOYCE, Christopher, W., Flemington, NJ 08822 (US); CAO, Jianhua, Edison, NJ 08820 (US); KOZLOWSKI, Joseph, A., Princeton, NJ 08543 (US); MANGIARACINA, Pietro, Monsey, NY 10952 (US); MC CORMICK, Kevin, D., Basking Ridge, NJ 07920 (US); MUTAHI, Mwangi, W., Nyeri (KE); ROSENBLUM, Stuart, B., West Orange, NJ 07052 (US); SHIH, Neng-Yang, North Caldwell, NJ 07006 (US); SOLOMON, Daniel, M., Edison, NJ 08817 (US); TOM, Wing, C., Cedar Grove, NJ 07009 (US)
(74) Representative: Adams, Harvey Vaughan John
(86) International application number: PCT/US2003/011672
(87) International publication number: WO 2003/088967

(56) References cited:
- EP-A- 0 580 541
- EP-A- 0 626 373
- WO-A-02/24659
- US-B1- 6 211 199
- JANSSENS F ET AL: "NEW ANTIHISTAMINIC N-HETEROCYCLIC 4-PIPERIDINAMINES. 3. SYNTHESIS AND ANTIHISTAMINIC ACTIVITY OF N-(4-PIPERIDINYL)-3H-IMIDAZO4,5-BPYRIDIN-2 -AMINES" JOURNAL OF MEDICINAL CHEMISTRY, AMERICAN CHEMICAL SOCIETY. WASHINGTON, US, vol. 28, no. 12, 1985, pages 1943-1947, XP001084054 ISSN: 0022-2623

## Description

### FIELD OF THE INVENTION

The present invention relates to novel substituted benzimidazoles and aza- and diaza-derivatives thereof useful as histamine H₃ antagonists. The invention also relates to pharmaceutical compositions comprising said compounds and their use in treating inflammatory diseases, allergic conditions and central nervous system disorders. The invention also relates to the use of a combination of novel histamine H₃ antagonists of this invention with histamine H₁ compounds for the treatment of inflammatory diseases and allergic conditions, as well as pharmaceutical compositions comprising a combination of one or more novel histamine H₃ antagonist compounds of the invention with one or more histamine H₁ compounds.

### BACKGROUND OF THE INVENTION

The histamine receptors, H₁, H₂ and H₃ are well-identified forms. The H₁ receptors are those that mediate the response antagonized by conventional antihistamines. H₁ receptors are present, for example, in the ileum, the skin, and the bronchial smooth muscle of humans and other mammals. Through H₂ receptor-mediated responses, histamine stimulates gastric acid secretion in mammals and the chronotropic effect in isolated mammalian atria.

H₃ receptor sites are found on sympathetic nerves, where they modulate sympathetic neurotransmission and attenuate a variety of end organ responses under control of the sympathetic nervous system. Specifically, H₃ receptor activation by histamine attenuates norepinephrine outflow to resistance and capacitance vessels, causing vasodilation.

Imidazole H₃ receptor antagonists are well known in the art. More recently, non-imidazole H₃ receptor antagonists have been disclosed in WO 02/32893 and US Provisional Application 60/275,417, filed March 13, 2001.

US 6 211 199 relates to substituted 1-(1H-benzimidazol-2-yl-amino) piperidines useful for the treatment of allergic diseases. EP 0 580 541 relates to piperidine derivatives of benzimidazole as anti-histaminic and anti-allergic agents. Janssens et al (Journal of Medicinal Chemistry, vol. 28, no. 12, 1985, pp. 1943-1947) relates to anti-histaminic N-heterocyclic 4-piperidinamines.

US 5,869,479 discloses compositions for the treatment of the symptoms of allergic rhinitis using a combination of at least one histamine H₁ receptor antagonist and at least one histamine H₃ receptor antagonist. WO 02/24659 relates to substituted imidazoles as dual histamine H1 and H3 agonists or antagonists.

### SUMMARY OF THE INVENTION

Disclosed herein are compounds of formula I: 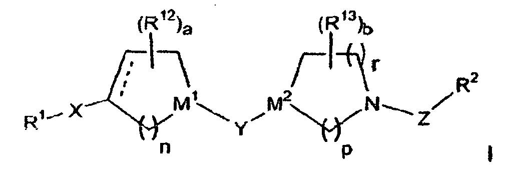 or a pharmaceutically acceptable salt or solvate thereof, wherein:
the dotted line represents an optional double bond;
a is 0 to 2;
b is 0 to 2;
n is 1, 2 or 3;
p is 1, 2 or 3;
r is 0, 1, 2, or 3;
with the provisos that when M² is N, p is not 1; and that when r is O, M² is C(R³); and that the sum of p and r is 1 to 4;
M¹ is C(R³) or N;
M² is C(R³) or N;
X is a bond or C₁-C₆ alkylene;
Y is -C(O)-, -C(S)-, -(CH₂)_{q}-, -NR⁴C(O)-, -C(O)NR⁴-, -C(O)CH₂-, -SO₂-, -N(R⁴)-, -NH-C(=N-CN)- or -C(=N-CN)-NH-; with the provisos that when M¹ is N, Y is not -NR⁴C(O)- or -NH-C(=N-CN)-; when M² is N, Y is not -C(O)NR⁴- or -C(=N-CN)-NH-; and when Y is -N(R⁴)-, M¹ is CH and M² is C(R³);
q is 1 to 5, provided that when both M¹ and M² are N, q is 2 to 5;
Z is a bond, C₁-C₆ alkylene, C₁-C₆ alkenylene, -C(O)-, -CH(CN)-, -SO₂- or -CH₂C(O)NR⁴-;
R¹ is 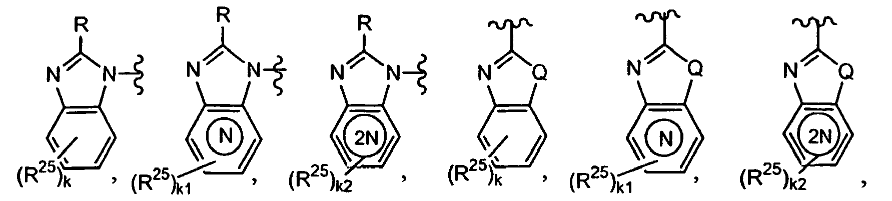 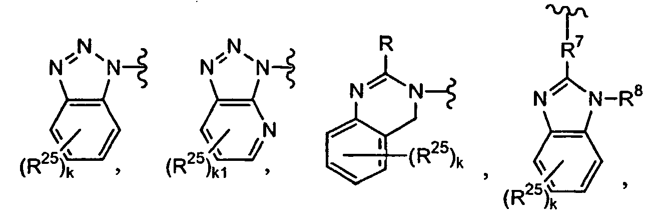 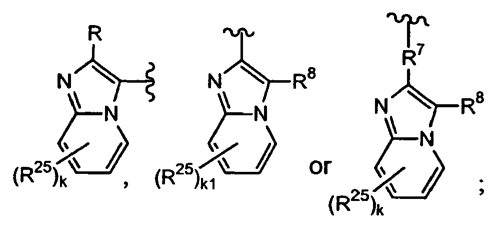
Q is -N(R⁸)-, -S- or -O-;
k is 0, 1, 2, 3 or 4;
k1 is 0, 1, 2 or 3;
k2 is 0, 1 or 2;
R is H, C₁-C₆ alkyl, halo(C₁-C₆)alkyl-, C₁-C₆ alkoxy, (C₁-C₆)alkoxy-(C₁-C₆)alkyl-, (C₁-C₆)-alkoxy-(C₁-C₆)alkoxy, (C₁-C₆)alkoxy-(C₁-C₆)alkyl-SO₀₋₂, R³²-aryl(C₁-C₆)alkoxy-, R³²-aryl(C₁-C₆)alkyl-, R³²-aryl, R³²-aryloxy, R³²-heteroaryl, (C₃-C₆)cycloalkyl, (C₃-C₆)cycloalkyl-(C₁-C₆)alkyl, (C₃-C₆)cycloalkyl-(C₁-C₆)alkoxy, (C₃-C₆)cycloalkyl-oxy-, R³⁷-heterocycloalkyl, R³⁷-heterocycloalkyl-oxy-, R³⁷-heterocycloalkyl-(C₁-C₆)alkoxy, N(R³⁰)(R³¹)-(C₁-C₆)alkyl-, -N(R³⁰)(R³¹), -NH-(C₁-C₆)alkyl-O-(C₁-C₆)alkyl, -NHC(O)NH(R²⁹); R²⁹-S(O)₀₋₂-, halo(C₁-C₆)alkyl-S(0)₀₋₂-, N(R³⁰)(R³¹)-(C₁-C₆)alkyl-S(O)₀₋₂- or benzoyl;
R⁸ is H, C₁-C₆ alkyl, halo(C₁-C₆)alkyl-, (C₁-C₆)alkoxy-(C₁-C₆)alkyl-, R³²-aryl(C₁-C₆)alkyl-, R³²-aryl, R³²-heteroaryl, (C₃-C₆)cycloalkyl, (C₃-C₆)cycloalkyl-(C₁-C₆)alkyl, R³⁷-heterocycloalkyl, N(R³⁰)(R³¹)-(C₁-C₆)alkyl-, R²⁹-S(O)₂-, halo(C₁-C₆)alkyl-S(O)₂-, R²⁹-S(O)₀₋₁-(C₂-C₆)alkyl-, halo(C₁-C₆)alkyl-S(O)₀₋₁-(C₂-C₆)alkyl-;
R² is a six-membered heteroaryl ring having 1 or 2 heteroatoms independently selected from N or N-O, with the remaining ring atoms being carbon; a five-membered heteroaryl ring having 1, 2, 3 or 4 heteroatoms independently selected from N, O or S, with the remaining ring atoms being carbon; R³²-quinolyl; R³²-aryl; heterocycloalkyl; (C₃-C₆)cycloalkyl; C₁-C₆ alkyl; hydrogen; thianaphthenyl; 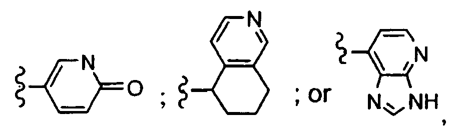 wherein said six-membered heteroaryl ring or said five-membered heteroaryl ring is optionally substituted by R⁶;
R³ is H, halogen, C₁-C₆ alkyl, -OH, (C₁-C₆)alkoxy or -NHSO₂-(C₁-C₆)alkyl;
R⁴ is independently selected from the group consisting of hydrogen, C₁-C₆ alkyl, C₃-C₆ cycloalkyl, (C₃-C₆)cycloalkyl(C₁-C₆)alkyl, R³³-aryl, R³³-aryl(C₁-C₆)alkyl, and R³²-heteroaryl;
R⁵ is hydrogen, C₁-C₆ alkyl, -C(O)R²⁰, -C(O)₂R²⁰, -C(O)N(R²⁰)₂, (C₁-C₆)alkyl-SO₂-, or (C₁-C₆)alkyl-SO₂-NH-;
or R⁴ and R⁵, together with the nitrogen to which they are attached, form an azetidinyl, pyrrolidinyl, piperidinyl, piperazinyl or morpholinyl ring;
R⁶ is 1 to 3 substituents independently selected from the group consisting of -OH, halogen, C₁-C₆ alkyl-, C₁-C₆ alkoxy, C₁-C₆ alkylthio, -CF₃, -NR⁴R⁵, -CH₂-NR⁴R⁵, -NHSO₂R²², -N(SO₂R²²)₂, phenyl, R³³-phenyl, NO₂, -CO₂R⁴, -CON(R⁴)₂, 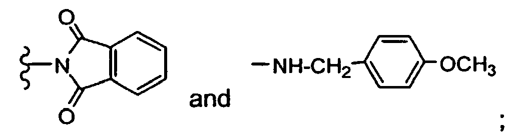
R⁷ is -N(R²⁹)-, -O or -S(O)₀₋₂-;
R¹² is independently selected from the group consisting of C₁-C₆ alkyl, hydroxyl, C₁-C₆ alkoxy, or fluoro, provided that when R¹² is hydroxy or fluoro, then R¹² is not bound to a carbon adjacent to a nitrogen; or two R¹² substituents form a C₁ to C₂ alkyl bridge from one ring carbon to another non-adjacent ring carbon; or R¹² is =O;
R¹³ is independently selected from the group consisting of C₁-C₆ alkyl, hydroxyl, C₁-C₆ alkoxy, or fluoro, provided that when R¹³ is hydroxy or fluoro then R¹³ is not bound to a carbon adjacent to a nitrogen; or two R¹³ substituents form a C₁ to C₂ alkyl bridge from one ring carbon to another non-adjacent ring carbon; or R¹³ is =O;
R²⁰ is independently selected from the group consisting of hydrogen, C₁-C₆ alkyl, or aryl, wherein said aryl group is optionally substituted with from 1 to 3 groups independently selected from halogen, -CF₃, -OCF₃, hydroxyl, or methoxy; or when two R²⁰ groups are present, said two R²⁰ groups taken together with the nitrogen to which they are bound can form a five or six membered heterocyclic ring;
R²² is C₁-C₆ alkyl, R³⁴-aryl or heterocycloalkyl;
R²⁴ is H, C₁-C₆ alkyl, -SO₂R²² or R³⁴-aryl;
R²⁵ is independently selected from the group consisting of C₁-C₆ alkyl, halogen, -CN, -NO₂, -CF₃, -OH, C₁-C₆ alkoxy, (C₁-C₆)alkyl-C(O)-, aryl-C(O)-, -C(O)OR²⁹, -N(R⁴)(R⁵), N(R⁴)(R⁵)-C(O)-, N(R⁴)(R⁵)-S(O)₁₋₂-, R²²-S(O)₀₋₂-, halo-(C₁-C₆)alkyl- or halo-(C₁-C₆)alkoxy-(C₁-C₆)alkyl-;
R²⁹ is H, C₁-C₆ alkyl, C₃-C₆ cycloalkyl, R³⁵-aryl or R³⁵-aryl(C₁-C₆)alkyl-;
R³⁰ is H, C₁-C₆ alkyl-, R³⁵-aryl or R³⁵-aryl(C₁-C₆)alkyl-;
R³¹ is H, C₁-C₆ alkyl-, R³⁵-aryl, R³⁵-aryl(C₁-C₆)alkyl-, R³⁵-heteroaryl, (C₁-C₆)alkyl-C(O)-, R³⁵-aryl-C(O)-, N(R⁴)(R⁵)-C(O)-, (C₁-C₆)alkyl-S(O)₂- or R³⁵-aryl-S(O)₂-;
or R³⁰ and R³¹ together are -(CH₂)₄-₅-, -(CH₂)₂-O-(CH₂)₂- or -(CH₂)₂-N(R³⁸)-(CH₂)₂- and form a ring with the nitrogen to which they are attached;
R³² is 1 to 3 substituents independently selected from the group consisting of H, -OH, halogen, C₁-C₆ alkyl, C₁-C₆ alkoxy, R³⁵-aryl-O-, -SR²², -CF₃, -OCF₃, -OCHF₂, -NR³⁹R⁴⁰, phenyl, R³³-phenyl, NO₂, -CO₂R³⁹, -CON(R³⁹)₂, -S(O)₂R²², -S(O)₂N(R²⁰)₂, -N(R²⁴)S(O)₂R²², -CN, hydroxy-(C₁-C₆)alkyl-, -OCH₂CH₂OR²², and R³⁵-aryl(C₁-C₆)alkyl-O-, or two R³² groups on adjacent carbon atoms together form a -OCH₂O- or -O(CH₂)₂O- group;
R³³ is 1 to 3 substituents independently selected from the group consisting of C₁-C₆ alkyl, halogen, -CN, -NO₂, -CF₃, -OCF₃, -OCHF₂ and -O-(C₁-C₆)alkyl;
R³⁴ is 1 to 3 substituents independently selected from the group consisting of H, halogen, -CF₃, -OCF₃, -OH and -OCH₃;
R³⁵ is 1 to 3 substituents independently selected from hydrogen, halo, C₁-C₆ alkyl, hydroxy, C₁-C₆ alkoxy, phenoxy, -CF₃, -N(R³⁶)₂, -COOR²⁰ and -NO₂;
R³⁶ is independently selected form the group consisting of H and C₁-C₆ alkyl;
R³⁷ is 1 to 3 substituents independently selected from hydrogen, halo, C₁-C₆ alkyl, hydroxy, C₁-C₆ alkoxy, phenoxy, -CF₃, -N(R³⁶)₂, -COOR²⁰, -C(O)N(R²⁹)₂ and -NO₂, or R³⁷ is one or two =O groups;
R³⁸ is H, C₁-C₆ alkyl, R³⁵-aryl, R³⁵-aryl(C₁-C₆)alkyl-, (C₁-C₆)alkyl-SO₂ or halo(C₁-C₆)alkyl-SO₂-;
R³⁹ is independently selected from the group consisting of hydrogen, C₁-C₆ alkyl, C₃-C₆ cycloalkyl, (C₃-C₆)cycloalkyl(C₁-C₆)alkyl, R³³-aryl, R³³-aryl(C₁-C₆)alkyl, and R³²-heteroaryl; and
R⁴⁰ is hydrogen, C₁-C₆ alkyl, -C(O)R²⁰, -C(O)₂R²⁰, -C(O)N(R²⁰)₂, (C₁-C₆)alkyl-SO₂-, or (C₁-C₆)alkyl-SO₂-NH-;
or R³⁹ and R⁴⁰, together with the nitrogen to which they are attached, form an azetidinyl, pyrrolidinyl, piperidinyl, piperazinyl or morpholinyl ring.

A part of this disclosure provides the compounds of the present invention. These compounds are defined in the appendant set of claims.

Also disclosed herein is a pharmaceutical composition comprising an effective amount of at least one compound of formula I, and in particular a compound of the present invention, and a pharmaceutically acceptable carrier.

Also disclosed herein is the use of a compound of the present invention for the preparation of a medicament for treating: allergy, allergy-induced airway (e.g., upper airway) responses, congestion (e.g., nasal congestion), hypotension, cardiovascular disease, diseases of the GI tract, hyper and hypo motility and acidic secretion of the gastro-intestinal tract, obesity, sleeping disorders (e.g., hypersomnia, somnolence, and narcolepsy), disturbances of the central nervous system, attention deficit hyperactivity disorder (ADHD), hypo and hyperactivity of the central nervous system (for example, agitation and depression), and/or other CNS disorders (such as Alzheimer's, schizophrenia, and migraine) comprising administering to a patient in need of such treatment (e.g., a mammal, such as a human being) an effective amount of at least one compound of formula I, and in particular a compound of the present invention.

Compounds disclosed herein, including those of the present invention, are particularly useful for treating allergy, allergy-induced airway responses and/or congestion.

This disclosure further provides a pharmaceutical composition comprising an effective amount of a combination of at least one compound of formula I, and in particular a compound of the present invention, and at least one H₁ receptor antagonist in combination with a pharmaceutically acceptable carrier.

This disclosure further provides a method of treating allergy, allergy-induced airway (e.g., upper airway) responses, and/or congestion (e.g., nasal congestion) comprising administering to a patient in need of such treatment (e.g., a mammal, such as a human being) an effective amount of a combination of at least one compound of formula I, and in particular a compound of the present invention, and at least one H₁ receptor antagonist.

Kits comprising a compound of formula I in a pharmaceutical composition, and a separate H₁ receptor antagonist in a pharmaceutical compositions in a single package are also contemplated.

### DETAILED DESCRIPTION OF THE INVENTION

Preferred definitions of the variables in the structure of formula I are as follows:
R¹ is preferably optionally substituted benzimidazolyl or 7-azabenzimidazolyl, wherein R is preferably alkyl, alkoxy, alkoxyalkoxy, alkylthio, heteroaryl or R³²-aryl. More preferably, R is -CH₃, -CH₂CH₃, -OCH₃, -OCH₂CH₃, -OCH₂CH₂CH₃, -OCH((CH₃)₂, -SCH₃, -SCH₂CH₃, pyridyl (especially 2-pyridyl), pyrimidyl, pyrazinyl, furanyl, oxazolyl or R³²-phenyl.
R²⁵ is preferably halogen or -CF₃ and k is 0 or 1.
R² is preferably a six-membered heteroaryl ring, optionally substituted with one substituent. More preferably, R² is pyrimidyl, R⁶-pyrimidyl, pyridyl, R⁶-pyridyl or pyridazinyl, wherein R⁶ is -NR⁴R⁵, wherein R⁴ and R⁵ are independently selected from the group consisting of H and (C₁-C₆)alkyl, or R⁴ and R⁵ together with the nitrogen to which they are attached form a pyrrolidinyl, piperidinyl or morpholinyl ring. More preferably, R⁶ is -NH₂.
X is preferably a bond.
Y is preferably -C(O)-.
Z is preferably straight or branched C₁-C₃ alkyl.
M¹ is preferably N; a is preferably 0; and n is preferably 2; the optional double bond is preferably not present (i.e., a single bond is present).
M² is preferably C(R³) wherein R³ is hydrogen or fluorine; b is preferably 0; r is preferably 1; and p is preferably 2.

As used herein, the following terms have the following meanings, unless indicated otherwise:
alkyl (including, for example, the alkyl portions of arylalkyl and alkoxy) represents straight and branched carbon chains and contains from one to six carbon atoms;
alkylene represents a divalent straight or branched alkyl chain, e.g., ethylene (-CH₂CH₂-) or propylene (-CH₂CH₂CH₂-);
Haloalkyl and haloalkoxy represent alkyl or alkoxy chains wherein one or more hydrogen atoms are replaced by halogen atoms, e.g., -CF₃, CF₃CH₂CH₂-, CF₃CF₂- or CF₃S;
aryl (including the aryl portion of arylalkyl) represents a carbocyclic group containing from 6 to 14 carbon atoms and having at least one aromatic ring (e.g., aryl is a phenyl or naphthyl ring), with all available substitutable carbon atoms of the carbocyclic group being intended as possible points of attachment;
arylalkyl represents an aryl group, as defined above, bound to an alkyl group, as defined above, wherein said alkyl group is bound to the compound;
cycloalkyl represents saturated carbocyclic rings of from 3 to 6 carbon atoms;
halogen (halo) represents fluoro, chloro, bromo and iodo;
heteroaryl represents cyclic groups, having 1 to 4 heteroatoms selected from O, S or N, said heteroatom interrupting a carbocyclic ring structure and having a sufficient number of delocalized pi electrons to provide aromatic character, with the aromatic heterocyclic groups preferably containing from 2 to 14 carbon atoms; examples include but are not limited to isothiazolyl, isoxazolyl, oxazolyl, furazanyl, triazolyl, tetrazolyl, thiazolyl, thiadiazolyl, isothiadiazolyl, thienyl, furanyl (furyl), pyrrolyl, pyrazolyl, pyranyl, pyrimidinyl, pyrazinyl, pyridazinyl, pyridyl (e.g., 2-, 3-, or 4-pyridyl), pyridyl N-oxide (e.g., 2-, 3-, or 4-pyridyl N-oxide), triazinyl, pteridinyl, indolyl (benzopyrrolyl), pyridopyrazinyl, isoqinolinyl, quinolinyl, naphthyridinyl; the 5- and 6-membered heteroaryl groups included in the definition of R² are exemplified by the heteroaryl groups listed above; all available substitutable carbon and nitrogen atoms can be substituted as defined;
heterocycloalkyl represents a saturated, carbocylic ring containing from 3 to 15 carbon atoms, preferably from 4 to 6 carbon atoms; examples include but are not limited to 2- or 3-tetrahydrofuranyl, 2- or 3- tetrahydrothienyl, 2-, 3- or 4-piperidinyl, 2- or 3-pyrrolidinyl, 2- or 3-piperazinyl, 2- or 4-dioxanyl, 1,3-dioxolanyl, 1,3,5-trithianyl, pentamethylene sulfide, perhydroisoquinolinyl, decahydroquinolinyl, trimethylene oxide, azetidinyl, 1-azacycloheptanyl, 1,3-dithianyl, 1,3,5-trioxanyl, morpholinyl, thiomorpholinyl, 1,4-thioxanyl, and 1,3,5-hexahydrotriazinyl, thiazolidinyl, tetrahydropyranyl.

In the definition of R³², when two R³² groups on adjacent carbon atoms of an aryl or heteroaryl ring are said to be taken together form a -OCH₂O- or -O(CH₂)₂O-group, this means that the two R³² groups form a methylenedioxy or ethylenedioxy ring fused to the aryl or heteroaryl ring. When R¹², R¹³ or R³⁷ is said to be one or two =O groups, this means that two hydrogen atoms on the same carbon atom of the ring can be replaced by =O; two such groups can be present on a ring.

Ⓝ, for example in the structure 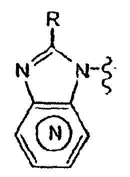 represents a nitrogen atom that is located at one of the 4 non-fused positions of the ring, i.e., positions 4, 5, 6 or 7 indicated below:

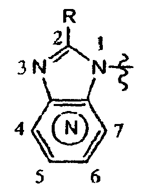

Similarly, 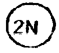 means that two nitrogens are located at any two of the 4 non-fused positions of the ring, e.g., the 4 and 6 positions, the 4 and 7 positions, or the 5 and 6 positions.

Also, as used herein, "upper airway" usually means the upper respiratory system--i.e., the nose, throat, and associated structures.

Also, as used herein, "effective amount" general means a therapeutically effective amount.

"Patient" means a mammal, typically a human, although veterinary use is also contemplated.

Lines drawn into the rings indicate that the indicated bond may be attached to any of the substitutable ring carbon atoms.

Certain compounds disclosed herein, including compounds of the present invention, may exist in different isomeric (e.g., enantiomeric, diastereoisomeric and geometric) forms. All such isomers both in pure form and in admixture, including racemic mixtures, are contemplated. Enol forms and tautomers are also included.

The compounds disclosed herein, including the compounds of the present invention, are ligands for the histamine H₃ receptor. They can also be described as antagonists of the H₃ receptor, or as H₃ antagonists.

The compounds disclosed herein, including the compounds of the present invention, are basic and form pharmaceutically acceptable salts with organic and inorganic acids. Examples of suitable acids for such salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those skilled in the art. The salts are prepared by contacting the free base form with a sufficient amount of the desired acid to produce a salt in the conventional manner. The free base forms may be regenerated by treating the salt with a suitable dilute aqueous base solution such as dilute aqueous sodium hydroxide, potassium carbonate, ammonia and sodium bicarbonate. The free base forms differ from their corresponding salt forms somewhat in certain physical properties, such as solubility in polar solvents, but the salts are otherwise equivalent to their corresponding free base forms for purposes of this disclosure.

Depending upon the substituents on the compounds disclosed herein, e.g. on the compounds of the present invention, one may be able to form salts with bases. Thus, for example, if there are carboxylic acid substituents in the molecule, salts may be formed with inorganic as well as organic bases such as, for example, NaOH, KOH, NH₄OH, tetraalkylammonium hydroxide, and the like.

The compounds of formula I can exist in unsolvated as well as solvated forms, including hydrated forms, e.g., hemi-hydrate. In general, the solvated forms, with pharmaceutically acceptable solvents such as water, ethanol and the like are equivalent to the unsolvated forms for purposes of this disclosure.

The compounds disclosed herein, including the compounds of the present invention, can be combined with an H₁ receptor antagonist (i.e., they can be combined with an H₁ receptor antagonist in a pharmaceutical composition, or they can be administered with an H₁ receptor antagonist).

Numerous chemical substances are known to have histamine H₁ receptor antagonist activity and can therefore be used. Many useful H₁ receptor antagonists can be classified as ethanolamines, ethylenediamines, alkylamines, phenothiazines or piperidines. Representative H₁ receptor antagonists include, without limitation: astemizole, azatadine, azelastine, acrivastine, brompheniramine, cetirizine, chlorpheniramine, clemastine, cyclizine, carebastine, cyproheptadine, carbinoxamine, descarboethoxyloratadine, diphenhydramine, doxylamine, dimethindene, ebastine, epinastine, efletirizine, fexofenadine, hydroxyzine, ketotifen, loratadine, levocabastine, meclizine, mizolastine, mequitazine, mianserin, noberastine, norastemizole, picumast, pyrilamine, promethazine, terfenadine, tripelennamine, temelastine, trimeprazine and triprolidine. Other compounds can readily be evaluated to determine activity at H₁ receptors by known methods, including specific blockade of the contractile response to histamine of isolated guinea pig ileum. See for example, WO98/06394 published February 19, 1998.

Those skilled in the art will appreciate that the H₁ receptor antagonist is used at its known therapeutically effective dose, or the H₁ receptor antagonist is used at its normally prescribed dosage.

Preferably, said H₁ receptor antagonist is selected from: astemizole, azatadine, azelastine, acrivastine, brompheniramine, cetirizine, chlorpheniramine, clemastine, cyclizine, carebastine, cyproheptadine, carbinoxamine, descarboethoxyloratadine, diphenhydramine, doxylamine, dimethindene, ebastine, epinastine, efletirizine, fexofenadine, hydroxyzine, ketotifen, loratadine, levocabastine, meclizine, mizolastine, mequitazine, mianserin, noberastine, norastemizole, picumast, pyrilamine, promethazine, terfenadine, tripelennamine, temelastine, trimeprazine or triprolidine.

More preferably, said H₁ receptor antagonist is selected from: astemizole, azatadine, azelastine, brompheniramine, cetirizine, chlorpheniramine, clemastine, carebastine, descarboethoxyloratadine, diphenhydramine, doxylamine, ebastine, fexofenadine, loratadine, levocabastine, mizolastine, norastemizole, or terfenadine.

Most preferably, said H₁ receptor antagonist is selected from: azatadine, brompheniramine, cetirizine, chlorpheniramine, carebastine, descarboethoxyloratadine, diphenhydramine, ebastine, fexofenadine, loratadine, or norastemizole.

Even more preferably, said H₁ antagonist is selected from loratadine, descarboethoxyloratadine, fexofenadine or cetirizine. Still even more preferably, said H₁ antagonist is loratadine or descarboethoxyloratadine.

In one preferred embodiment, said H₁ receptor antagonist is loratadine.

In another preferred embodiment, said H₁ receptor antagonist is descarboethoxyloratadine.

In still another preferred embodiment, said H₁ receptor antagonist is fexofenadine.

In yet another preferred embodiment, said H₁ receptor antagonist is cetirizine.

Preferably, in the above methods, allergy-induced airway responses are treated.

Also, preferably, in the above methods, allergy is treated.

Also, preferably, in the above methods, nasal congestion is treated.

When a combination of an H₃ antagonist of formula I, and in particular a compound of this invention, is administered with a H₁ antagonist, the antagonists can be administered simultaneously or sequentially (first one and then the other over a period of time). In general, when the antagonists are administered sequentially, the H₃ antagonist is administered first.

Compounds of formula I can be prepared by a number of ways evident to one skilled in the art. Preferred methods include, but are not limited to, the general synthetic procedures described herein. One skilled in the art will recognize that one route will be optimal depending on the choice of appendage substituents. Additionally, one skilled in the art will recognize that in some cases the order of steps has to be controlled to avoid functional group incompatibilities.

The starting material and reagents used in preparing compounds described are either available from commercial suppliers such as Aldrich Chemical Co. (Wisconsin, USA) and Acros Organics Co. (New Jersey, USA) or were prepared by literature methods known to those skilled in the art.

One skilled in the art will recognize that the synthesis of compounds of formula I may require the construction of carbon-nitrogen bond. Methods include but are not limited to the use of a substituted aromatic compound or heteroaromatic compound and amine at 0 °C to 200 °C. The reaction may be carried out neat or in a solvent. Suitable solvents for the reaction are halogenated hydrocarbons, ethereal solvents, toluene, dimethylformamide and the like.

One skilled in the art will recognize that the synthesis of compounds of formula I may require the construction of heterocycle. Methods include but are not limited to the use of a diamino compound and a carbonyl equivalent at 0 °C to 200 °C. The reaction may be carried out in acidic, basic or neutral conditions. Suitable solvents for the reaction are water, halogenated hydrocarbons, ethereal solvents, alcoholic solvents, toluene, ketones, dimethylformamide and the like.

One skilled in the art will recognize that the synthesis of compounds of formula I may require the need for the protection of certain functional groups (i.e. derivatization for the purpose of chemical compatibility with a particular reaction condition). See, for example, Green et al. Protective Groups in Organic Synthesis. A suitable protecting group for an amine is methyl, benzyl, ethoxyethyl, t-butoxycarbonyl, phthaloyl and the like which can appended to and removed by literature methods known to those skilled in the art.

One skilled in the art will recognize that the synthesis of compounds of formula I may require the construction of an amide bond. Methods include but are not limited to the use of a reactive carboxy derivative (e.g. acid halide) or the use of an acid with a coupling reagent (e.g. EDCI, DCC, HATU) with an amine at 0 °C to 100 °C. Suitable solvents for the reaction are halogenated hydrocarbons, ethereal solvents, dimethylformamide and alike.

One skilled in the art will recognize that the synthesis of compounds of formula I may require the reduction of a functional group. Suitable reducing reagents for the reaction include NaBH₄, lithium aluminum hydride, diborane and the like at -20 °C to 100 °C. Suitable solvents for the reaction are halogenated hydrocarbons, ethereal solvents, and the like.

The starting materials and the intermediates of the reaction may be isolated and purified if desired using conventional techniques, including but not limited to filtration, distillation, crystallization, chromatography and alike. Such materials can be characterized using conventional means, including physical constants and spectral data.

One method shown in Scheme 1, below, is for the preparation of compounds of formula IA wherein R¹ is 1-benzimidazolyl or 2-benzamidazolyl and X is a bond or alkyl. Similar procedures can be used to prepare compounds wherein the benzene ring of the benzimidazolyl group is substituted, as well as the aza-benzimidazoles compounds (i.e., compounds wherein R¹ is other than benzimidazolyl as defined above) and the benzoxazolyl and benzothiazolyl derivatives. 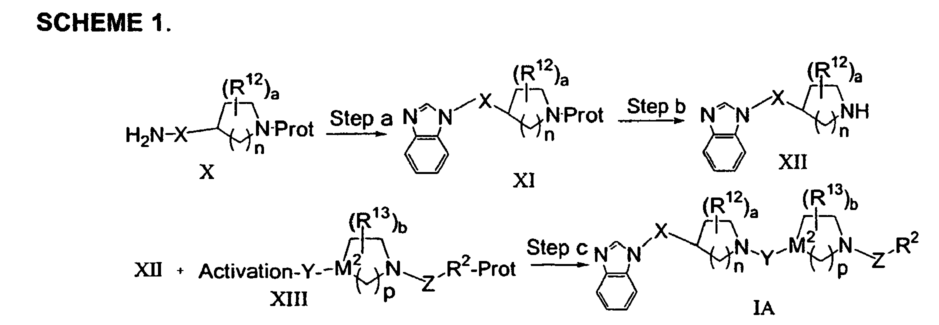
Step a: A suitably monoprotected diamine of formula **X,** wherein X is a bond or alkyl, Prot is a protecting group, and the remaining variables are as defined above is alkylated or arylated with a halide. The intermediate diamine is then cyclized with an appropriate carbonyl or formyl equivalent to form a compound of formula **XI.** Suitable protecting groups are methyl, benzyl, butoxycarbonyl, or ethoxycarbonyl. A suitable halide for alkylation is a substituted aromatic compound or a substituted heteroaromatic compound as described by Henning et al, J. Med. Chem. 30, (1987), 814-819.
Step b: The protected amine of formula **XI** is deprotected using methods known to those skilled in the art. A suitable method for methyl deprotection is reaction with a haloformate or the like. A suitable method for benzyl deprotection is cleavage with hydrogen at or above atmospheric pressure and a catalyst such as palladium. Suitable methods for carbamate deprotection are treatment with an acid, base or trimethylsilyl iodide.
Step c: An amine of formula **XII** is reacted with an activated functional group Y of formula **XIII** to form the bond between the nitrogen and functional group Y in formula IA. When Y is a carbonyl group and M² is carbon, activation can be via a halide (i.e. acid chloride intermediate) or other coupling reagents (EDCI, DCC, HATU, or like). Suitable reaction conditions may require a base such as triethylamine or N,N-diisopropylethylamine.
   Another method for the preparation of compounds of formula IA wherein R¹ is 1-benzimidazolyl or 2-benzimidazolyl and X is a bond or alkyl is shown in Scheme 2, below. Similar procedures can be used to prepare compounds wherein the benzene ring of the benzimidazolyl group is substituted, as well as the aza-benzimidazoles compounds (i.e., compounds wherein R¹ is other than benzimidazolyl as defined above). 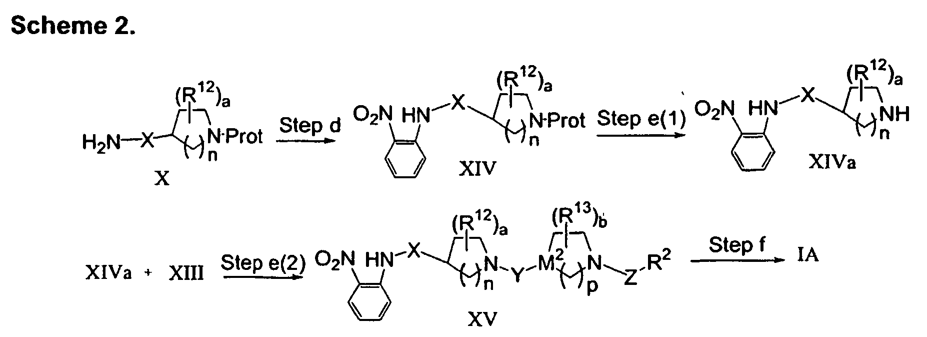
Step d: A suitably monoprotected diamine of formula **X,** wherein X is a bond or alkyl, Prot is a protecting group, and the remaining variables are as defined above, is alkylated or arylated with a halide to form a compound of formula **XIV.** Suitable protecting groups are methyl, benzyl, butoxycarbonyl, and ethoxycarbonyl. A suitable halide for alkylation is a substituted aromatic compound or a substituted heteroaromatic compound as described by Henning et al.
Step e:
   (1) The protected amine of formula **XIV** is deprotected using methods known to those skilled in the art. A suitable method for methyl deprotection is reaction with a haloformate or the like. A suitable method for benzyl deprotection is cleavage with hydrogen at or above atmospheric pressure and a catalyst such as palladium. Suitable methods for carbamate deprotection are treatment with an acid, base or trimethylsilyl iodide.
   (2) The resulting amine from Step e(1) is reacted with an activated functional group Y of formula **XIII** to form the bond between the nitrogen and functional group Y to obtain the compound of formula **XV.** When Y is a carbonyl group and M² is carbon, activation can be via a halide (i.e. acid chloride intermediate) or other coupling reagents (EDCI, DCC, HATU, or the like). Suitable reaction conditions may require a base such as triethylamine, N,N-diisopropylethylamine, pyridine, or the like.
Step f: After reduction of formula **XV,** the resulting compound is reacted with a carbonyl equivalent to give the cyclized compound of formula IA. The reduction conditions can be hydrogen in the presence of catalyst, metal in the presence of an acid or a base, or other reduction reagent. The cyclization can be performed in acidic or basic conditions.

More detailed methods for synthesis of compounds are shown in **Scheme 3** below. The preparation of compounds of formula IB wherein R¹ is 1-benzimidazolyl (Methods A, B, C and F), Y is -C(O)- and R² is substituted pyridyl, and compounds of formulas IC and IC' wherein R¹ is 2-benzimidazolyl (Methods D and E), Y is -C(O)- and R² is substituted pyridyl are shown, but those skilled in the art will recognize that similar procedures can be used to prepare compounds wherein the benzene ring of the benzimidazolyl group is substituted, R² is other than pyridyl, and aza-benzimidazoles compounds (i.e., compounds wherein R¹ is other than benzimidazolyl as defined above).

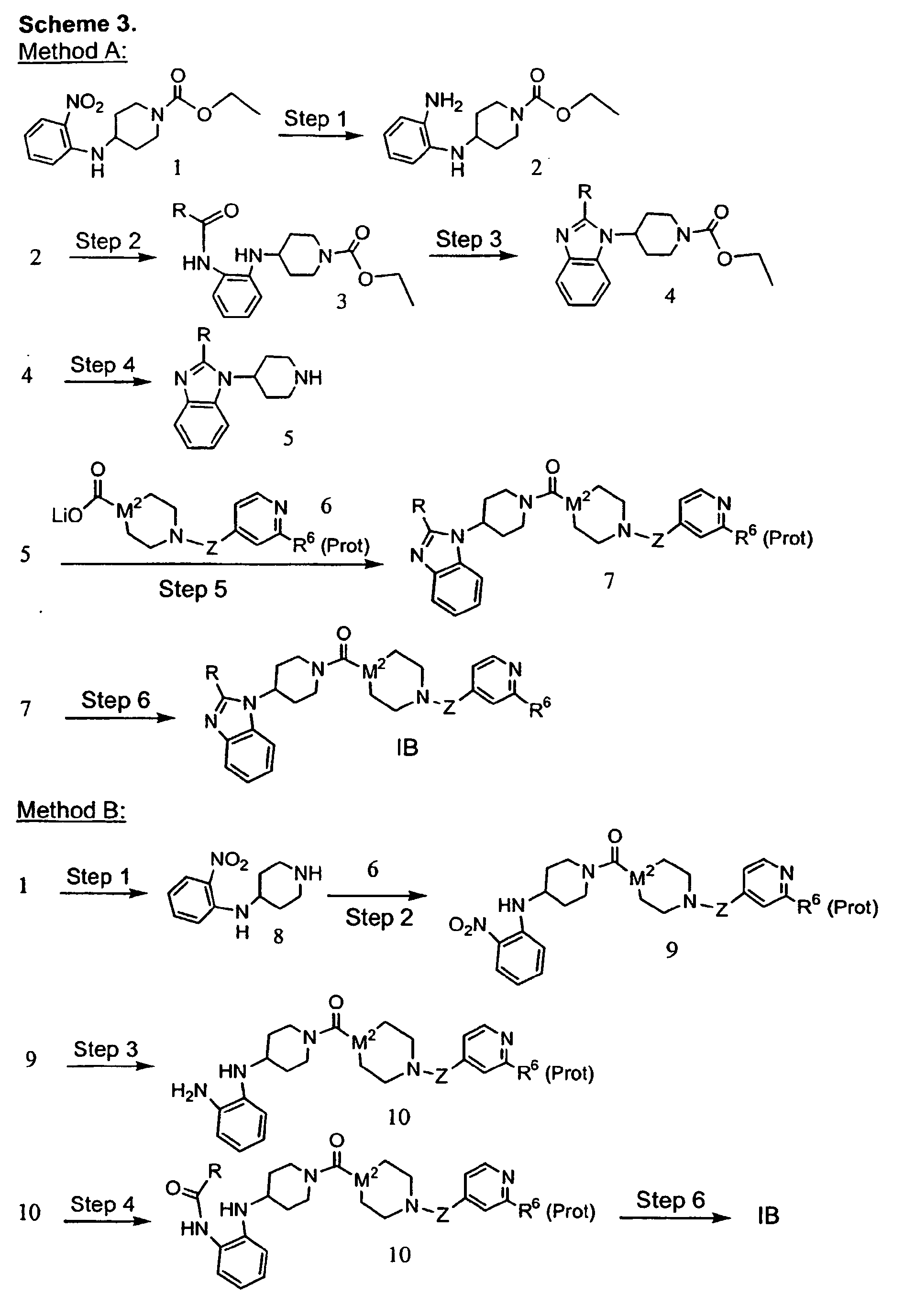

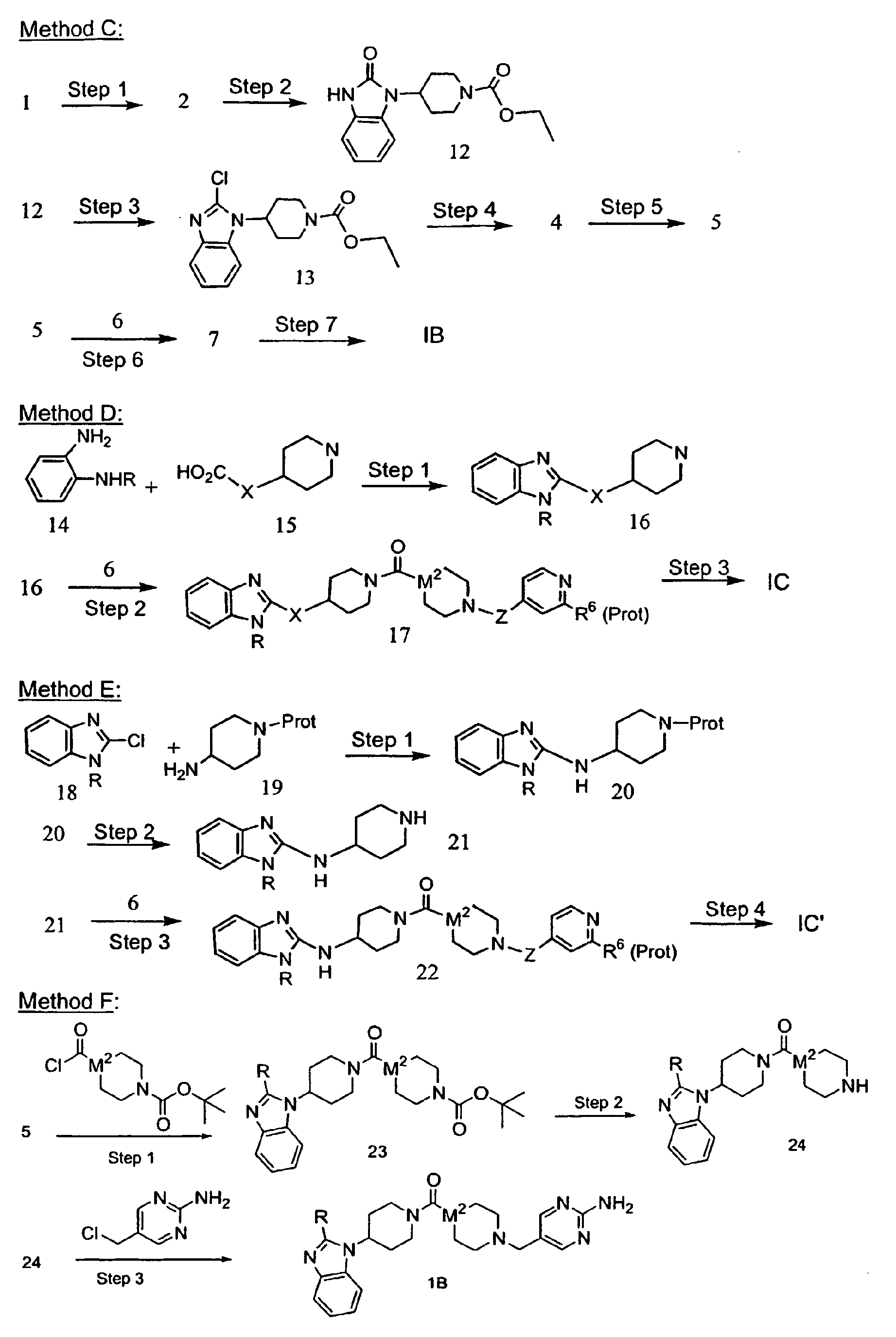

Specifically exemplified compounds were prepared as described in the examples below, from starting materials known in the art or prepared as described below. These examples are being provided to further illustrate the present invention. They are for illustrative purposes only; the scope of the invention is not to be considered limited in any way thereby.

Unless otherwise stated, the following abbreviations have the stated meanings in the Examples below:
Me=methyl; Et=ethyl; Bu=butyl; Pr=propyl; Ph=phenyl; t-BOC=tert-butyloxycarbonyl;
CBZ=carbobenzyloxy; and Ac=acetyl
DCC= dicyclohexylcarbodiimide
DMAP=4-dimethylaminopyridine
DMF=dimethylformamide
EDCI= 1-(3-dimethylaminopropyl)-3-ethylcarbodiimide
ESMS=Electron spray mass spectroscopy
FAB=Fast atom bombardment mass spectroscopy
HATU=O-(7-Azabenzotriazol-1-yl)-N,N,N',N'-tetramethyl uronium hexafluorophosphate
HOBT= 1-hydroxybenzotriazole
LAH= lithium aluminum hydride
LDA= lithium diisopropylamide
NaBH(OAc)₃= sodium triacetoxyborohydride
NBS=N-bromosuccinimide
PPA= polyphosphoric acid
RT=room temperature
TBAF=tetrabutylammonium fluoride
TBDMS=t-butyldimethylsilyl
TMEDA=N,N,N',N'-tetramethylethylenediamine
TEMPO=2,2,6,6-tetramethyl-1-piperidinyloxy, free radical
TLC=thin layer chromatography
HRMS= High Resolution Mass Spectrometry
LRMS= Low Resolution Mass Spectrometry
nM= nanomolar
Ki= Dissociation Constant for substrate/receptor complex
pA2= -logEC₅₀, as defined by J. Hey, Eur. J. Pharmacol., (1995), Vol. 294, 329-335.
Ci/mmol= Curie/mmol (a measure of specific activity)

### Preparation 1

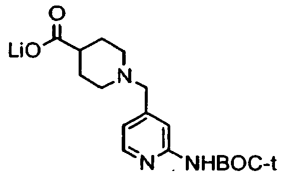

### Step 1:

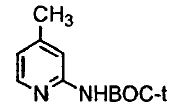

To a solution of 2-amino-4-methylpyridine (10.81 g, 100 mmol) in tert-butanol (250 ml) was added t-BOC anhydride (26.19 g, 120 mmol). The reaction mixture was stirred at 23 °C overnight, and then concentrated to an oil. The crude product was dry loaded onto a silica gel column and flash chromatographed (eluant: 30% hexanes-CH₂Cl₂ to 0-2% acetone-CH₂Cl₂) to produce 15.25 g (73.32 mmol; 73%) of the desired product as a white solid.

### Step 2:

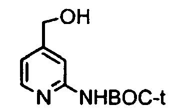

To a solution of the product of Step 1 (35.96 g, 173 mmol) in THF (1.4 l) at -78 °C was added a n-BuLi solution (1.4 M, 272 ml, 381 mmol) in hexanes portionwise over 30 min. The reaction mixture was then allowed to warm slowly and was stirred for 2 h at 23 °C, which resulted in the formation of an orange precipitate. The mixture was then cooled back to -78 °C, and pre-dried oxygen (passed through a Drierite column) was bubbled through the suspension for 6 h while the temperature was maintained at -78 °C. The color of the reaction mixture changed from orange to yellow during this time. The reaction was quenched at -78 °C with (CH₃)₂S (51.4 ml, 700 mmol) followed by AcOH (22 ml, 384 mmol) and allowed to warm with stirring to 23 °C. After 48 h, water was added and the product extracted into EtOAc. Purification by silica gel flash chromatography (eluant: 0-15% acetone/ CH₂Cl₂) provided 20.15 g (90 mmol; 52%) of the alcohol as a pale yellow solid.

### Step 3:

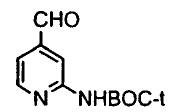

To a solution of the product of Step 2 (19.15 g, 85.5 mmol) in CH₂Cl₂ (640 ml) was added a saturated aqueous solution of NaHCO₃ (8.62 g, 103 mmol) and NaBr (444 mg, 4.3 mmol). The reaction mixture was cooled to 0 °C, and TEMPO (140 mg, 0.90 mmol) was introduced. Upon vigorous stirring, commercial bleach solution (122 ml, 0.7 M, 85.4 mmol) (5.25% in NaOCl) was added portionwise over 40 min. After an additional 20 min at 0 °C, the reaction mixture was quenched with saturated aqueous Na₂S₂O₃ and allowed to warm to 23 °C. Dilution with water and extraction with CH₂Cl₂, followed by concentration and flash chromatography (eluant: 30% hexanes-CH₂Cl₂ to 0-2% acetone-CH₂Cl₂) afforded 15.97 g (71.9 mmol; 84% yield) of the aldehyde as an off-white solid.

### Step 4:

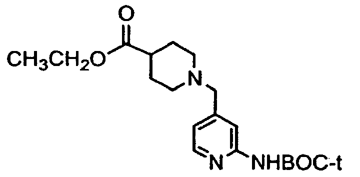

To a solution of the product of Step 3 (11.87 g, 53.5 mmol) in CH₂Cl₂ (370 ml) was added ethyl isonipecotate (9.07 ml, 58.8 mmol) followed by four drops of AcOH. The reaction mixture was then stirred for 40 min at 23 °C, after which NaB(OAc)₃H (22.68 g, 107 mmol) was added. The reaction mixture was stirred overnight at 23 °C, neutralized with saturated aqueous NaHCO₃, diluted with water and extracted with CH₂Cl₂. Concentration of the organic extracts, followed by silica gel flash chromatography (eluant: 0-4% sat. NH₃ in CH₃OH-CH₂Cl₂) provided 19.09 g (52.6 mmol; 98%) of the ester as an off-white solid.

### Step 5:

To a solution of the product of Step 4 (1.57 g, 4.33 mmol) in THF-water-CH₃OH (10 ml of a 3:1:1 mixture) was added LiOH monohydrate (0.125 g, 5.21 mmol). The reaction mixture was stirred overnight at 23 °C, concentrated and exposed to high vacuum to obtain 1.59 g of crude title compound as a yellowish solid which was used without purification.

### Preparation 2

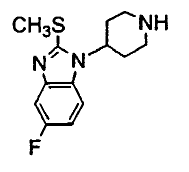

### Step 1:

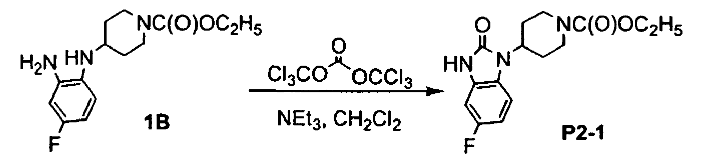

A solution of diamine **1B** (see Method A, Step 1) (20g, 71.1mmol) and Et₃N (30 ml, 213 mmol) in CH₂Cl₂ (400 ml) was cooled to 0 °C in an ice-water bath. To the well-stirred solution was added triphosgene (14.2 g, 47.3 mmol) cautiously (exotherm!) and portionwise over a period of 30 min. When addition was complete, stirring was continued at 0 °C for 1 h, then at RT for 16 h. The mixture was washed with 0.5N NaOH (200 ml), the organic layer was dried over anhydrous MgSO₄ and concentrated under vacuum. Hot EtOAc (200 ml) was added to the semi-solid residue, and the resultant mixture was cooled to RT. Filtration yielded compound P2-1 as a white solid (16.5g); and silica gel flash chromatography [CH₂Cl₂/CH₃OH (2N NH₃) = 40:1] of the filtrate provided additional product as a white solid (2.7 g) [combined yield: 88%]. FABMS: 308 (MH⁺; 100%).

### Step 2:

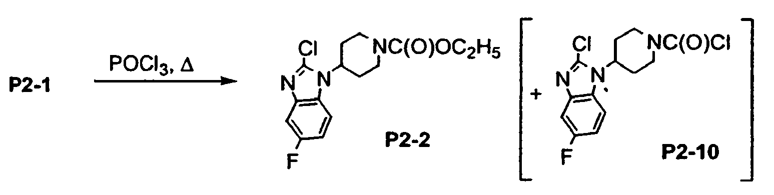

POCl₃ (100 ml) was added to **P2-1** (17.2 g; 56 mmol) in a round-bottomed flask flushed with dry N₂. The mixture was placed in an oil bath heated to 108 °C and was maintained at reflux for 6 h. POCl₃ was then removed in vacuo. The residue was adjusted to pH ∼ 9-10 with 7N methanolic ammonia and was concentrated to dryness under vacuum. CH₂Cl₂ was added to the residue, insoluble material was filtered off, and the filtrate was again concentrated in vacuo. The residue was crystallized from EtOH to obtain compound **P2-2** as a white solid (12.6 g; 67%). ES-MS: 326.1 (MH⁺; 100%).

Varying amounts of compound **P2-10** may be formed in this process and can be converted to desired product **P2-2** by careful in situ treatment in CH₂CI₂ solution at 0 °C with one equivalent each of EtOH and NaH, followed by workup with ice-water and CH₂Cl₂. Low temperature is maintained in order to minimize reaction at the 2-position of the benzimidazole nucleus.

### Step 3:

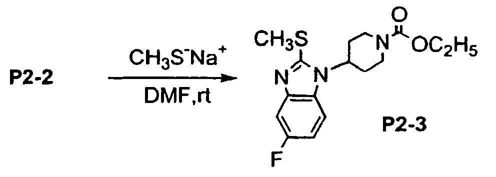

Sodium thiomethoxide (1.05 g; 15.0 mmol) was added to DMF (15 ml) in a round-bottomed flask flushed with N₂. After stirring at RT for 30 min, solid chloride **P2-2** (3.25 g, 10 mmol) was added, and the resultant mixture was kept stirring at RT for 16 h. EtOAc (100 ml) and water (50 ml) were added to the reaction mixture. The aqueous layer was separated and further extracted with EtOAc (50 ml). The combined extracts were dried over anhydrous MgSO₄ and concentrated under vacuum. The residue was purified via flash chromatography on silica gel, eluting with EtOAc-hexanes (3:4), to obtain compound **P2-3** as a white solid (2.12 g; 63%). FABMS: 338.3 (MH⁺; 100%).

### Step 4 :

To a stirred solution of **P2-3** (300 mg, 12.5 mmol) in EtOH (40 ml)-isopropyl alcohol (40 ml) was added 25% (w/w) aqueous NaOH solution (20 ml). The resultant mixture was stirred at 85 °C for 24 h, then at 100 °C for an additional 4 h. Alcohols were removed under vacuum, and the aqueous residue was extracted sequentially with CH₂CI₂ (2 x 40 ml), then EtOAc (30 ml). Combined extracts were dried over anhydrous MgSO₄. Drying agent was removed by filtration, and the filtrate was concentrated under vacuum. The residue was purified by silica gel flash chromatography (CH₂Cl₂/2N methanolic ammonia = 12:1) to obtain Preparation 2 as an off-white solid (2.85 g, 70%). ES-MS: 266 (MH⁺; 100%).

### Preparation 3

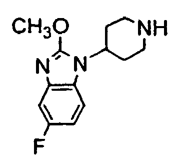

### Step 1:

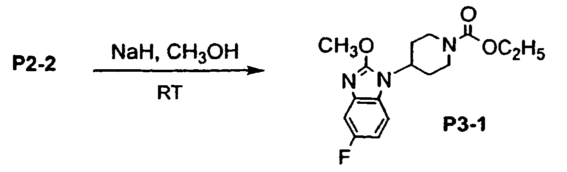

NaH (60 mg of a 60% dispersion; 1.48 mmol) was added to CH₃OH (4 ml) in a flask charged with N₂. After stirring at RT for 30 min, chloride **P2-2** (400 mg, 1.23 mmol) was added, and the resultant mixture was stirred at RT for 16 h. CH₃OH was removed in vacuo, and to the residue were added CH₂Cl₂ (30 ml) and water (10 ml). The organic layer was dried over anhydrous MgSO₄, filtered, and the filtrate concentrated under vacuum. The residue was purified via flash chromatography on silica gel, eluting with EtOAc-hexanes (3:2) to obtain **P3-1** as a white foam (0.232g; 59%). ES-MS: 322.1 (MH⁺; 100%).

### Step 2:

1 N aqueous KOH (4.82 mL; 4.82 mmol) was added to a solution of **P3-1** in EtOH (15 ml), and the resultant mixture was stirred at 80 °C for 48 h. The mixture was concentrated under vacuum. Water (3 ml) and CH₂Cl₂ (15 ml) were added to the residue, and the organic layer was separated and dried over anhydrous MgSO₄. Drying agent was filtered, and the filtrate was concentrated in vacuo to obtain Preparation 3 as a colorless glass (160mg; 95%). FABMS: 250.2 (MH⁺; 100%).

### Preparation 4

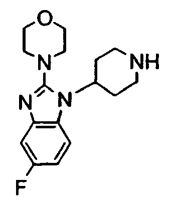

### Step 1:

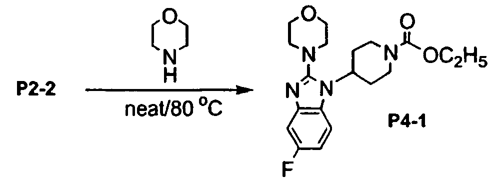

**P2-2** (300 mg; 0.923 mmol) and morpholine (3 ml) were mixed in a round-bottomed flask under N₂, and the resultant mixture was heated to 80 °C for 16 h. Morpholine was removed under vacuum, and the residue was dissolved in CH₂Cl₂ (20 ml). An insoluble white precipitate was filtered off, and the filtrate was concentrated and purified by means of flash chromatography on silica gel, eluting with CH₂Cl₂/2N methanolic ammonia (45:1), to obtain **P4-1** as a colorless glass (0.325g; 94%). ES-MS: 377.1 (MH⁺; 100%).

### Step 2:

Trimethylsilyl iodide (240 microliters; 1.64mmol) was added to a solution of **P4-1** (316 mg; 0.843 mmol) in CHCl₃ (2 ml) under N₂, and the resultant solution was stirred at 55 °C for 7 h. The reaction was quenched with EtOH (2 ml), and the mixture was concentrated to dryness under vacuum. The residue was basified with a 1:1 (v/v) mixture of concentrated NH₄OH and water to pH ∼10 and extracted with CH₂CI₂ (2 x 5 ml). The combined extracts were dried over anhydrous MgSO₄. Drying agent was filtered, and the filtrate was concentrated under vacuum. The residue was purified via flash chromatography on silica gel, eluting with CH₂Cl₂-2N methanolic ammonia (13:1), to obtain compound Preparation 4 as a colorless glass. (181 mg; 70%). ES-MS: 305.1 (MH⁺; 100%).

### Preparation 5

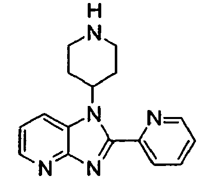

### Step 1:

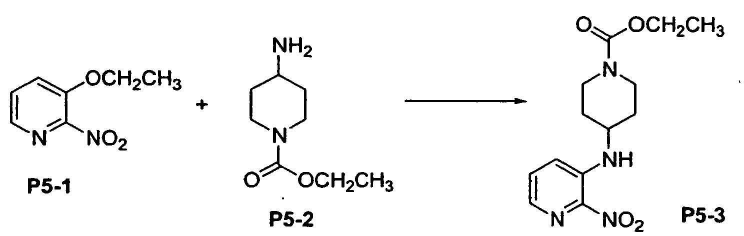

A solution of **P5-1** (3.5 g, 21 mmol) and **P5-2** (6.5 g, 38 mmol) in CH₂Cl₂ (3 ml) was heated to 110° C for 24 h and RT for 24 h. The reaction was diluted with CH₂Cl₂, washed with water and brine, and dried (Na₂SO₄). Purification on a flash column (SiO₂, 40% to 60% EtOAc in hexanes) gave P5-3 (1.3 g, 21%; M+H = 295).

### Step 2:

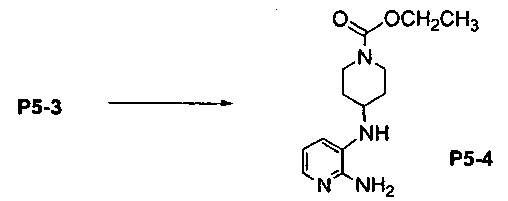

To a solution of **P5-3** (1.3 g, 4.4 mmol) in CH₃OH (30 ml) was added Ra-Ni (0.5 g) and the mixture was hydrogenated under a H₂ atmosphere (50 psi) for 18 h. Filtration through a pad of celite gave **P5-4** as a grey solid that was used without further purification (1.05 g, 90%; M+H = 265).

### Step 3:

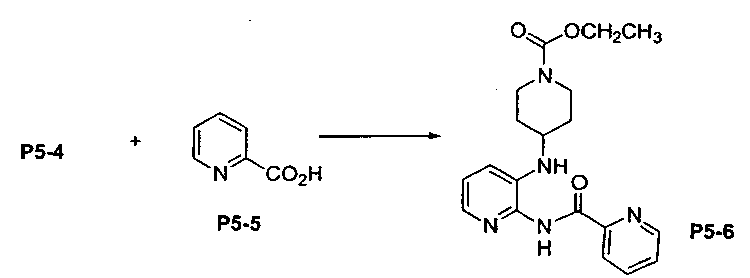

A solution of **P5-4** (1.05 g, 3.97 mmol), **P5-5** (0.49 g, 3.97 mmol), DEC (1.14 g, 5.96 mmol) and HOBT (0.8 g, 5.96 mmol) in CH₂Cl₂ (10 ml) were stirred for 18 h at RT. The crude reaction mixture was diluted with additional CH₂CI₂ and washed with 5% aqueous NaOH and brine and dried (Na₂SO₄). Purification using flash chromatography (SiO, 8% EtOAc in hexane to 10% CH₃OH in EtOAc) gave **P5-6** (0.35 g, 24%; M+H = 370).

### Step 4:

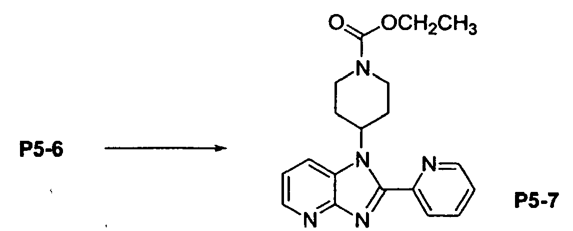

Compound **P5-6** (0.7 g, 1.89 mmol) was dissolved in HOAc (10 ml) and heated to 120° C for 3.5 h. The reaction was cooled to RT, concentrated in vacuo, neutralized by the addition of 10% aqueous NaOH and extracted with CH₂Cl₂. The combined organic layers were dried (Na₂SO₄) and concentrated to give **P5-7** (0.58 g, 87%; M+H = 352) which was used in the next step without further purification.

### Step 5:

A solution of **P5-7** (0.58 g, 1.65 mmol) and NaOH (0.43 g, 13.2 mmol) in EtOH/H₂O (9/1, 10 ml) was heated to 100° C for 18 h. The reaction was cooled and concentrated and the residue purified on a flash column (SiO₂, 10% CH₃OH saturated with ammonia in CH₂Cl₂) to give Preparation 5 (0.42 g, 91%; M+H = 280).

### Preparation 6

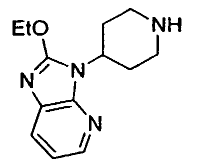

### Step 1:

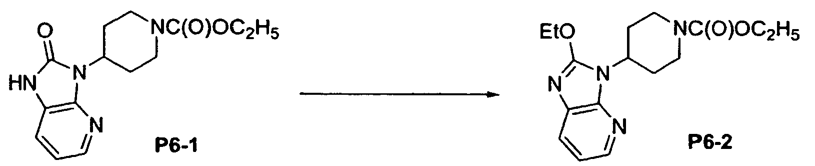

A solution of compound **P6-1** (prepared by procedures analogous to **P2-1**) (10.5 g, 36.2 mmol) and 2,6-di-*tert*-butylpyridine (12.2 ml, 54.4 mmol) in CH₂Cl₂ (400 ml) was treated with 1M sol. of Et₃O⁺BF₄⁻ (in CH₂Cl₂, 55 ml, 55 mmol). The reaction mixture was stirred at RT for 2h, quenched with 1 N NaOH (100 ml), extracted with CH₂Cl₂ (3x), dried with Na₂SO₄ and concentrated. Purification by silica gel chromatography (eluant: 5-10% acetone/ CH₂Cl₂) to give 6.37 g of **P6-2** (20.0 mmol, 55%).

### Step 2:

In a manner similar to that described in Preparation 3, Step 2, **P6-2** was converted to Preparation 6.

### Preparation 7

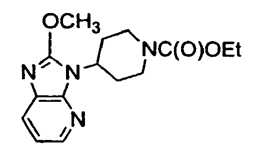

### Step 1:

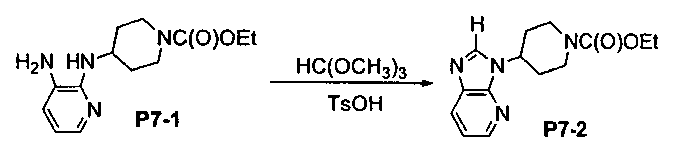

A mixture of **P7-1** (40g, 150 mmol), trimethyl orthoformate (66 ml, 64.0 g, 600 mmol) and a catalytic amount of p-toluenesulfonic acid monohydrate (300 mg, 1.58 mmol) was stirred under N₂ at 120 °C for 3 h. Excess orthoformate was removed under vacuum. The residue was partitioned between EtOAc (200 ml) and 1 N NaOH (100 ml). The organic layer was washed with brine (100 ml) and dried over anhydrous MgSO₄. Drying agent was removed by filtration, and the filtrate was concentrated under vacuum. The residue was purified by silica gel flash chromatography (CH₂Cl₂/CH₃OH (2N NH₃) = 45:1) to obtain **P7-2** as a dark purple syrup (27.2 g, 66%), which solidified upon standing. ES-MS: 275 (MH⁺; 100%).

### Step 2:

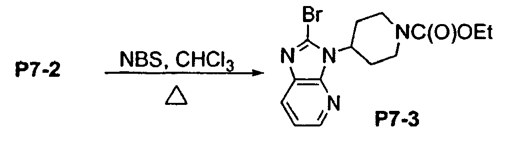

NBS was added portionwise (exotherm) to a solution of **P7-2** (27 g, 100 mmol) in CHCl₃ (300 ml), and the resultant solution was stirred at 60 °C for 16 h. Solvent was then removed under vacuum, and the residue was partitioned between EtOAc (200 ml) and 0.7N Na₂S₂O₄ (250 ml). The organic layer was washed with brine (150 ml) and dried over anhydrous MgSO₄. Drying agent was removed by filtration, and the filtrate was concentrated under vacuum. The residue was purified by silica gel flash chromatography [CH₂Cl₂/acetone = 45:1] to obtain **P7-3** as a yellow solid (24.2 g, 69%). ES-MS: 353 (MH⁺; 100%).

### Step 3:

NaH (544 mg of a 60% dispersion, 13.6 mmol) was added to a solution of CH₃OH (0.551 ml, 436 mg, 13.6 mmol) in DMF (5 ml). The resultant mixture was stirred at RT for 30 min before adding solid bromide **P7-3** (3.99 g, 11.3 mmol). The reaction suspension was stirred at RT for 16 h. The mixture was then partitioned between EtOAc (800 ml) and water (40 ml). The aqueous layer was extracted with EtOAc (40 ml). Combined extracts were washed with brine (30 ml) and dried over anhydrous MgSO₄. Drying agent was removed by filtration, and the filtrate was concentrated under vacuum to obtain Preparation 7 as a white syrup (2.81 g, 81%), which was used without further purification. ES-MS: 305 (MH⁺; 100%).

### Preparation 8

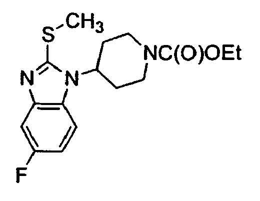

### Step 1:

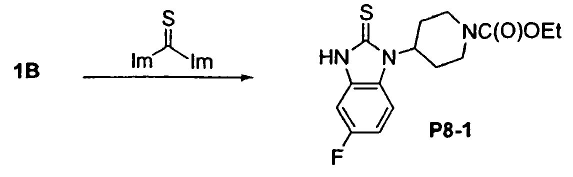

A solution of **1B** (15 g, 52.8 mmol) and 1,1'-thiocarbonyldiimidazole (25 g, 140 mmol) in THF (300 ml) was stirred at 72 °C under N₂ for 16 h, during which time a precipitate formed. THF was removed under vacuum, and the residue was purified by silica gel flash chromatography (CH₂Cl₂/acetone = 20:1) to obtain **P8-1** as a light yellow solid (16.7 g, >95%). ES-MS: 324 (MH⁺; 100%).

### Step 2:

To a stirred mixture of **P8-1** (4.00 g, 12.5 mmol) and K₂CO₃ (2.05 g, 13.6 mmol) in DMF (40 ml) under a N₂ atmosphere was added CH₃I (0.85 ml, 1.94 g, 13.6 mmol). The resultant mixture was stirred at RT for 16 h before partitioning between EtOAc (100 ml) and water (40 ml). The aqueous layer was extracted with EtOAc (40 ml). Combined extracts were washed with brine (30 ml) and dried over anhydrous MgSO₄. Drying agent was removed by filtration, and the filtrate was concentrated under vacuum to obtain Preparation 8 as a foamy white solid (4.20 g, >95%; contained a small amount of DMF), which was used without further purification. ES-MS: 338 (MH⁺; 100%).

### Preparation 9

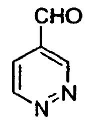

### Step 1:

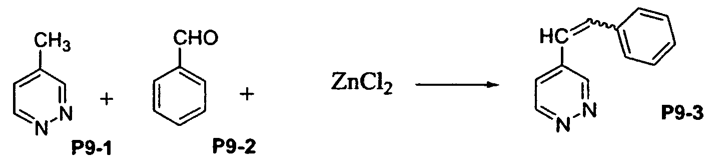

(Modified published procedure: G. Heinisch, E. Luszczak, and M. Pailer: Monatshefte für Chemie, 1973 (104), 1372.

**P9-1** (4.5 g, 47.8 mmoles), **P9-2** (8.12g, 76.5 mmoles), and anhydrous ZnCl₂ were heated, under N₂, in a dry apparatus, at a bath temperature of 160 °C for 5 h. The resulting oil was purified by flash chromatography on silica gel using 30% Hexanes/EtOAc, yielding 5.92 grams (67%) of the product.

### Step 2:

OsO₄ (5.0 ml in t-butanol, 2.5% w/w) was added to **P9-3** (5.9 g, 32.38 mmoles) dissolved in p-dioxane (87 ml) and water (29 ml). NalO₄ (14.1 g, 65.92 mmoles) was added, with good stirring, in small portions, over a period of 6 h. The mixture was then diluted with p-dioxane and filtered. After removing most of the solvent under reduced pressure, the residue was taken in CH₂Cl₂ (600 ml) and dried over anhydrous Na₂SO₄. After removal of the solvent, the mixture was purified by flash chromatography on silica gel using 5% CH₃OH/CH₂Cl₂ as eluent to obtain Preparation 9. Yield: 2.89 g (82%).

### Preparation 10

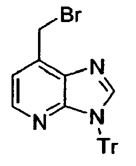

### Step 1:

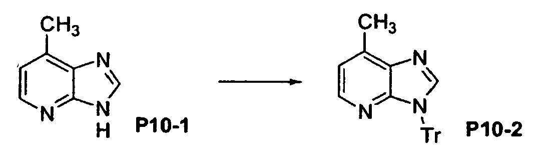

A solution of **P10-1** (2 g, 15 mmol) in CH₂Cl₂ (50 ml) was treated with Et₃N (3 g, 30 mmol) and triphenylmethyl chloride (TrCl, 4.25 g, 15.3 mmol) and stirred at RT overnight. The solvent was removed in vacuo and the residue purified via flash column chromatography (SiO₂, 20% EtOAc in hexane) to give **P10-2** (5.2 g, 46%).

### Step 2:

A solution of **P10-2** (5.2 g, 14.6 mmol) in CCI₄ (80 ml) was treated with NBS (7.8 g, 43 mmol) and the reaction heated to 80° C overnight. The reaction was cooled, filtered and concentrated, and the residue was purified via flash column chromatography (SiO₂, 20% to 30% EtOAc in hexane) to give Preparation 10 (2.8 g, 42%, M+H = 453, 455)

### Preparation 11

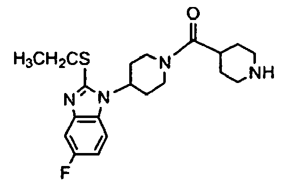

### Step 1:

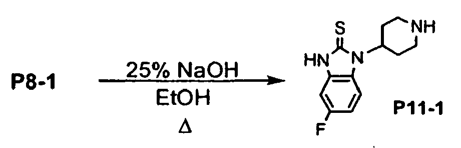

To a stirred solution of **P8-1** (6.5 g, 20.1 mmol) in EtOH (80 ml) was added 25% (w/w) aqueous NaOH solution (20 ml). The resultant mixture was stirred at 90 °C for 16 h. EtOH was removed under vacuum, and the residue was adsorbed directly onto silica gel and subjected to flash chromatography (CH₂Cl₂/2N methanolic ammonia = 9:1) to obtain **P11-1** as a white solid (4.46 g, 70%). ES-MS: 252 (MH⁺; 100%).

### Step 2:

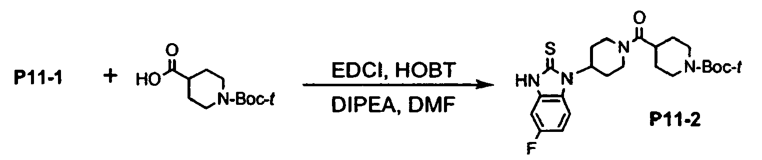

A mixture of **P11-1** (3.95 g; 15.7 mmol), BOC-isonipecotic acid (3.60 g; 15.7 mmol), HOBT (3.19 g; 23.6 mmol), DIPEA (3ml; 2.23g; 17.2 mmol) and EDCI (4.50 g; 23.6 mmol) in DMF (30 ml) was stirred under N₂ at RT for 16 h. The reaction mixture was partitioned between EtOAc (60 ml) and water (40 ml). The aqueous phase was extracted with EtOAc (40 ml), and the combined extracts were washed with brine (40 ml) and dried over anhydrous MgSO₄. Drying agent was removed by filtration, and the filtrate was concentrated under vacuum. The residue was purified by silica gel flash chromatography (CH₂Cl₂/CH₃OH (2N NH₃) = 40:1) to obtain **P11-2** as a white solid (-7.3 g, ∼100%), containing a small amount of DMF, used without further purification in Step 3 below. ES-MS: 463 (MH⁺; 70%); 407 (100%).

### Step 3:

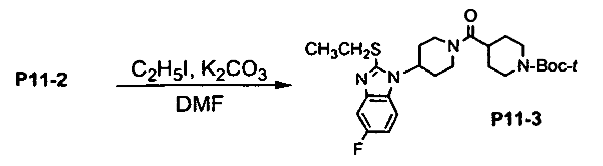

To a stirred mixture of **P11-2** (460 mg; 1 mmol) and K₂CO₃ (165 mg; 1.20 mmol) in DMF (4 ml) under a N₂ atmosphere was added EtI (92 microliters; 179 mg; 1.15 mmol). The resultant mixture was stirred at RT for 16 h and was then partitioned between EtOAc (20 ml) and water (10 ml). The aqueous phase was extracted with EtOAc (10 ml), and the combined extracts were washed with brine (20 ml) and dried over anhydrous MgSO₄. Drying agent was removed by filtration, and the filtrate was concentrated under vacuum to obtain **P11-3** as a pale yellow foam (471 mg, 96%), containing a small amount of DMF, used without further purification in Step 4 below. ES-MS: 463 (MH⁺; 85%); 435 (100%).

### Step 4:

To a solution of **P11-3** (465 mg; 0.949 mmol) in CH₂Cl₂ (4 ml) was added TFA (1 ml; 1.54 g; 13.5 mmol). The resultant solution was stirred for 2 h at RT and was then partitioned between CH₂Cl₂ (20 ml) and 1:1 (v/v) concentrated NH₄OH:water (5 ml). The aqueous phase was extracted successively with 95:5 CH₂CL₂:EtOH (5 ml) and EtOAc (5 ml). The combined extracts were dried over anhydrous MgSO₄. Drying agent was removed by filtration, and the filtrate was concentrated under vacuum to obtain Preparation 11 as a pale white foam (353 mg, 95%), used without further purification. ES-MS: 391 (MH⁺; 100%).

### Example 1

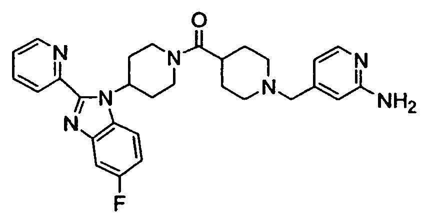

### Method A

### Step 1:

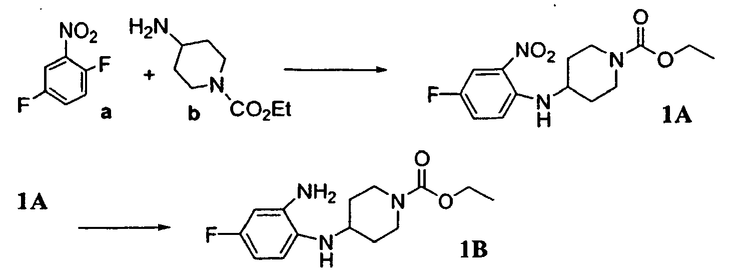

A mixture of a (25 g, 0.16 mol), b (27 g, 0.16 mol), K₂CO₃ (26 g, 0.19 mol), and Nal (2.4g, 0.016 mol) in dimethylacetamide (50 ml) was heated at 140 °C for 3.5 h. The reaction mixture was concentrated to one-third volume, poured onto saturated aqueous NaHCO₃, and extracted with EtOAc (4×). The combined organic layers were washed with water (2×) and brine, dried over Na₂SO₄, and concentrated. Recrystallization with EtOH provided **1A** (48 g, 98%).

A suspension of **1A** (20.00 g, 64.2 mmol,) and Raney® 2800 Nickel (5.0 g) in ethanol (70 ml) and THF (140 ml) was shaken under H₂ (40 psi) for 2 h. The mixture was filtered through a short pad of celite. The filtrate was concentrated and dried on vacuum to deliver a tan solid (18.20 g, ∼100%).

### Step 2:

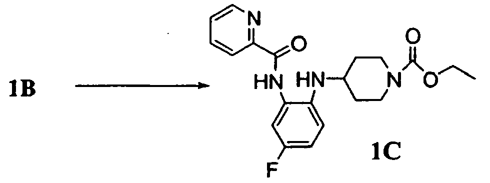

A solution of **1B** (5.00 g, 17.77 mmol) and picolinoyl chloride hydrochloride (3.16g, 17.75 mmol) in CH₂Cl₂ (400 ml) and Et₃N (15 ml) was stirred at RT. After 15 h, the reaction was diluted with CH₂Cl₂, washed with water, dried over Na₂SO₄, concentrated, and dried on vacuum to provide a brown foam (6.47g, 94%).

### Step 3:

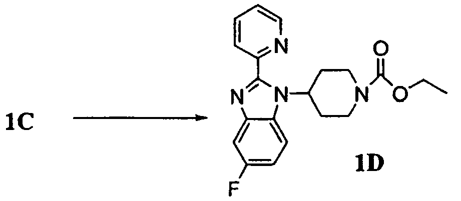

A solution of **1C** (1.77g, 4.58 mmol) in ethanol (50 ml) and concentrated H₂SO₄ (5.0 ml) was refluxed for 3 h, cooled to RT, and neutralized with 1.0 M NaOH until pH = 10. The resulting mixture was extracted with CH₂Cl₂. The combined organic solutions were dried over Na₂SO₄ and concentrated on reduced pressure. The residue was purified by flash chromatography (silica gel, 5% CH₃OH in CH₂Cl₂ as eluent) to provide a tan foam (1.58g, 94%).

### Step 4:

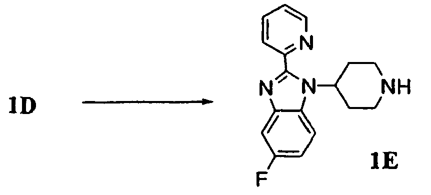

lodotrimethylsilane (6.30g, 31.48 mmol) was added to a solution of **1 D** (3.88g, 10.53 mmol) in anhydrous 1,2-dichloroethane (40 ml). The resulting solution was stirred at 75 °C for 4 hours, cooled to RT, and treated with 1.0 M NaOH solution. The mixture was then extracted with CH₂Cl₂. The combined extracts were washed with water, dried over Na₂SO₄, and the solvent evaporated. Purification of the residue by flash chromatography (silica gel, 10% CH₃OH in CH₂Cl₂ as eluent) delivered an off-white foam (2.10g, 67%).

### Step 5:

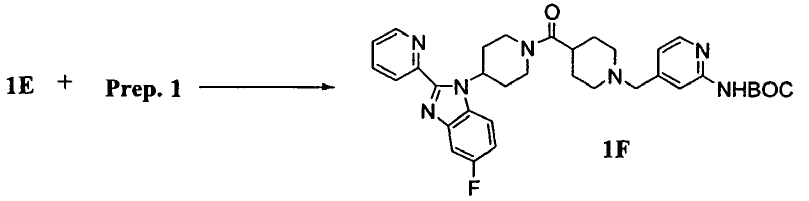

Amine **1E** (5.80g, 19.6 mmol) and Preparation 1 (5.32g, 23.4 mmol) were dissolved in DMF (60 ml) and CH₂Cl₂ (60 ml). To the resulting solution, EDCI hydrochloride (5.70g, 24.50 mmol), HOBT (1.30g, 24.50 mmol), and diisopropylethylamine (5.08g, 39.6 mmol) were added successively. The resulting reaction mixture was stirred at 70°C for 4 hours, cooled to RT, diluted with CH₂Cl₂, washed with water, dried over Na₂SO₄, and concentrated. Flash chromatography (SiO₂, 5% CH₃OH in CH₂Cl₂ → 90:10:0.5 CH₂Cl₂:CH₃OH:NH₄OH) of the residue provided a tan foam (7.89g, 65%).

### Step 6:

A solution of **1F** (7.89g, 12.88 mmol) and TFA (29g, 257 mmol) in CH₂Cl₂ (65 ml) was stirred at RT for 12 h, neutralized with 1.0 M NaOH, and extracted with CH₂Cl₂. The combined organic layers were washed with water, dried over Na₂SO₄ and concentrated. Purification of the crude product by flash chromatography (SiO₂, 5% CH₃OH in CH₂Cl₂ to 90:10:0.5 CH₂Cl₂:CH₃OH:NH₄OH) provided the title compound as a white solid (5.80g, 88%). MS: 514 (MH⁺).

### Example 2

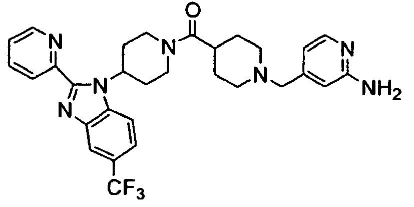

### Method B

### Step 1:

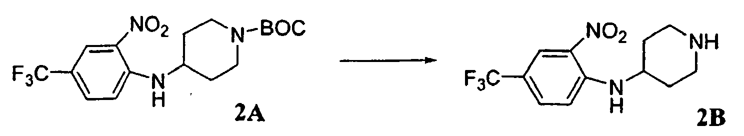

TFA (200 ml, 2.596 mol) was added to a solution of **2A** (20g, 51.36 mmol) in CH₂Cl₂ (100 ml). The resulting reaction mixture was stirred at RT for 6 h, neutralized with 1.0 M NaOH, and extracted. The combined extracts were washed with water, dried over Na₂SO₄, and concentrated. Flash chromatography gave an orange solid (13.50g, 91%).

### Step 2:

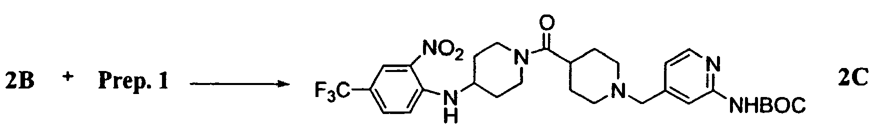

Amine **2B** (1.50g, 5.19 mmol) and Preparation 1 (1.75g, 5.13 mmol) were dissolved in DMF (10 ml) and CH₂Cl₂ (10 ml). To the resulting solution, EDCI hydrochloride (1.50g, 7.83 mmol), HOBT (1.05g, 7.82 mmol), and diisopropylethylamine (3.71g, 28.70 mmol) were added successively. The resulting reaction mixture was stirred at 70°C for 18 h, cooled to RT, diluted with CH₂Cl₂, washed with water, dried over Na₂SO₄, and concentrated. Flash chromatography of the residue provided an orange gel (2.31 g, 74%).

### Step 3:

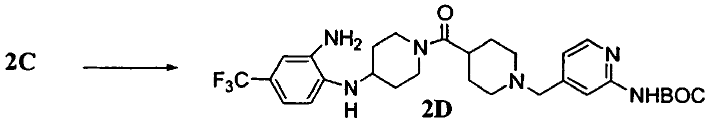

A suspension of **2C** (2.10 g, 3.46 mmol,) and Raney® 2800 Nickel (1.0 g) in CH₃OH (100 ml) was shaken under H₂ (30 psi) for 6 h. The mixture was filtered through a short pad packed with celite. The filtrate was concentrated and dried on vacuum to deliver an orange solid (1.80 g, 90%).

### Step 4:

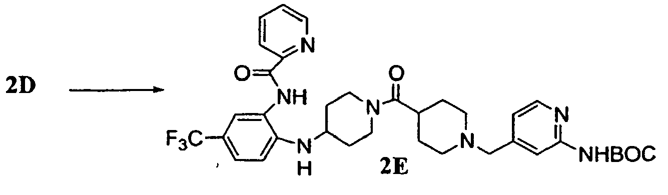

Amine **2D** (200 mg, 0.347 mmol) and picolinoyl chloride hydrochloride (62 mg, 0.348 mmol) were dissolved in CH₂Cl₂. Et₃N was then introduced via a syringe. The resulting solution was stirred at RT for 6 h, treated with 1.0 M NaOH solution, and extracted. The extracts were washed with water, dried over Na₂SO₄, and concentrated. Purification of the residue by flash chromatography gave a white foam (167 mg, 71 % yield).

### Step 5:

A solution of **2E** (160 mg, 0.235 mmol) and H₂SO₄ (concentrated, 0.50 ml) in ethanol (10 ml) was refluxed for 2.5 h, cooled to RT, and neutralized with 1.0 M NaOH. After extraction of the mixture, the combined organic layers were washed with water, dried over Na₂SO₄, and concentrated. Purification of the crude product using prep TLC (10% CH₃OH in CH₂Cl₂) provided the title compound as a white solid (88 mg, 66%). MS: 564 (MH⁺)

### Example 3

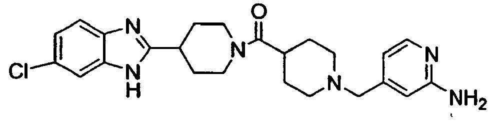

### Method D

### Step 1:

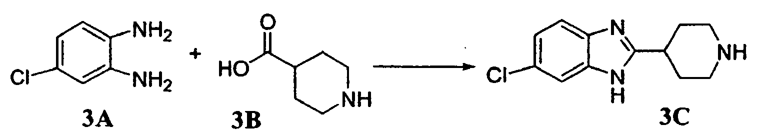

Diamine **3A** (1.43 g, 10 mmol) and isonipecotic acid 3B (1.29 g, 10 mmol) were mixed, and PPA (20 g) was added. The resulting mixture was heated at 180 °C for 3.5 h, cooled to RT and diluted with water to 100 ml. The solution was then basified with solid NaOH to pH 14. The resultant copious precipitate was filtered off. The precipitate was washed repeatedly with CH₃OH, and combined CH₃OH extracts were concentrated-dry loaded on silica gel and flash chromatographed (25-40% 5N NH₃ in CH₃OH/ CH₂Cl₂) to provide 3C as a dark solid (1.90 g, 81%).

### Step 2:

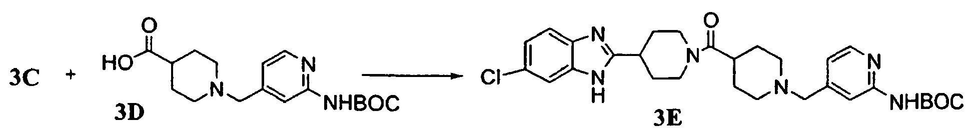

To the mixture of acid **3D** (181 mg, 0.54 mmol), HATU (247 mg, 0.65 mmol) and Et₃N (84 µl, 0.6 mmol) in DMF (12 ml) was added amine **3C** (126 mg, 0.54 mmol). The resulting mixture was stirred at RT for 24 h, concentrated, redissolved in CH₃OH, concentrated-dry loaded on silica gel and flash chromatographed (5-10% 5N NH₃ in CH₃OH/ CH₂Cl₂) to provide **3E** as a yellow oil (210mg, 70%).

### Step 3:

A solution of **3E** (96 mg, 0.174 mmol) in 15 ml of 1 M HCl in 25% CH₃OH/dioxane was stirred at RT for 48 h. The mixture was concentrated, exposed to high vacuum, redissolved in CH₃OH, concentrated-dry loaded on silica gel and flash chromatographed (10-15% 5N NH₃ in CH₃OH/ CH₂Cl₂) to provide the title compound as a colorless oil (48 mg, 61 %). MS: 453 (MH⁺)

### Example 4

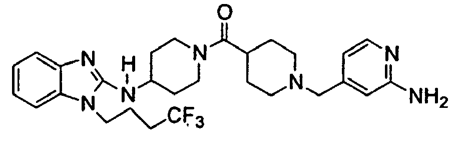

### Method E

### Step 1:

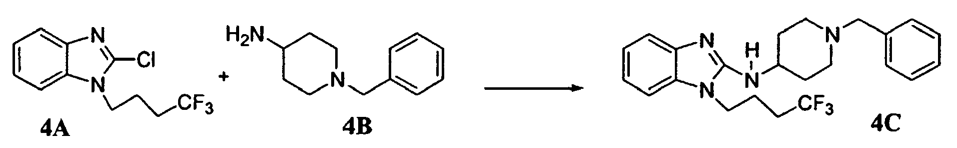

A mixture of neat **4A** (1.75g, 6.66 mmol) and **4B** (2.93g, 15.07 mmol) was stirred at 120 °C for 2 days, cooled to RT, treated with 1.0 M NaOH solution (30 ml), and extracted with EtOAc. The combined organic layers were washed with water and dried over Na₂SO₄. After evaporation to dryness, the crude residue was flash chromatographed (silica gel, 50% EtOAc in hexanes as eluent) to give 510 mg of **4C** (18%).

### Step 2:

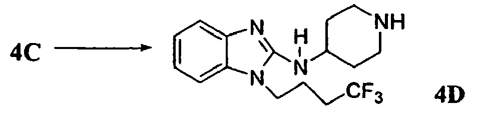

To a 500 ml pressure bottle was added **4C** (490 mg, 1.18 mmol) in CH₃OH (20 ml). Under N₂ stream, palladium hydroxide (300 mg, 20 wt.% on carbon) solid was added. The reaction mixture was shaken under 55 psi of hydrogen for 40 h and filtered. The filtrate was concentrated and dried on vacuum to deliver a yellow solid (340 mg, 88%).

### Step 3:

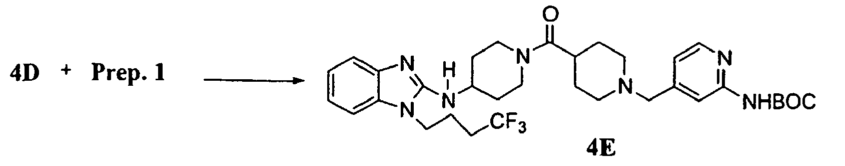

To a 50 ml round-bottomed flask were successively added **4D** (287 mg, 0.88 mmol), Preparation 1 (300 mg, 0.88 mmol), EDCI hydrochloride (210 mg, 1.10 mmol), HOBT (149 mg, 1.10 mmol), and diisopropylethylamine (228 mg, 1.76 mmol). DMF (3 ml) and CH₂Cl₂ (3 ml) were introduced via a syringe. The resulting reaction mixture was stirred at 70 °C for 15 h and cooled to RT. After addition of 1 N NaHCO₃ solution, the resulting mixture was extracted with CH₂Cl₂. The combined organic solutions were dried over Na₂SO₄ and concentrated. Purification of the crude product by flash chromatography on silica gel with 10% CH₃OH in CH₂Cl₂ as the eluent provided 4E as a solid (231 mg, 41 %).

### Step 4:

To a 25 ml round-bottomed flask was added **4E** (200 mg, 0.31 mmol) in CH₂Cl₂ (2.5 ml). TFA was then introduced via a syringe. The resulting solution was stirred at RT for 15 h, diluted with CH₂Cl₂, neutralized with 1.0 M NaOH solution, and separated. The organic solution was washed with water and dried over Na₂SO₄. After evaporation of the solvent, the crude product was purified on a preparative TLC plate with 10% CH₃OH in CH₂Cl₂ as the eluent to provide the title compound as a white solid (85 mg, 50%). MS: 544 (MH⁺).

### Example 5

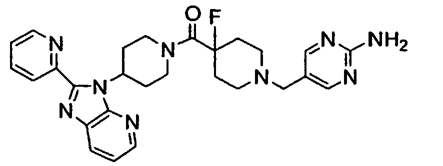

### Step 1:

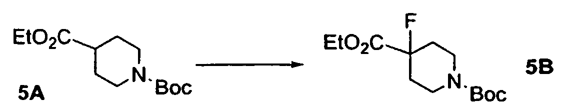

A solution of compound **5A** (100g, 0.389 mol) in THF (400 ml) was added dropwise over 1.0 h to a solution of LDA (233 mL, 2.0 M in THF/heptane/ethylbenzene, 0.466 mol) in THF (300ml) at 0 °C. The red-orange solution was stirred at 0 °C for 30 min, and then transferred by cannula to a pre-cooled (0 °C) solution of N-fluorobenzenesulfonimide (153 g, 0.485 mol) in dry THF (600 ml). The reaction mixture was stirred at 0 °C for 30 min, and then at 20 °C for 18 h. The total solvent volume was reduced to approximately one third, and EtOAc (1I) was added. The solution was washed successively with water, 0.1 N aq. HCl, saturated aq. NaHCO₃, and brine. The organic layer was dried over MgSO₄, filtered, and concentrated under reduced pressure to yield a crude liquid. Separation by flash chromatography (6:1 hexanes-EtOAc) gave compound 5B (93.5 g, 87%).

### Step 2:

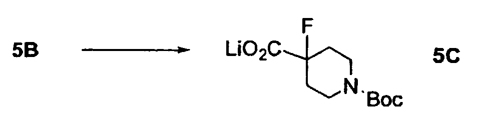

A solution of **5B** (50g, 0.181 mol) in THF (300 ml) and CH₃OH (200 ml) was treated with a solution of LiOH-H₂O (9.2 g, 0.218 mol) in water (100 ml) and then heated to 45 °C for 6 h. The mixture was then concentrated and dried in vacuo to provide 5C (45 g, 100%).

### Step 3:

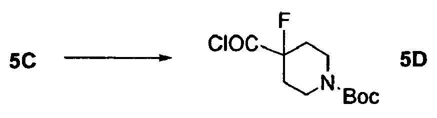

Compound 5C (20.4 g, 0.081 mol) was added slowly to a stirred flask of CH₂Cl₂ (250 ml) at 20 °C. The resulting white slurry was cooled to 0 °C and treated slowly with oxalyl chloride (6.7 ml, 0.075 mol) and a drop of DMF. After stirring at 20 °C for 0.5 h, the mixture was concentrated and dried in vacuo to provide **5D.**

### Step 4A:

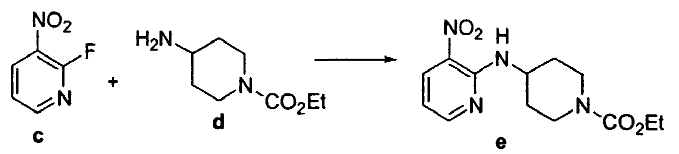

A mixture of **c** (64 g, 0.40 mol), **d** (84 ml, 0.52 mol), and K₂CO₃ (66 g, 0.48 mol) in anhydrous toluene (350 ml) was heated at reflux overnight. The reaction mixture was diluted with CH₂Cl₂, washed three times with 5% aqueous NaOH, dried over Na₂SO₄, and concentrated. Recrystallization with MeOH provided e (121 g, ∼100%) as a yellow solid.

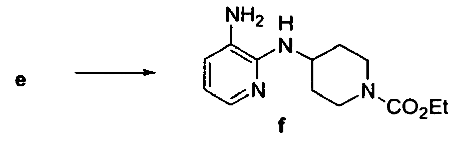

A suspension of e (121 g, 0.41 mol) and Raney Nickel (10 g) in EtOH (400 ml) was shaken under H₂ (40 psi) for 4 h. The mixture was filtered through a short pad of Celite (washing with CH₃OH). The filtrate was concentrated and dried in vacuo to provide **f** (109 g, ∼100%) as a dark brown solid.

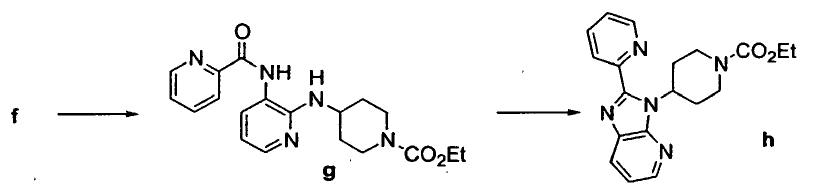

A solution of **f** (109 g, 0.41 mol) in CH₂Cl₂-DMF (1:1, 500 ml) was treated with picolinic acid (61 g, 0.50 mol), EDCI (119 g, 0.62 mol), HOBt (84 g, 0.62 mol) and iPr₂NEt (141 ml, 1.03 mol). The mixture was stirred at 70 °C for 6 h and then overnight at 20 °C. The reaction mixture was diluted with EtOAc, washed 3 times with 5% aqueous NaOH, dried over Na₂SO₄, and concentrated. Flash chromatography (0-100% EtOAc/hexane) provided **g** (131 g, 86%).

A solution of **g** (131 g, 0.36 mol) in AcOH (200 ml) was heated at 120 °C overnight. The reaction mixture was cooled, carefully basified with 5% aqueous NaOH and extracted with CH₂Cl₂. The combined organic extracts were dried over Na₂SO₄ and concentrated. Flash chromatography (0-80% EtOAc/hexane) provided **h** (95 g, 76%) as a yellow solid.

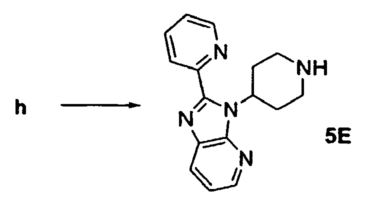

A solution of h (95 g, 0.27 mol) in anhydrous CHCl₃ (300 ml) was treated with iodotrimethylsilane (272 g, 1.36 mol) and heated at 70 °C for 5 h. The reaction mixture was cooled, quenched with cold 10% aqueous NaOH, and extracted with CH₂Cl₂. The combined organic extracts were dried over Na₂SO₄ and concentrated. Flash chromatography (2N NH₃-CH₃OH/EtOAc) provided 5E (43 g, 57%) as a pale yellow solid.

### Step 4B:

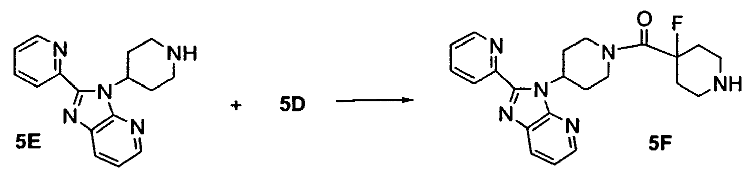

A mixture of **5D** (0.075 mol) in CH₂Cl₂ (250 ml) was treated with **5E** (15 g, 0.054 mol) and iPr₂NEt (25 ml, 0.135 mol) while maintaining a temperature of 20 °C. After 1 h, the mixture was concentrated and then stirred in CH₃OH (200 ml)/CH₂Cl₂ (200 ml)/H₂O (1 ml) for 1 h at 20 °C. The solvent was then evaporated. Treatment with TFA (200 ml) in CH₂Cl₂ (250 ml) at 20 °C followed by flash chromatography (0-7% 7N NH₃-CH₃OH/CH₂Cl₂) provided **5F** (80-90% from **5C**).

### Step 5:

### Method A:

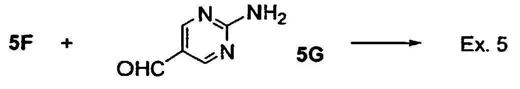

A solution of **5F** (0.41 g, 1.0 mmol) in CH₂Cl₂ (20 ml) was treated with **5G** (0.31 g, 2.5 mmol, JP Patent 63227573, 1988), NaBH(OAc)₃ (0.53 g, 2.5 mmol) and few drops of AcOH and then stirred overnight at 20 °C. The mixture was partitioned between 10% NaOH and CH₂Cl₂. The organic layer was dried with Na₂SO₄ and concentrated. Flash chromatography (0-5% 7N NH₃-CH₃OH/CH₂Cl₂) provided the title compound (0.45g, 87%). MS: 516 (M+H).

### Method B:

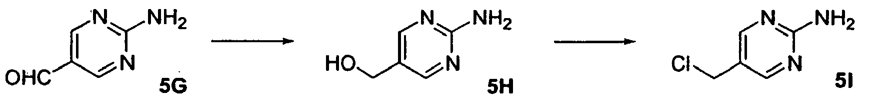

A solution of **5G** (50 g, 0.41 mol) in CH₃OH (300 ml) was cooled to 0 °C and carefully treated with NaBH₄ (20g, 0.53 mol in 6 batches) over 20 min. The reaction was then allowed to warm to 20 °C and was stirred for 4 h. The mixture was again cooled to 0 °C, carefully quenched with saturated aqueous NH₄Cl, and concentrated. Flash chromatography (5-10% 7N NH₃-CH₃OH/CH₂Cl₂) provided **5H** (31 g, 62%) as a light yellow solid.

A slurry of **5H** (31 g, 0.25 mol) in CH₂Cl₂ (500 ml) was cooled to 0 °C and slowly treated with SOCI₂ (55ml, 0.74 mol over 30 min). The reaction was then stirred overnight at 20 °C. The material was concentrated, slurried in acetone, and then filtered. The resulting beige solid **5I** was dried overnight in vacuo (38.4g, 52%, HCl salt).

A homogeneous solution of **5F** (16.4 g, 40 mmol) in anhydrous DMF (200 ml) was cooled to 0 °C, carefully treated with NaH (8g, 200 mmol), and stirred at 20 °C for 20 min. The reaction mixture was then cooled to 0 °C, treated with Nal (6g, 40 mmol) and **5I** (14.5g, 80 mmol), and then stirred overnight at 20 °C. The reaction was diluted with CH₂Cl₂ (500 ml), washed with 1 N aqueous NaOH, washed with brine, filtered through Celite, and concentrated. Flash chromatography (0-4% 7N NH₃-CH₃OH/CH₂Cl₂) provided Ex. 5 (16.9g, 82%) as a beige solid.

### Example 6

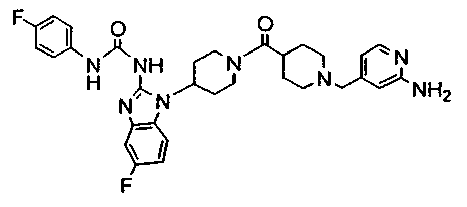

### Step 1:

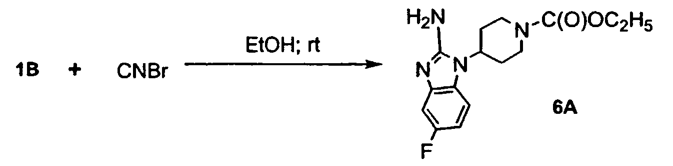

To a stirred solution of diamine **1B** (1.0g, 3.55 mmol) in C₂H₅OH (25 ml), at RT was added portionwise solid CNBr (564 mg; 5.33 mmol). The resultant solution was allowed to stir at RT for 5 days before removing solvent under vacuum. The residual oil was partitioned between EtOAc (30 ml) and 2M Na₂CO₃ (10 ml). The aqueous layer was adjusted to pH ∼10 by addition of a few drops of 6N NaOH and was then reextracted with EtOAc (2 x 10 ml). Combined extracts were washed with brine (5 ml) and filtered through anhydrous MgSO₄. The filtrate was stripped in vacuo to obtain compound **6A** as brown powder (1.03 g; 94%) sufficiently pure for use without purification. FABMS: 307 (MH⁺; 100%).

### Step 2:

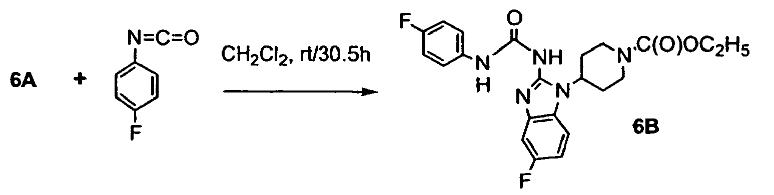

In a dry flask, under an inert atmosphere, a mixture of compound **6A** (369 mg; 1.20 mmol) and CH₂CI₂ (11 ml) was stirred and sonicated until the formation of a clear, amber solution to which was added via syringe 4-fluorophenyl isocyanate (158 microliters; 190 mg; 1.38 mmol). After 30.5 h at RT, a few drops of CH₃OH were added to the reaction solution, and solvent was removed under vacuum. The residual solid was dissolved in boiling Et₂O (∼30 ml). Insoluble matter was filtered, and the filtrate was diluted to a volume of -60 ml with hot hexanes. The solution was concentrated on a steam bath to a volume of ∼30 ml, by which point precipitation had begun. The mixture was allowed to stand at RT for ∼3 h. Filtration and washing with Et₂O-hexanes (1:1 v/v) yielded compound 6B as a reddish-brown powder (394 mg; 74%). FABMS: 444 (MH⁺; 100%). Although TLC and NMR indicated the presence of minor impurities, the product was sufficiently pure for use in Step 3 below.

### Step 3:

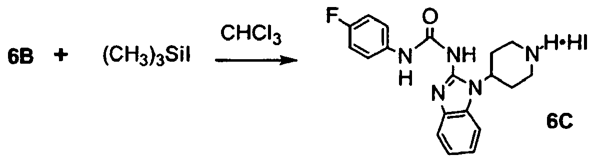

To a stirred suspension of compound 6B (333 mg; 0.751 mmol) in CHCl₃ (2 ml), contained in a flask equipped for reflux under an inert atmosphere, was added via syringe (CH₃)₃Sil (214 microliters; 301 mg; 1.51 mmol). Solids dissolved rapidly to produce a dark reddish-brown solution. Stirring was continued at RT for 20 min before placing the reaction mixture in an oil bath preheated to 50 °C. After 5 h at 50 °C, a second portion of (CH₃)₃Sil (54 microliters; 75 mg; 0.378 mmol) was added and heating continued at 50 °C for another 2.5 h. The reaction mixture (consisting of solid and solution phases) was removed from the heating bath and was treated with CH₃OH (2.5 ml) added in two portions. The reaction mixture was stirred and warmed to 50 °C for a few minutes, allowed to cool and was then filtered. Collected solids were washed with 1:1 (v/v) CH₃OH-EtOAc to obtain the hydriodide salt form of 6C as a pale reddish-brown powder (356 mg) wich was used in the next step without further purification. FABMS: 372 (MH⁺; 100%).

### Step 4:

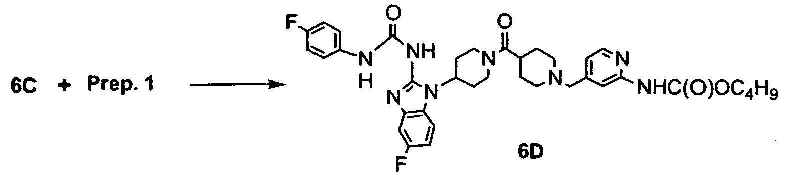

To a stirred suspension of **6C** (340 mg; 0.681 mmol), Prep. 1 (228 mg; 0.681 mmol), HOBT (9.2 mg; 0.0681 mmol) and NEt₃ (379 microliters; 275 mg; 2.72 mmol) in DMF (13 ml) was added solid EDCI (163 mg; 0.851 mmol). The cloudy reaction mixture was placed in a preheated oil bath and was stirred at 50 °C for 30 min, after which the resultant clear, amber solution was stirred for 23.5 h at RT. A few drops of water were added, and the reaction mixture was concentrated at 60 °C under vacuum. The concentrate was partitioned between EtOAc (20 ml) and water (5 ml)-brine (2.5 ml). The aqueous phase was extracted with EtOAc (2 x 5 ml). Combined extracts were washed with brine (2.5 ml) and filtered through anhydrous MgSO₄. The filtrate was evaporated under vacuum, and the residue was purified by flash chromatography on silica gel, eluting with a gradient of CH₂Cl₂-CH₃OH-NH₄OH (97:3:0.5 -> 96:4:0.5). Product **6D** (222 mg; 47%) was obtained as pale yellow powder. FABMS: 689 (MH⁺; ∼93%); 578 (∼58%); 478 (100%).

### Step 5:

To a solution of **6D** (208 mg; 0.302 mmol) in CH₂Cl₂ (3 ml) was added TFA (928 microliters; 1.37 g; 12.1 mmol) with swirling of the flask, which was then flushed with dry N₂, sealed and allowed to stand at RT for 6 h. The reaction solution was evaporated under vacuum, and the residue was partitioned between EtOAc (20 ml) and 2M Na₂CO₃ (3 ml) plus sufficient water to produce two clear phases. The aqueous phase was extracted with EtOAc (3 x 5 ml). Combined extracts were washed with brine (3 ml) and filtered through anhydrous MgSO₄. The filtrate was stripped of solvent in vacuo, and the residue was subjected to flash chromatography on silica gel, eluting with CH₂Cl₂-CH₃OH-NH₄OH (97:3:0.5). The title compound (130 mg; 72%) was obtained as pale yellow powder. FABMS: 589 (MH⁺; ∼64%); 478 (100%).

Using procedures similar to those described above, employing the appropriate starting materials, compounds in the following tables are prepared:

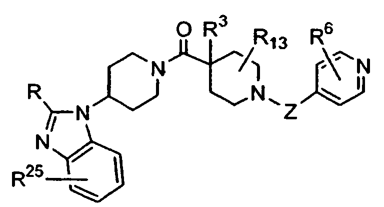

| No. | R | R²⁵ | R³ | R¹³ | Z | R⁶ | Physical Data MS (MH⁺) |
|---|---|---|---|---|---|---|---|
| 7 | -CH₃ | 5-OCH₃ | H | H | -CH₂- | 2-NH₂ | 463 |
| 8 | -CH₃ | 6-Cl | H | H | -CH₂- | 2-NH₂ | 467 |
| 9 | -CH₃ | 5-Cl | H | H | -CH₂- | 2-NH₂ | 467 |
| 10 | -CH₃ | 5-Br | H | H | -CH₂- | 2-NH₂ | 512 |
| 11 | 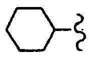 | 5-CI | H | H | -CH₂- | 2-NH₂ | 535 |
| 12 | benzyl | 5-F | H | H | -CH₂- | 2-NH₂ | 527 |
| 13 | -CH(CH₃)₂ | 5-Br | H | H | -CH₂- | 2-NH₂ | 540 |
| 14 | -CH₂NH₂ | H | H | H | -CH₂- | 2-NH₂ | 488 |
| 15 | -CH₂NHSO₂CH₃ | H | H | H | -CH₂- | 2-NH₂ | 526 |
| 16 | -CH₂NHC(O)CH₃ | 5-CI | H | H | -CH₂- | 2-NH₂ | 524 |
| 17 | -CH₂OCH₃ | 5-F | H | H | -CH₂- | 2-NH₂ | 481 |
| 18 | -CH₂NH₂ | 5-CI | H | H | -CH₂- | 2-NH₂ | 482 |
| 19 | -CH₂OCH₃ | 6,7-di-F | H | H | -CH₂- | 2-NH₂ | 499 |
| 20 | 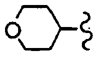 | 6-F | H | H | -CH₂- | 2-NH₂ | 521 |
| 21 | 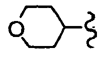 | 5-F | H | H | -CH₂- | 2-NH₂ | 521 |
| 22 | 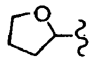 | 6-F | H | H | -CH₂- | 2-NH₂ | 507 |
| 23 | 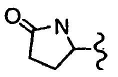 | 5-F | H | H | -CH₂- | 2-NH₂ | 520 |
| 24 | 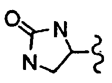 | 5-F | H | H | -CH₂- | 2-NH₂ | 521 |
| 25 | 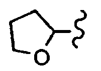 | 5-Br | H | H | -CH₂- | 2-NH₂ | 568 |
| 26 | 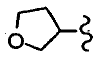 | 5-F | H | H | -CH₂- | 2-NH₂ | 507 |
| 27 | 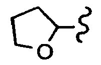 | 5-F | H | H | -CH₂- | 2-NH₂ | 507 |
| 28 | 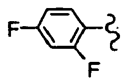 | H | H | H | -CH₂- | 2-NH₂ | 531 |
| 29 | 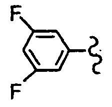 | 5-F | H | H | -CH₂- | 2-NH₂ | 549 |
| 30 | 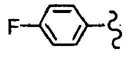 | 6-F | H | H | -CH₂- | 2-NH₂ | 531 |
| 31 | 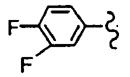 | 6,7-di-F | H | H | -CH₂- | 2-NH₂ | 567 |
| 32 | 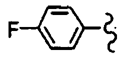 | 6-CI | H | H | -CH₂- | 2-NH₂ | 547 |
| 33 | 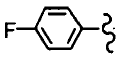 | 5-F | H | H | -CH₂- | 2-NH₂ | 531 |
| 34 | 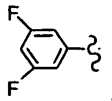 | 5-Cl | H | H | -CH₂- | 2-NH₂ | 565 |
| 35 | 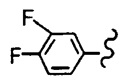 | H | H | H | -CH₂- | 2-NH₂ | 531 |
| 36 | 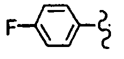 | 5-Cl | H | H | -CH₂- | 2-NH₂ | 547 |
| 37 | 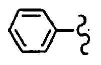 | 5-Cl | H | H | -CH₂- | 2-NH₂ | 529 |
| 38 | 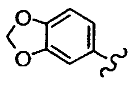 | 6-F | H | H | -CH₂- | 2-NH₂ | 557 |
| 39 | 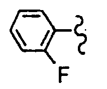 | 5-Br | H | H | -CH₂- | 2-NH₂ | 592 |
| 40 | 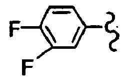 | 5-Br | H | H | -CH₂- | 2-NH₂ | 610 |
| 41 | 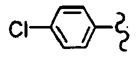 | 5-F | H | H | -CH₂- | 2-NH₂ | 547 |
| 42 | 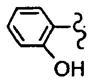 | 5-F | H | H | -CH₂- | 2-NH₂ | 529 |
| 43 | 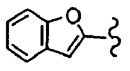 | 6-F | H | H | -CH₂- | 2-NH₂ | 553 |
| 44 | 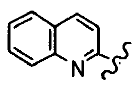 | 6-F | H | H | -CH₂- | 2-NH₂ | 564 |
| 45 | 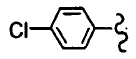 | H | H | H | -CH₂- | 2-NH₂ | 529 |
| 46 | 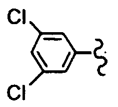 | 5-F | H | H | -CH₂- | 2-NH₂ | 581 |
| 47 | 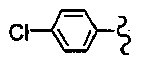 | 5-Cl | H | H | -CH₂- | 2-NH₂ | 563 |
| 48 | 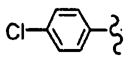 | 6-Cl | H | H | -CH₂- | 2-NH₂ | 563 |
| 49 | 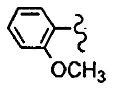 | 5-F | H | H | -CH₂- | 2-NH₂ | 543 |
| 50 | 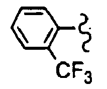 | 5-F | H | H | -CH₂- | 2-NH₂ | 581 |
| 51 | 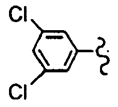 | 5-Cl | H | H | -CH₂- | 2-NH₂ | 597 |
| 52 | 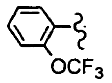 | 5-F | H | H | -CH₂- | 2-NH₂ | 597 |
| 53 | 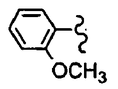 | 5-Br | H | H | -CH₂- | 2-NH₂ | 604 |
| 54 | 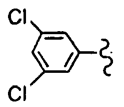 | 6-Cl | H | H | -CH₂- | 2-NH₂ | 597 |
| 55 | 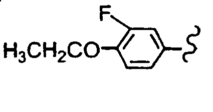 | 5-CH₃ | H | H | -CH₂- | 2-NH₂ | 571 |
| 56 | 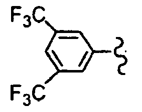 | 5-Cl | H | H | -CH₂- | 2-NH₂ | 665 |
| 57 | 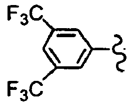 | 5-Br | H | H | -CH₂- | 2-NH₂ | 710 |
| 58 | 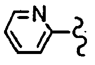 | 6-ethoxy | H | H | -CH₂- | 2-NH₂ | 540 |
| 59 | 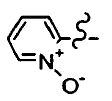 | 5-Cl | H | H | -CH₂- | 2-NH₂ | 546 |
| 60 | 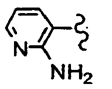 | H | H | H | -CH₂- | 2-NH₂ | 511 |
| 61 | 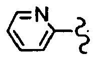 | 5-F | H | H | -CH₂- | H | 499 |
| 62 | 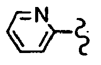 | 6-Cl | H | H | -CH₂- | 2-NH₂ | 530 |
| 63 | 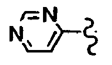 | 5-F | H | H | -CH₂- | 2-NH₂ | 515 |
| 64 | 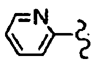 | 6-F | H | H | -CH₂- | 2-NH₂ | 514 |
| 65 | 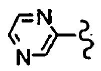 | 6-F | H | H | -CH₂- | 2-NH₂ | 515 |
| 66 | 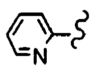 | 7-Cl | H | H | -CH₂- | 2-NH₂ | 531 |
| 67 | 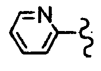 | H | H | H | -CH₂- | 2-NH₂ | 496 |
| 68 | 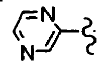 | 5-F | H | H | -CH₂- | 2-NH₂ | 515 |
| 69 | 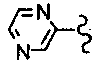 | 5-Cl | H | H | -CH₂- | 2-NH₂ | 531 |
| 70 | 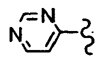 | 5-Cl | H | H | -CH₂- | 2-NH₂ | 531 |
| 71 | 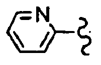 | 5,6-di-F | H | H | -CH₂- | 2-NH₂ | 532 |
| 72 | 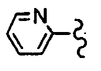 | 5-Br | H | H | -CH₂- | 2-NH₂ | 575 |
| 73 | 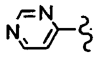 | 6-ethoxy | H | H | -CH₂- | 2-NH₂ | 541 |
| 74 | 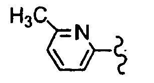 | 5-F | H | H | -CH₂- | 2-NH₂ | 528 |
| 75 | 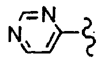 | 6-F | H | H | -CH₂- | 2-NH₂ | 515 |
| 76 | 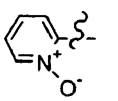 | 5-Br | H | H | -CH₂- | 2-NH₂ | 591 |
| 77 | 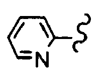 | 5-Cl | H | H | -CH₂- | 2-NH₂ | 530 |
| 78 | 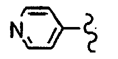 | 5-Cl | H | H | -CH₂- | 2-NH₂ | 530 |
| 79 | 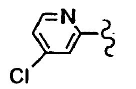 | 5-F | H | H | -CH₂- | 2-NH₂ | 548 |
| 80 | 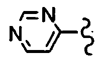 | 5-CF₃ | H | H | -CH₂- | 2-NH₂ | 565 |
| 81 | 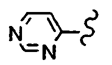 | H | H | H | -CH₂- | 2-NH₂ | 497 |
| 82 | 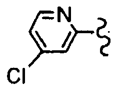 | 6,7-di-F | H | H | -CH₂- | 2-NH₂ | 567 |
| 83 | 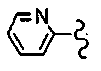 | 6,7-di-F | H | H | -CH₂- | 2-NH₂ | 532 |
| 84 | 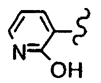 | 5-F | H | H | -CH₂- | 2-NH₂ | 530 |
| 85 | 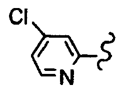 | 5-CF₃,7-F | H | H | -CH₂- | 2-NH₂ | 617 |
| 86 | 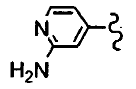 | 5-F | H | H | -CH₂- | 2-NH₂ | 529 |
| 87 | 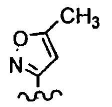 | H | H | H | -CH₂- | 2-NH₂ | 500 |
| 88 | 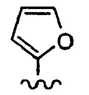 | H | H | H | -CH₂- | 2-NH₂ | 485 |
| 89 | 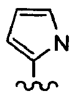 | H | H | H | -CH₂- | 2-NH₂ | 489 |
| 90 | 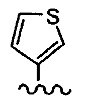 | 6-F | H | H | -CH₂- | 2-NH₂ | 514 |
| 91 | 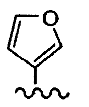 | 6-F | H | H | -CH₂- | 2-NH₂ | 503 |
| 92 | 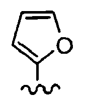 | 5-F | H | H | -CH₂- | 2-NH₂ | 503 |
| 93 | 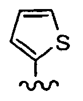 | H | H | H | -CH₂- | 2-NH₂ | 501 |
| 94 | 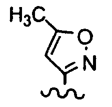 | 5-F | H | H | -CH₂- | 2-NH₂ | 518 |
| 95 | 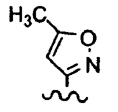 | 5-Cl | H | H | -CH₂- | 2-NH₂ | 534 |
| 96 | 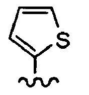 | 5-F | H | H | -CH₂- | 2-NH₂ | 519 |
| 97 | 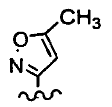 | 6,7-di-F | H | H | -CH₂- | 2-NH₂ | 536 |
| 98 | 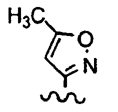 | 5-Br | H | H | -CH₂- | 2-NH₂ | 579 |
| 99 | 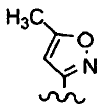 | 6-ethoxy | H | H | -CH₂- | 2-NH₂ | 544 |
| 100 | 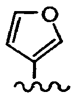 | 5-F | H | H | -CH₂- | 2-NH₂ | 503 |
| 101 | 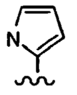 | 5-Br | H | H | -CH₂- | 2-NH₂ | 563 |
| 102 | 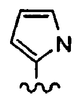 | 5-F | H | H | -CH₂- | 2-NH₂ | 502 |
| 103 | 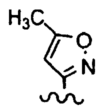 | 5-CF₃ | H | H | -CH₂- | 2-NH₂ | 568 |
| 104 | 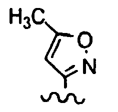 | 5-CF₃,7-F | H | H | -CH₂- | 2-NH₂ | 586 |
| 105 | 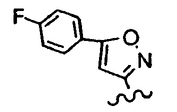 | 5-F | H | H | -CH₂- | 2-NH₂ | 598 |
| 106 | 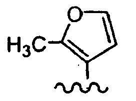 | 5-F | H | H | -CH₂- | 2-NH₂ | 517 |
| 107 | 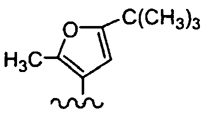 | 5-F | H | H | -CH₂- | 2-NH₂ | 573 |
| 108 | 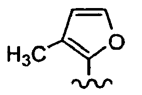 | 5-F | H | H | -CH₂- | 2-NH₂ | 517 |
| 109 | CH₃-S- | 5-F | H | H | -CH₂- | 2-NH₂ | 483 |
| 110 | CH₃-CH₂-S- | 5-F | H | H | -CH₂- | 2-NH₂ | 497 |
| 111 | CH₃-SO₂- | 5-F | H | H | -CH₂- | 2-NH₂ | 515 |
| 112 | 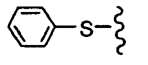 | 5-F | H | H | -CH₂- | 2-NH₂ | 545 |
| 113 | 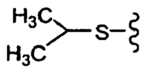 | 5-F | H | H | -CH₂- | 2-NH₂ | 511 |
| 114 | 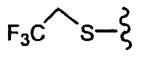 | 5-F | H | H | -CH₂- | 2-NH₂ | 551 |
| 115 | 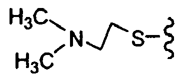 | 5-F | H | H | -CH₂- | 2-NH₂ | 540 |
| 116 | HS- | 5-F | H | H | -CH₂- | 2-NH₂ | 469 |
| 117 | CH₃-S- | 5-F | H | 2-CH₃ | -CH₂- | 2-NH₂ | 497 |
| 118 | CH₃-S- | 5-F | F | H | -CH₂- | 2-NH₂ | 501 |
| 119 | 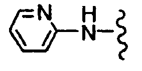 | 5-F | H | H | -CH₂- | 2-NH₂ | 529 |
| 120 | 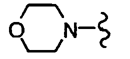 | 5-F | H | H | -CH₂- | 2-NH₂ | 522 |
| 121 | 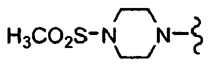 | 5-F | H | H | -CH₂- | 2-NH₂ | 599 |
| 123 | 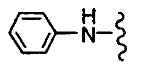 | 5-F | H | H | -CH₂- | 2-NH₂ | 528 |
| 124 | 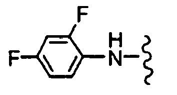 | 5-F | H | H | -CH₂- | 2-NH₂ | 564 |
| 125 | 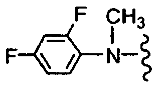 | 5-F | H | H | -CH₂- | 2-NH₂ | 578 |
| 126 | 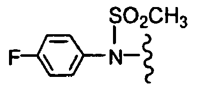 | 5-F | H | H | -CH₂- | 2-NH₂ | 624 |
| 127 | 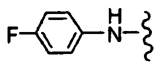 | 5-F | H | H | -CH₂- | 2-NH₂ | 546 |
| 128 | 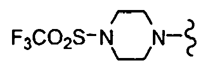 | 5-F | H | H | -CH₂- | 2-NH₂ | 653 |
| 129 | CH₃-O-(CH₂)₂-NH- | 5-F | H | H | -CH₂- | 2-NH₂ | 510 |
| 130 | 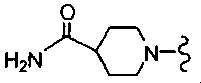 | 5-F | H | H | -CH₂- | 2-NH₂ | 563 |
| 131 | 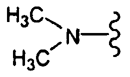 | 5-F | H | H | -CH₂- | 2-NH₂ | 480 |
| 132 | CH₃-O- | 5-F | H | H | -CH₂- | 2-NH₂ | 467 |
| 133 | CH₃-CH₂-O- | 5-F | H | H | -CH₂- | 2-NH₂ | 481 |
| 134 | CH₃-O-(CH₂)₂-O- | 5-F | H | H | -CH₂- | 2-NH₂ | 511 |
| 135 | (CH₃)₂-CH-O- | 5-F | H | H | -CH₂- | 2-NH₂ | 495 |
| 136 | 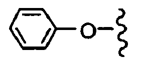 | 5-F | H | H | -CH₂- | 2-NH₂ | 529 |
| 137 | 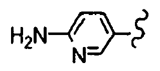 | H | H | H | -CH₂- | 2-NH₂ | 511 |
| 138 | 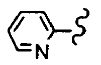 | 5-CF₃,7-F | H | H | -CH₂- | 2-NH₂ | 582 |
| 139 | 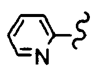 | 5-F | H | H | 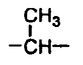 | 2-NH₂ | 528 |
| 140 | 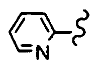 | 5-F | F | H | -CH₂- | 2-NH₂ | 532 |
| 141 | 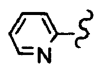 | 5-F | OH | H | -CH₂- | 2-NH₂ | 530 |
| 142 | 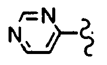 | 5-F | H | H | 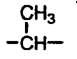 | 2-NH₂ | 529 |
| 143 | 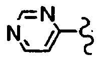 | 5-F | H | H | 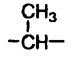 | 2-NH₂ | 529 |
| 144 | 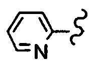 | 5-F | -CH₃ | H | -CH₂- | 2-NH₂ | 528 |
| 145 | 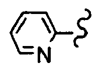 | 6-F | H | H | 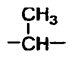 | 2-NH₂ | 528 |
| 146 | H | 5-F | H | H | -CH₂- | 2-NH₂ | 437 |
| 147 | 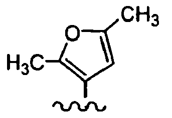 | 5-F | H | H | -CH₂- | 2-NH₂ | 531 |
| 148 | 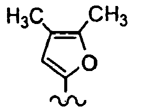 | 5-F | H | H | -CH₂- | 2-NH₂ | 531 |
| 149 | 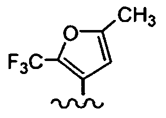 | 5-F | H | H | -CH₂- | 2-NH₂ | 585 |
| 150 | 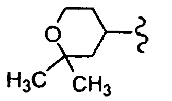 | 5-F | H | H | -CH₂- | 2-NH₂ | 549 |
| 151 | 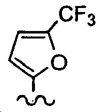 | 5-F | H | H | -CH₂- | 2-NH₂ | 571 |
| 152 | 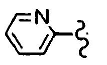 | H | F | H | -CH₂- | 2-NH₂ | 514 |
| 153 | (CH₃)₂N-(CH₂)₂-NH- | 5-F | H | H | -CH₂- | 2-NH₂ | 523 |
| 154 | CH₃-S- | 5-F | H | H | 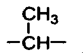 | 2-NH₂ | 497 |
| 155 | 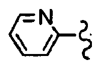 | 5-F | H | 2-CH₃ | -CH₂- | 2-NH₂ | 528 |
| 156 | 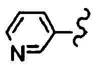 | 5-F | H | H | -CH₂- | 2-NH₂ | 514 |
| 157 | 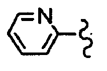 | 5-F | H | H | -CH₂- | 3-NH₂ | 514 |
| 158 | 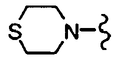 | 5-F | H | H | -CH₂- | 2-NH₂ | 589 |
| 159 | 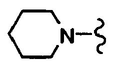 | 5-F | H | H | -CH₂- | 2-NH₂ | 520 |
| 160 | CH₃CH₂O- | 5-F | F | H | -CH₂- | 2-NH₂ | 499 |
| 161 | 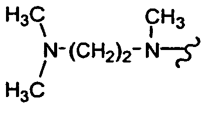 | 5-F | H | H | -CH₂- | 2-NH₂ | 537 |
| 162 | 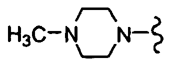 | 5-F | H | H | -CH₂- | 2-NH₂ | 535 |
| 163 | 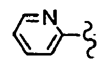 | 5-F | H | 5-OH | -CH₂- | 2-NH₂ | 530 |
| 164 | 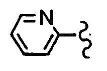 | 5-F | F | H | -CH₂- | 3-NH₂ | 532 |
| 165 | 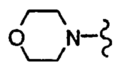 | 5-F | F | H | -CH₂- | 2-NH₂ | 540 |
| 166 | 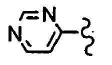 | 5-F | H | H | -CH₂- | 3-NH₂ | 515 |

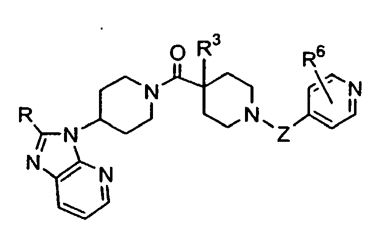

| No. | R | R³ | Z | R⁶ | Physical Data MS (MH⁺) |
|---|---|---|---|---|---|
| 167 | 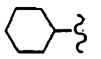 | H | -CH₂- | 2-NH₂ | 502 |
| 168 | -CH₂OCH₃ | H | -CH₂- | 2-NH₂ | 464 |
| 169 | 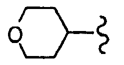 | H | -CH₂- | 2-NH₂ | 504 |
| 170 | 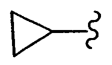 | H | -CH₂- | 2-NH₂ | 460 |
| 171 | (CH₃)₂-CH- | H | -CH₂- | 2-NH₂ | 462 |
| 172 | 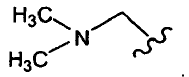 | H | -CH₂- | 2-NH₂ | 477 |
| 173 | 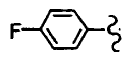 | H | -CH₂- | 2-NH₂ | 514 |
| 174 | 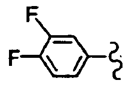 | H | -CH₂- | 2-NH₂ | 532 |
| 175 | 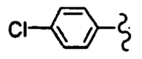 | H | -CH₂- | 2-NH₂ | 530 |
| 176 | 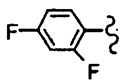 | H | -CH₂- | 2-NH₂ | 532 |
| 177 | 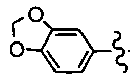 | H | -CH₂- | 2-NH₂ | 540 |
| 178 | 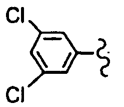 | H | -CH₂- | 2-NH₂ | 564 |
| 179 | 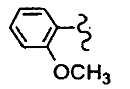 | H | -CH₂- | 2-NH₂ | 526 |
| 180 | 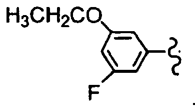 | H | -CH₂- | 2-NH₂ | 558 |
| 181 | 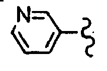 | H | -CH₂- | 2-NH₂ | 497 |
| 182 | 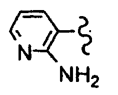 | H | -CH₂- | 2-NH₂ | 512 |
| 183 | 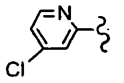 | H | -CH₂- | 2-NH₂ | 531 |
| 184 | 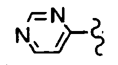 | H | -CH₂- | 2-NH₂ | 498 |
| 185 | 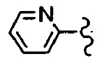 | H | -CH₂- | 2-NH₂ | 497 |
| 186 | 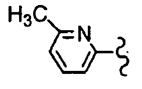 | H | -CH₂- | 2-NH₂ | 511 |
| 187 | 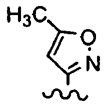 | H | -CH₂- | 3-NH₂ | 501 |
| 188 | 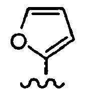 | H | -CH₂- | 2-NH₂ | 486 |
| 189 | 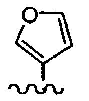 | H | -CH₂- | 2-NH₂ | 486 |
| 190 | 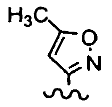 | H | -CH₂- | 2-NH₂ | 501 |
| 191 | 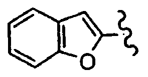 | H | -CH₂- | 2-NH₂ | 536 |
| 192 | 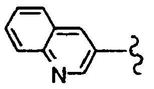 | H | -CH₂- | 2-NH₂ | 547 |
| 193 | 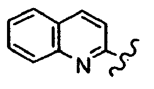 | H | -CH₂- | 2-NH₂ | 547 |
| 194 | 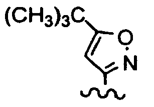 | H | -CH₂- | 2-NH₂ | 543 |
| 195 | 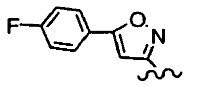 | H | -CH₂- | 2-NH₂ | 581 |
| 196 | 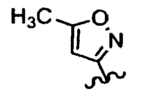 | F | -CH₂- | 2-NH₂ | 519 |
| 197 | 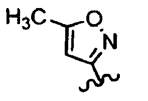 | H | 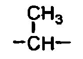 | 2-NH₂ | 515 |
| 198 | 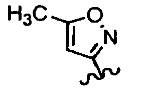 | OH | -CH₂- | 2-NH₂ | 517 |
| 199 | 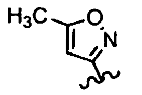 | 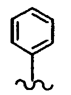 | -CH₂- | 2-NH₂ | 577 |
| 200 | 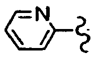 | F | -CH₂- | 2-NH₂ | 515 |
| 201 | 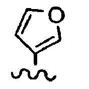 | F | -CH₂- | 2-NH₂ | 504 |
| 202 | 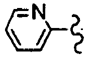 | H | -CH₂- | 3-NH₂ | 497 |
| 203 | 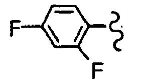 | H | -CH₂- | 3-NH₂ | 532 |
| 204 | 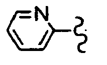 | F | -CH₂- | 3-NH₂ | 515 |
| 205 | 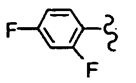 | F | -CH₂- | 3-NH₂ | 550 |

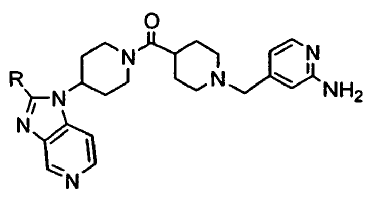

| No. | R | Physical Data MS (MH⁺) |
|---|---|---|
| 206 | -CH₃ | 434 |
| 207 | 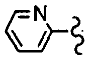 | 497 |
| 208 | 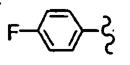 | 514 |
| 209 | 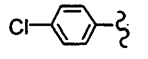 | 530 |

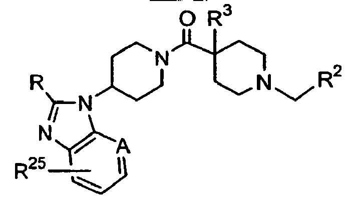

| No. | R | R²⁵ | A | R³ | R² | Physical Data MS (MH⁺) |
|---|---|---|---|---|---|---|
| 210 | 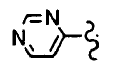 | 5-Cl | C | H | 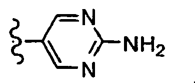 | 532 |
| 211 | 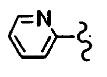 | 5-F | C | H | 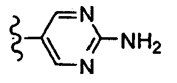 | 515 |
| 212 | 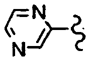 | 5-Cl | C | H | 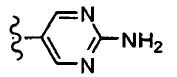 | 532 |
| 213 | 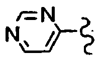 | 5-F | C | H | 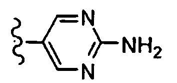 | 516 |
| 214 | 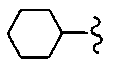 | H | N | H | 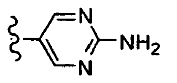 | 503 |
| 215 | 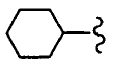 | H | N | H | 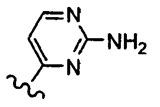 | 503 |
| 216 | (CH₃)₂CH- | H | N | H | 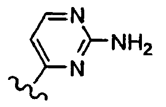 | 463 |
| 217 | 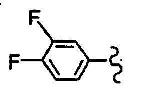 | 5-F | C | H | 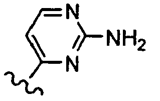 | 550 |
| 218 | 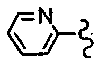 | 5-F | C | H | 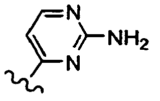 | 515 |
| 219 | 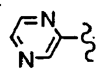 | 5-CI | C | H | 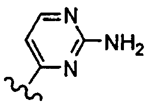 | 532 |
| 220 | 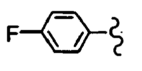 | 6-Cl | C | H | 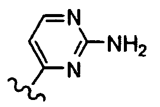 | 548 |
| 221 | 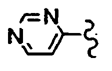 | 5-F | C | H | 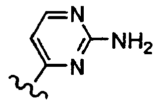 | 516 |
| 222 | 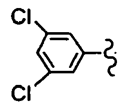 | 6-CI | C | H | 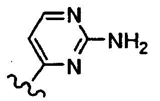 | 600 |
| 223 | 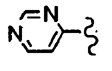 | 5-Cl | C | H | 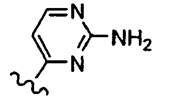 | 532 |
| 224 | 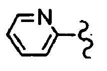 | 6-F | C | H | 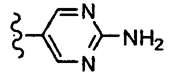 | 515 |
| 225 | 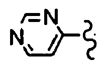 | H | N | H | 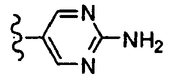 | 499 |
| 226 | 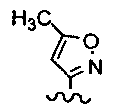 | H | N | H | 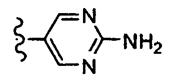 | 502 |
| 227 | 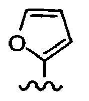 | H | N | H | 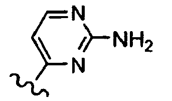 | 487 |
| 228 | 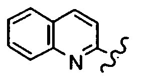 | H | N | H | 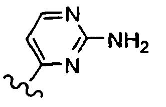 | 548 |
| 229 | 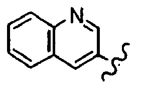 | H | N | H | 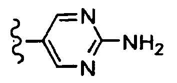 | 548 |
| 230 | 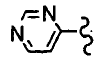 | H | N | H | 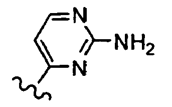 | 499 |
| 231 | 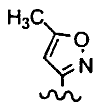 | H | N | H | 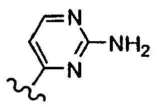 | 502 |
| 232 | 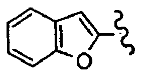 | H | N | H | 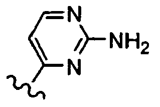 | 537 |
| 233 | 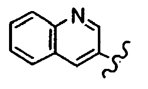 | H | N | H | 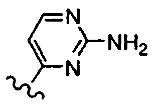 | 548 |
| 234 | 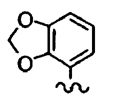 | H | N | H | 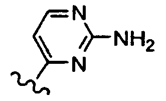 | 541 |
| 235 | 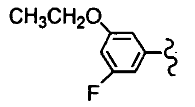 | H | N | H | 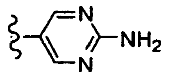 | 559 |
| 236 | 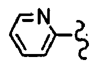 | H | N | H | 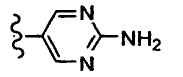 | 498 |
| 237 | 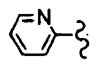 | 5-F | C | F | 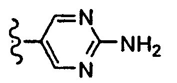 | 533 |
| 238 | 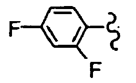 | 5-F | C | H | 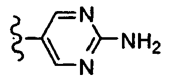 | 550 |
| 239 | 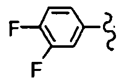 | 5-F | C | H | 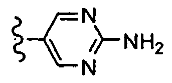 | 550 |
| 240 | 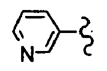 | 5-F | C | H | 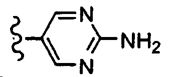 | 515 |
| 241 | 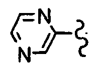 | 5-F | C | H | 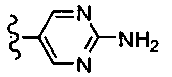 | 516 |
| 242 | 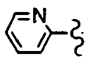 | H | C | H | 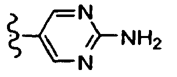 | 497 |
| 243 | (CH₃)₂N-CH₂- | H | N | H | 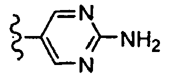 | 478 |
| 244 | 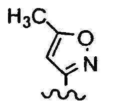 | 5-F | C | H | 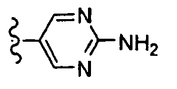 | 519 |
| 245 | 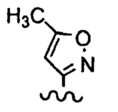 | H | C | H | 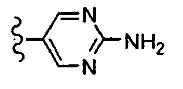 | 501 |
| 246 | 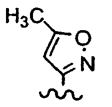 | 5,6-di-F | C | H | 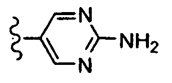 | 537 |
| 247 | 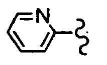 | 5-F | C | H | 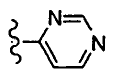 | 500 |
| 248 | 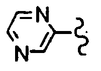 | 5,6-di-F | C | H | 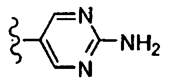 | 534 |
| 249 | 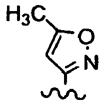 | 5-F | C | F | 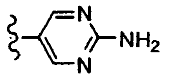 | 537 |
| 250 | 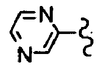 | 5-F | C | F | 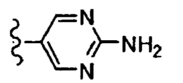 | 534 |
| 251 | 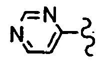 | 5-F | C | F | 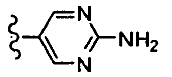 | 534 |
| 252 | 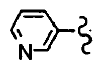 | 5-F | C | F | 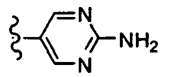 | 533 |
| 253 | 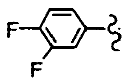 | 5-F | C | F | 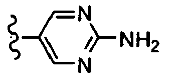 | 568 |
| 254 | 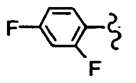 | 5-F | C | F | 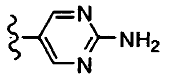 | 568 |
| 255 | 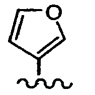 | H | N | H | 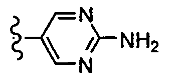 | 487 |
| 256 | 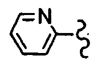 | H | C | F | 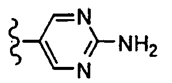 | 515 |
| 257 | 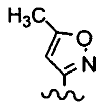 | H | C | F | 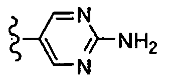 | 519 |
| 258 | 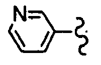 | H | N | F | 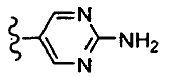 | 516 |
| 259 | 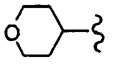 | H | N | H | 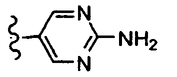 | 505 |
| 260 | 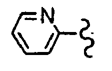 | H | N | F | 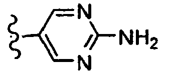 | 516 |
| 261 | 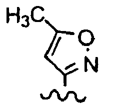 | H | N | F | 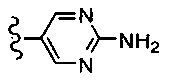 | 520 |
| 262 | 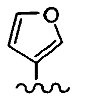 | 5-F | C | H | 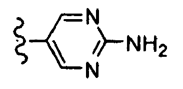 | 504 |
| 263 | 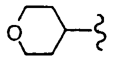 | 5-F | C | H | 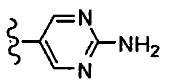 | 522 |
| 264 | 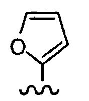 | 5-F | C | H | 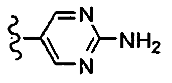 | 504 |
| 265 | 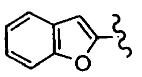 | H | N | H | 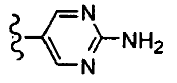 | 537 |
| 266 | (CH₃)₂N-CH₂- | H | N | F | 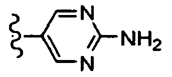 | 496 |
| 267 | 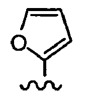 | H | N | F | 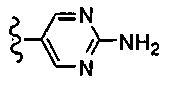 | 505 |
| 268 | CH₃CH₂-O- | 5-F | C | H | 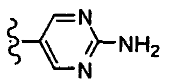 | 482 |
| 269 | CH₃-S- | 5-F | C | H | 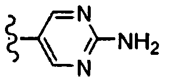 | 484 |
| 270 | CH₃CH₂-O- | 5-F | C | F | 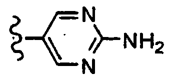 | 500 |
| 271 | 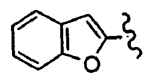 | H | N | F | 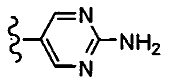 | 555 |
| 272 | 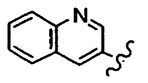 | H | N | F | 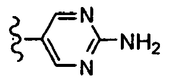 | 566 |
| 273 | 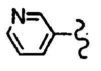 | H | N | H | 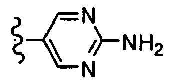 | 498 |
| 274 | 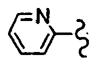 | 5,6-di-F | C | F | 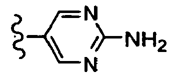 | 551 |
| 275 | 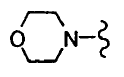 | 5-F | C | F | 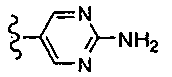 | 541 |
| 276 | 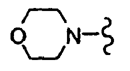 | 5-F | C | H | 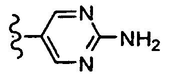 | 523 |
| 277 | 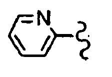 | 5-F | C | H | 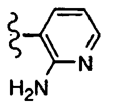 | 514 |
| 278 | 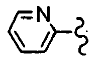 | 5-F | C | H | 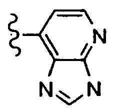 | 539 |
| 279 | 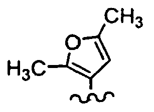 | H | N | H | 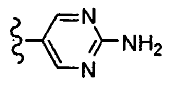 | 515 |
| 280 | 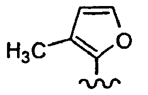 | H | N | H | 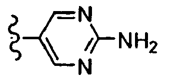 | 501 |
| 281 | 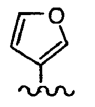 | H | N | F | 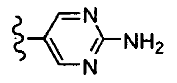 | 505 |
| 282 | 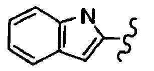 | H | N | H | 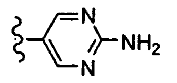 | 536 |
| 283 | 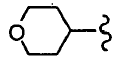 | H | N | F | 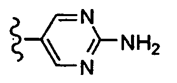 | 523 |
| 284 | 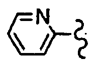 | 5-F | C | F | 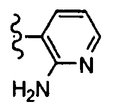 | 532 |
| 285 | 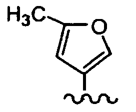 | H | N | H | 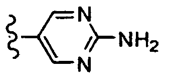 | 501 |
| 286 | 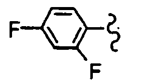 | H | N | H | 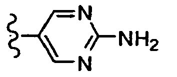 | 533 |
| 287 | 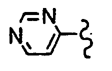 | H | N | F | 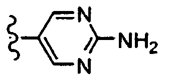 | 517 |
| 288 | 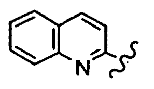 | H | N | H | 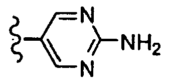 | 548 |
| 289 | 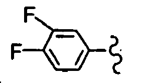 | H | N | H | 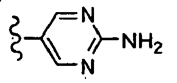 | 533 |
| 290 | CH₃S- | 5-F | C | F | 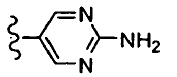 | 502 |
| 291 | 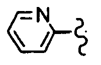 | H | N | F | 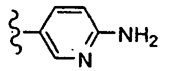 | 515 |
| 292 | 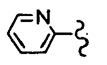 | 5-F | C | F | 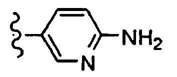 | 532 |
| 293 | 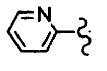 | 5-F | C | H | 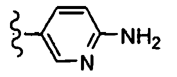 | 514 |
| 294 | 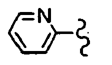 | H | N | H | 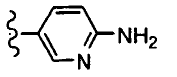 | 497 |
| 295 | (CH₃)₂N- | 5-F | C | F | 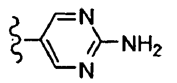 | 499 |
| 296 | CH₃CH₂-S- | 5-F | C | F | 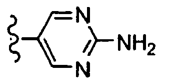 | 516 |
| 297 | CH₃-O- | 5-F | C | F | 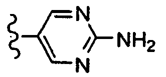 | 486 |
| 298 | 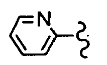 | H | N | H | 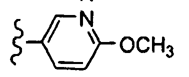 | 512 |
| 299 | 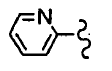 | H | N | F | 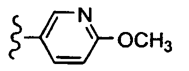 | 530 |
| 300 | 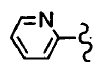 | 5-F | C | F | 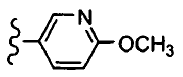 | 547 |
| 301 | 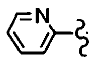 | 5-F | C | H | 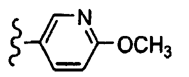 | 529 |
| 302 | 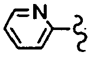 | 5-F | C | H | 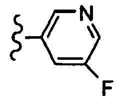 | 517 |
| 303 | 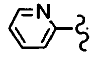 | 5-F | C | F | 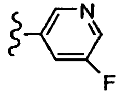 | 535 |
| 304 | 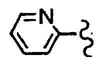 | H | N | H | 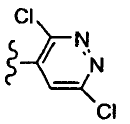 | 551 |
| 305 | 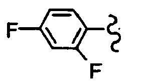 | H | N | F | 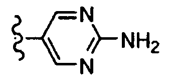 | 551 |
| 306 | 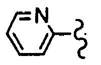 | 5-F | C | H | 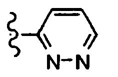 | 500 |
| 307 | 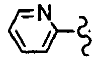 | 5-F | C | H | 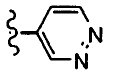 | 500 |
| 308 | 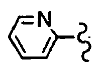 | 5-F | C | F | 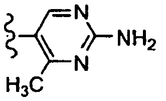 | 547 |
| 309 | (CH₃CH₂)₂N- | 5-F | C | F | 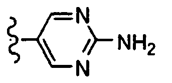 | 527 |
| 310 | 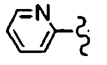 | H | N | H | 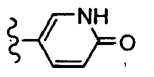 | 498 |
| 311 | 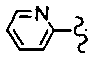 | H | N | F | 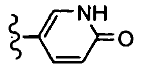 | 516 |
| 312 | 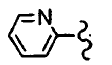 | 5-F | C | H | 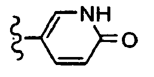 | 515 |
| 313 | 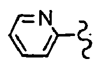 | 5-F | C | F | 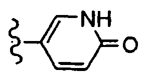 | 533 |
| 314 | 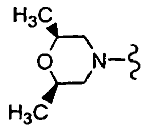 | 5-F | C | F | 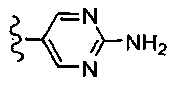 | 569 |
| 315 | CH₃-S- | H | N | F | 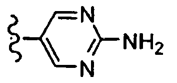 | 485 |
| 316 | CH₃CH₂-O- | H | N | F | 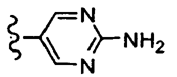 | 483 |
| 317 | 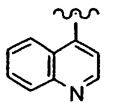 | H | N | F | 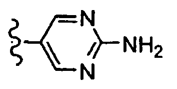 | 566 |
| 318 | 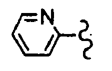 | H | N | F | 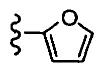 | 489 |
| 319 | 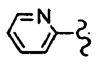 | H | N | F | 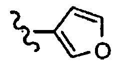 | 489 |
| 320 | 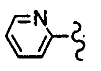 | H | N | F | 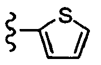 | 505 |
| 321 | 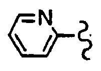 | H | N | F | 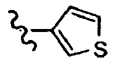 | 505 |
| 322 | 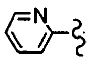 | 5-F | C | F | 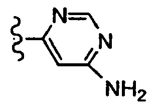 | 533 |
| 323 | 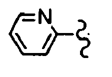 | H | N | F | 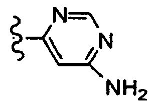 | 516 |
| 325 | 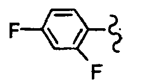 | H | N | F | 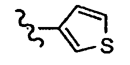 | 540 |
| 325 | 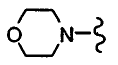 | H | N | F | 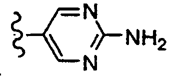 | 524 |
| 326 | (CH₃)₂CH-O- | 5-F | C | F | 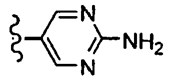 | 514 |
| 327 | 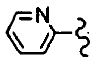 | H | N | F | 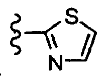 | 506 |
| 328 | 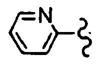 | H | N | F | 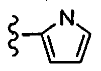 | 488 |
| 329 | 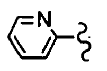 | H | N | F | 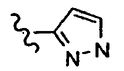 | 489 |
| 330 | 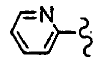 | H | N | F | 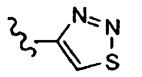 | 507 |
| 331 | 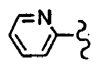 | H | N | F | 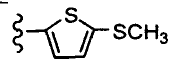 | 551 |
| 332 | 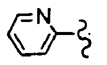 | H | N | F | 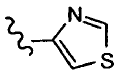 | 506 |
| 333 | 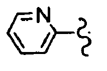 | H | N | F | 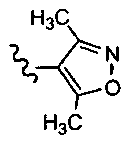 | 518 |
| 334 | 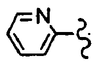 | H | N | F | 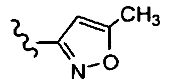 | 504 |
| 335 | CH₃-O- | H | N | F | 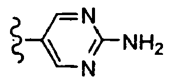 | 464 |
| 336 | 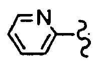 | H | N | F | 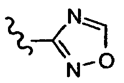 | 491 |
| 337 | 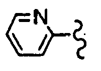 | H | N | F | 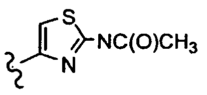 | 563 |
| 338 | 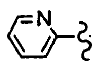 | 5-F | C | H | 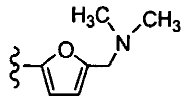 | 545 |
| 339 | 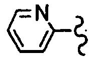 | 5-F | C | F | 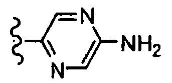 | 533 |
| 340 | 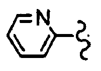 | H | N | F | 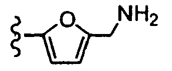 | 518 |
| 341 | 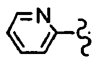 | 5-F | C | H | 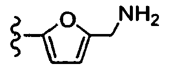 | 535 |
| 342 | 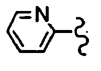 | H | N | F | 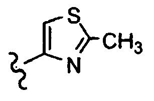 | 520 |
| 343 | 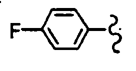 | 6-CI | C | H | 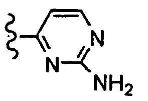 | 548 |
| 345 | 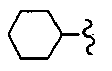 | H | N | H | 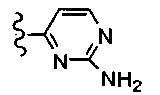 | 503 |
| 346 | (CH₃)₂-CH- | H | N | H | 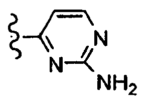 | 463 |

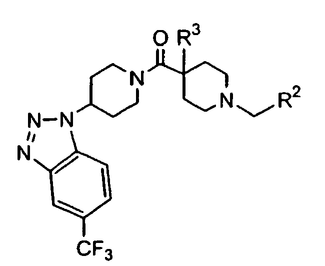

| No. | R³ | R² | Physical Data MS (MH⁺) |
|---|---|---|---|
| 347 | H | 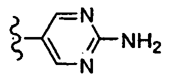 | 489 |
| 348 | F | 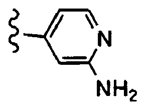 | 506 |
| 349 | F | 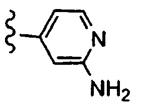 | 488 |
| 350 | F | 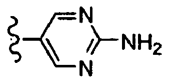 | 507 |
| 351 | F | 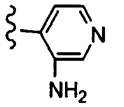 | 506 |

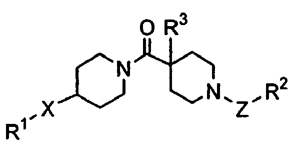

| No. | R¹-X- | Z | R³ | R² | Physical Data MS (MH⁺) |
|---|---|---|---|---|---|
| 352 | 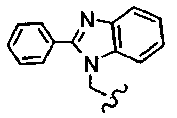 | -CH₂- | H | 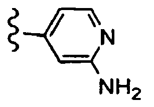 | 509 |
| 353 | 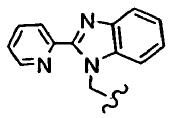 | -CH₂- | H | 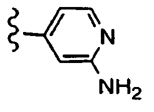 | 510 |
| 354 | 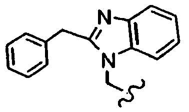 | -CH₂- | H | 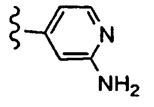 | 523 |
| 355 | 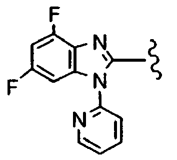 | -CH₂- | H | 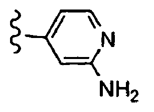 | 532 |
| 356 | 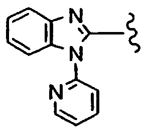 | -CH₂- | H | 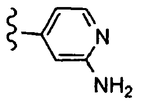 | 496 |
| 357 | 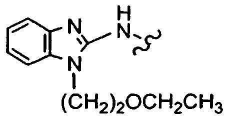 | -CH₂- | H | 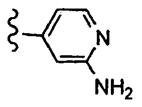 | 506 |
| 358 | 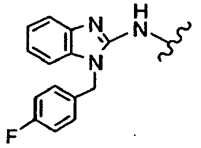 | -CH₂- | H | 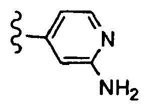 | 542 |
| 359 | 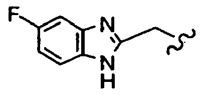 | -CH₂- | H | 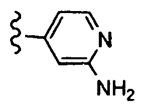 | 451 |
| 360 | 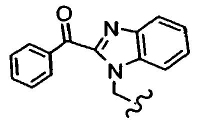 | -CH₂- | H | 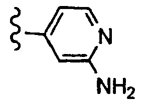 | 537 |
| 361 | 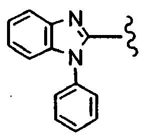 | -CH₂- | H | 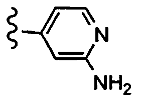 | 495 |
| 362 | 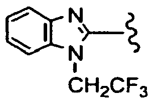 | -CH₂- | H | 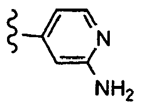 | 501 |
| 363 | 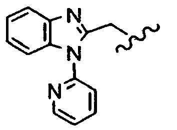 | -CH₂- | H | 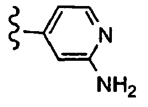 | 510 |
| 364 | 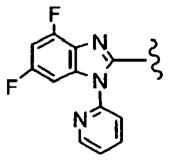 | -CH₂- | H | 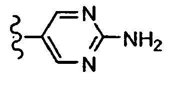 | 533 |
| 365 | 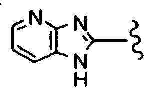 | -CH₂- | H | 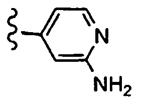 | 420 |
| 366 | 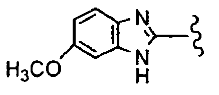 | -CH₂- | H | 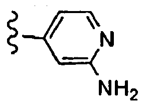 | 449 |
| 367 | 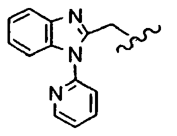 | -CH₂- | H | 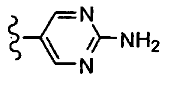 | 497 |
| 368 | 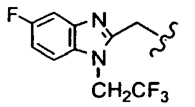 | -CH₂- | H | 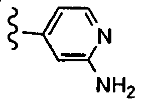 | 533 |
| 369 | 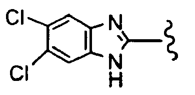 | -CH₂- | H | 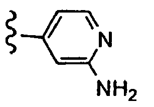 | 487 |
| 370 | 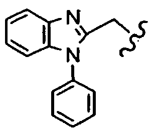 | -CH₂- | H | 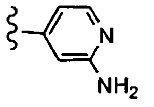 | 509 |
| 371 | 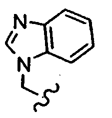 | -CH₂- | H | 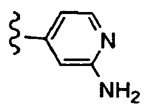 | 433 |
| 372 | 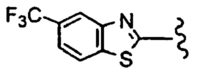 | -CH₂- | H | 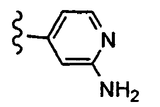 | 504 |
| 373 | 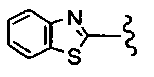 | -CH₂- | H | 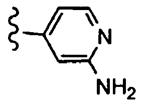 | 436 |
| 374 | 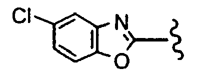 | -CH₂- | H | 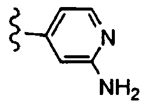 | 472 |
| 375 | 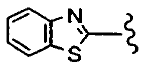 | -(CH₂)₃- | H | 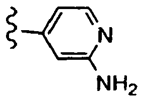 | 464 |
| 376 | 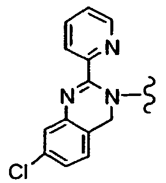 | -CH₂- | H | 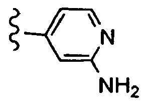 | 544 |
| 377 | 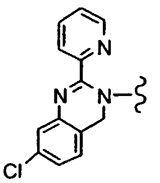 | -CH₂- | F | 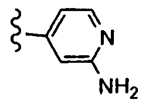 | 562 |

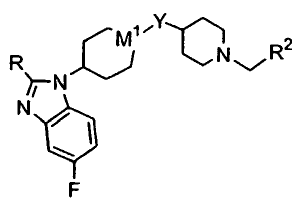

| No. | R | M¹ | Y | R² | Physical Data MS (MH⁺) |
|---|---|---|---|---|---|
| 378 | 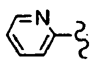 | CH | -CH₂- | 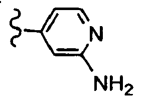 | 500 |
| 379 | 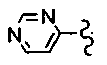 | N | -NH- | 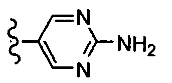 | 502 |
| 380 | 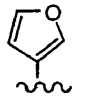 | N | -NH- | 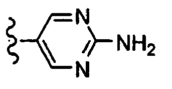 | 490 |
| 381 | 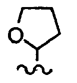 | N | -NH- | 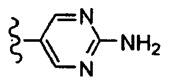 | 494 |
| 382 | 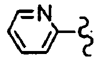 | N | -NH- | 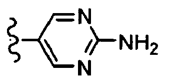 | 501 |
| 383 | 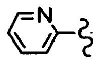 | N | -NH- | 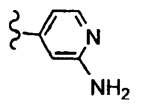 | 500 |

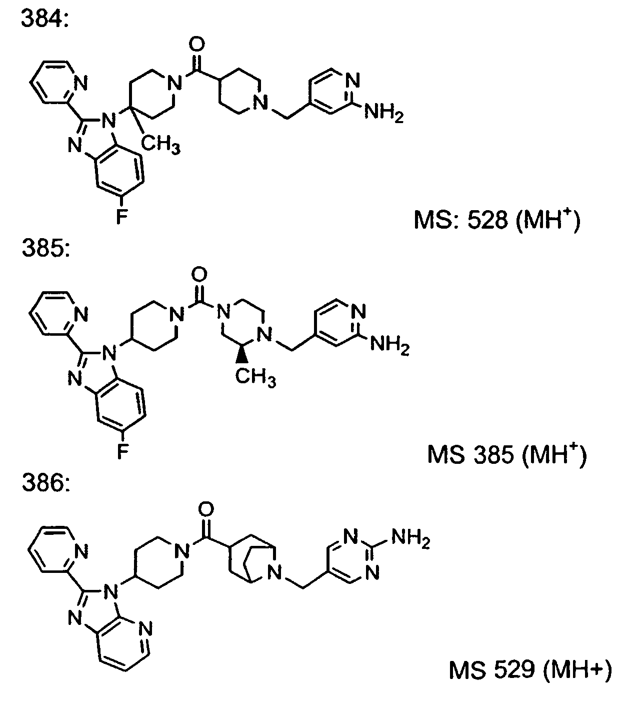

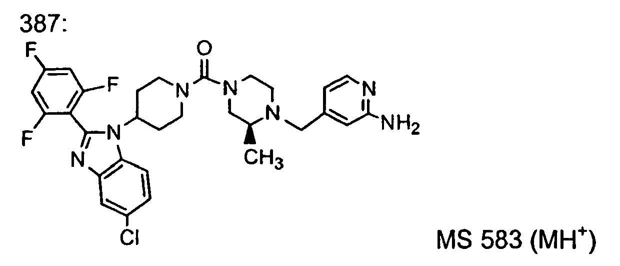

### Example 388

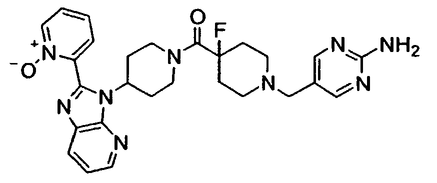

### Step 1:

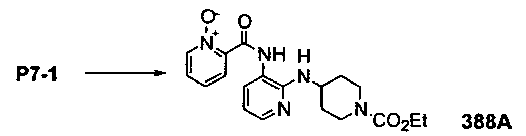

A solution of P7-1 (2.3 g, 8.9 mmol) in CH₂Cl₂-DMF (1:1, 50 ml) was treated with picolinic acid N-oxide (1.5 g, 10.6 mmol), EDCI (2.6 g, 13.3 mmol) and HOBT (1.8 g, 13.3 mmol). The mixture was stirred at 70 °C overnight. The reaction mixture was concentrated, diluted with EtOAc, washed three times with 5% aqueous NaOH, dried over Na₂SO₄, and concentrated. Flash chromatography (50% EtOAc/hexane) provided **388A** (2.5 g, 74%).

### Step 2:

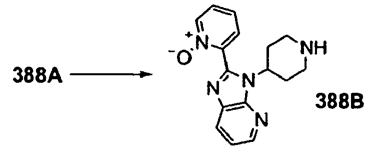

In a manner similar to that described in Preparation 5, Step 4, compound **388A** was converted to compound **388B.**

### Step 3:

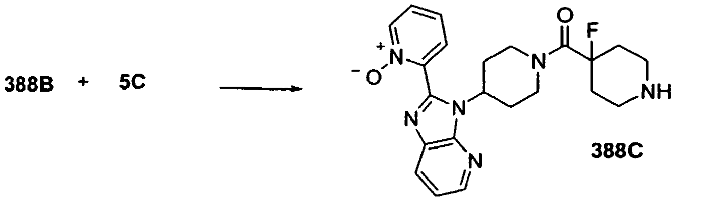

A solution of **388B** (0.66 g, 2.2 mmol) in DMF (15 ml) was treated with 5C (0.62 g, 2.5 mmol), 1-propanephosphonic acid cyclic anhydride (3.3 ml, 11.2 mmol, 50 wt. % in EtOAc) and N-ethylmorpholine (1.4 ml, 10.7 mmol). The mixture was stirred at 50 °C for 3h. The reaction mixture was concentrated and diluted with EtOAc. The solution was washed three times with 5% aqueous NaOH, dried over Na₂SO₄, concentrated and subjected to flash chromatography (10% 2N NH₃-CH₃OH/EtOAc). The material was then taken up in CH₂Cl₂ (20 ml) and treated with 4 M HCl-dioxane (4 ml). After stirring overnight at 20 °C, the reaction was carefully basified with 10% aqueous NaOH and extracted with CH₂Cl₂. The combined organic layers were dried over Na₂SO₄, concentrated and subjected to flash chromatography (30% 2N NH₃-CH₃OH/EtOAc) to provide **388C** as a white solid (0.08g, 10%).

### Step 4:

In a manner similar to that described in Example 5, Step 5, compound **388C** was converted to Example 388.

### Example 389

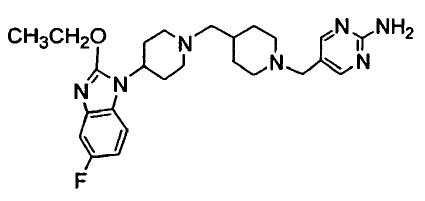

### Step 1:

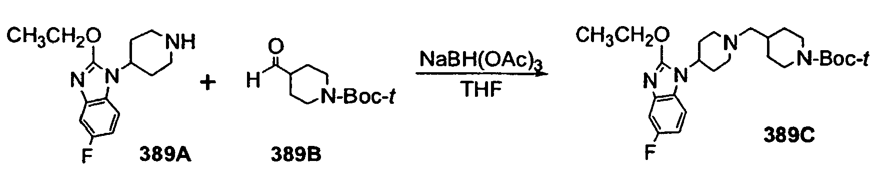

To a stirred, cloudy solution of **389A** (300 mg, 1.14 mmol) in THF (15 ml) were added a solution of **389B** (292 mg, 1.37 mmol) in THF (1 ml), followed by NaBH(OAc)₃ (483 mg, 2.28 mmol). After stirring at RT for 39 h, TLC revealed the presence of unchanged starting materials in the cloudy white reaction suspension. Therefore, another quantity of NaBH(OAc)₃ (242 mg, 1.14 mmol) was added and stirring at RT continued for a total of 113 h. The reaction mixture was then filtered and collected solids washed thoroughly with CH₂Cl₂. The combined filtrate and washings were stripped of solvent under vacuum, and the residue was partitioned between EtOAc (60 ml) and a solution consisting of water (2.5 ml), 2M Na₂CO₃ (6.5 ml) and 6N NaOH (5 ml). The aqueous layer was further extracted with EtOAc (3 x 15 ml). The combined extracts were washed with brine (5 ml) and dried over anhydrous MgSO₄. Drying agent was removed by filtration, and the filtrate was concentrated under vacuum. The residue was purified by silica gel flash chromatography (EtOAc/hexanes = 1:1) to obtain **389C** as a mixture of colorless gum and white foam (368 mg, 70%), homogeneous to TLC, which solidified upon standing. ES-MS: 461 (MH⁺; 100%).

### Step 2:

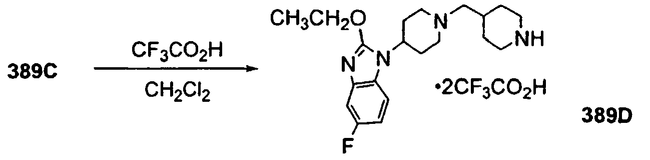

To a stirred, ice-cold solution of **389C** (358 mg, 0.777 ml) in CH₂Cl₂ (7 ml) was added via syringe cold, neat TFA (576 microliters, 886 mg, 7.77 mmol). The resultant solution was stirred in an ice-water bath for 30 min, then at RT for 29.5 h. Volatiles were removed under vacuum, and the gummy residue was triturated (magnetic stirrer) with Et₂O (35 ml) for 16 h. Filtration and washing with Et₂O yielded the bis-trifluoroacetate salt of **389D** as a white powder (449 mg, 98%).

### Step 3:

To a stirred suspension of **389D** (100 mg, 0.170 mmol) in CH₂Cl₂ (5 ml) was added Et₃N (47.4 microliters, 34.4 mg, 0.340 mmol), whereupon all solids dissolved. To the stirred solution were then added 5G (25.1 mg, 0.204 mmol), followed by NaBH(OAc)₃ (72.1 mg, 0.340 mmol). After stirring at RT for 66 h, TLC revealed the presence of unchanged starting materials in the light yellow reaction suspension. Therefore, another quantity of NaBH(OAc)₃ (72.1 mg, 0.340 mmol) was added and stirring at RT continued for a total of 90 h. The reaction mixture was then filtered and collected solids washed thoroughly with CH₂Cl₂. The combined filtrate and washings were stripped of solvent under vacuum, and the residue was partitioned between EtOAc (20 ml) and a solution consisting of water (0.6 ml), 2M Na₂CO₃ (1.5 ml) and 6N NaOH (1.2 ml). The aqueous layer was further extracted with EtOAc (3 x 5 ml). The combined extracts were washed with brine (2 ml) and dried over anhydrous MgSO₄. Drying agent was removed by filtration, and the filtrate was concentrated under vacuum. The residue was purified by preparative TLC (silica gel; CH₂Cl₂/CH₃OH/conc. NH₄OH = 90:9:1) to obtain the title compound as a light beige foam (36 mg, 45%). FABMS: 468 (MH⁺; 100%).

Using procedures similar to those described above in Examples 1-6 and 388-389, the following compounds were prepared:

| Ex. | Structure | Mass Spec |
|---|---|---|
| | | (M+H) |
| 390 | 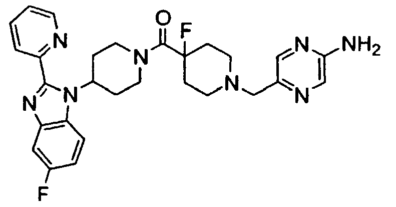 | 533 |
| | | (ESMS) |
| 391 | 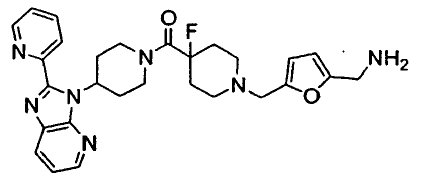 | 518 |
| | | (ESMS) |
| 392 | 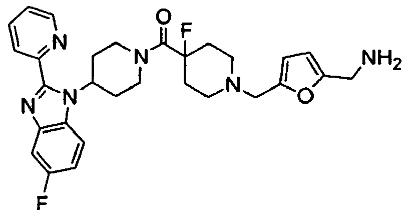 | 535 |
| | | (ESMS) |
| 393 | 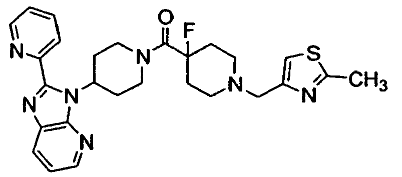 | 520 |
| | | (ESMS) |
| 394 | 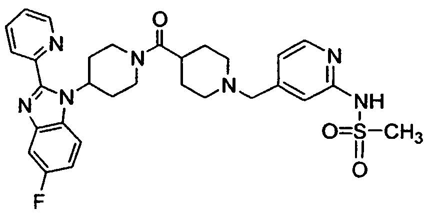 | 592 |
| | | (FAB) |
| 395 | 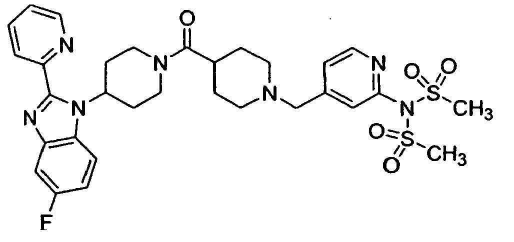 | 670 |
| | | (FAB) |
| 396 | 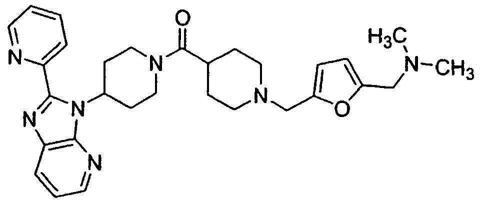 | 528 |
| | | (ESMS) |
| 397 | 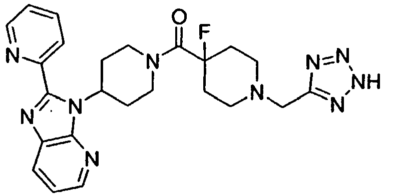 | 491 |
| | | (ESMS) |
| 398 | 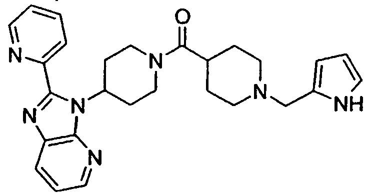 | 470 |
| | | (ESMS) |
| 399 | 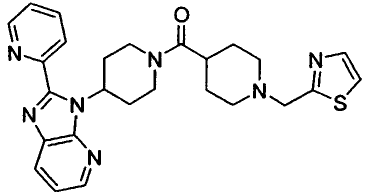 | 488 |
| | | (ESMS) |
| 400 | 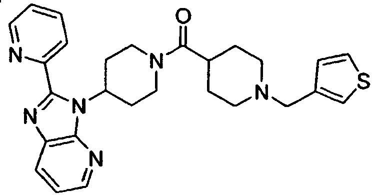 | 487 |
| | | (ESMS) |
| 401 | 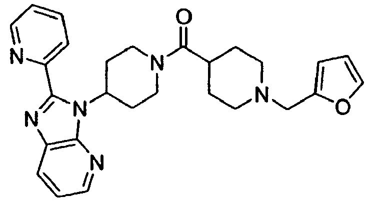 | 471 |
| | | (ESMS) |
| 402 | 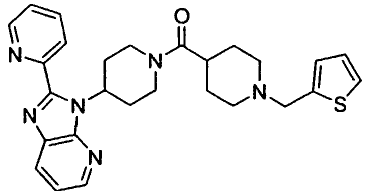 | 487 |
| | | (ESMS) |
| 403 | 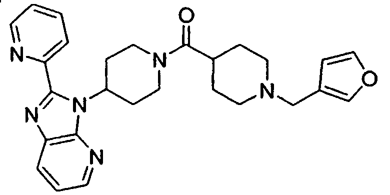 | 471 |
| | | (ESMS) |
| 404 | 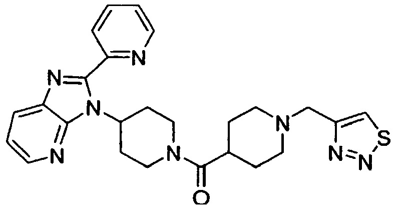 | 489 |
| | | (ESMS) |
| 405 | 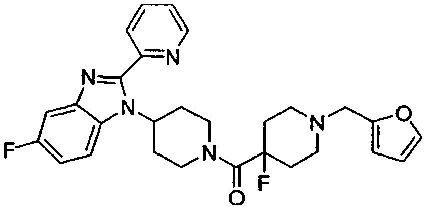 | 506 |
| | | (ESMS) |
| 406 | 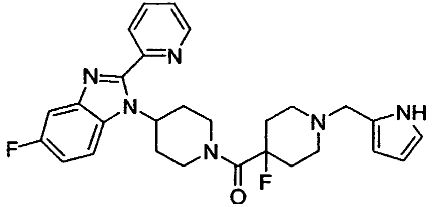 | 505 |
| | | (ESMS) |
| 407 | 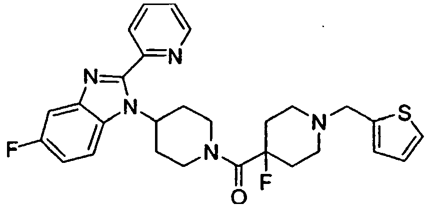 | 522 |
| | | (ESMS) |
| 408 | 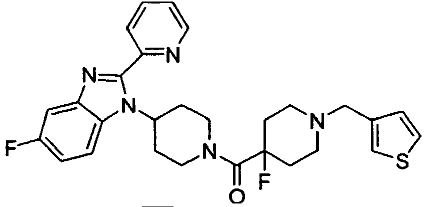 | 522 |
| | | (ESMS) |
| 409 | 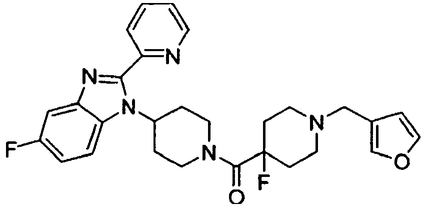 | 506 |
| | | (ESMS) |
| 410 | 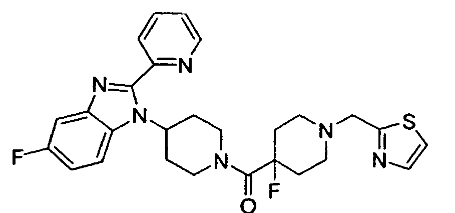 | 523 |
| | | (ESMS) |
| 411 | 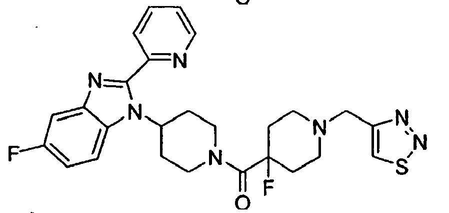 | 524 |
| | | (ESMS) |
| 412 | 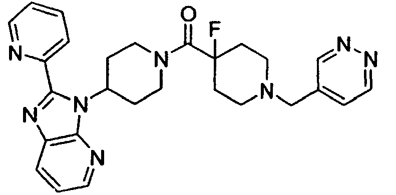 | 501 |
| | | (ESMS) |
| 413 | 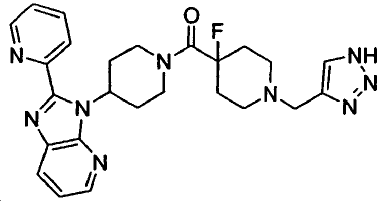 | 490 |
| | | (ESMS) |
| 414 | 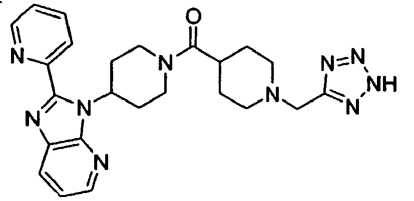 | 473 |
| | | (ESMS) |
| 415 | 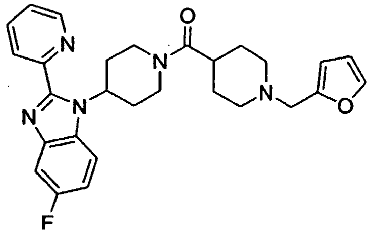 | 488 |
| | | (ESMS) |
| 416 | 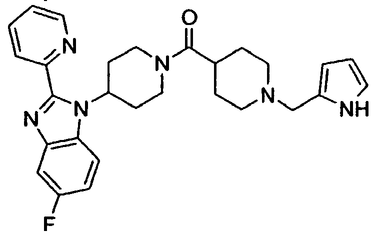 | 487 |
| | | (ESMS) |
| 417 | 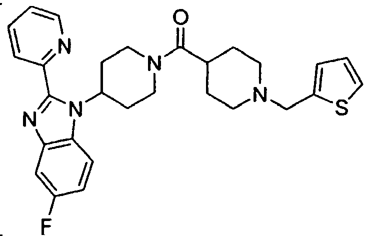 | 504 |
| | | (ESMS) |
| 418 | 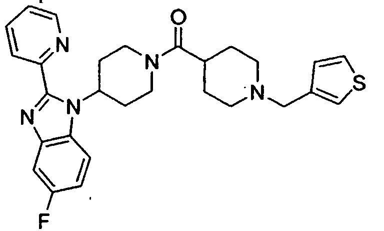 | 504 |
| | | (ESMS) |
| 419 | 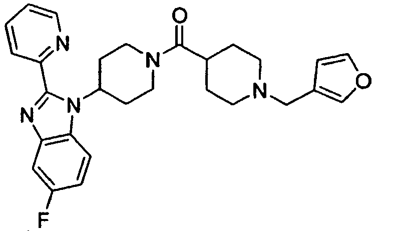 | 488 |
| | | (ESMS) |
| 420 | 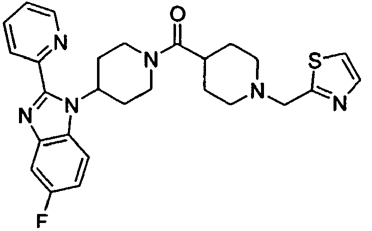 | 505 |
| | | (ESMS) |
| 421 | 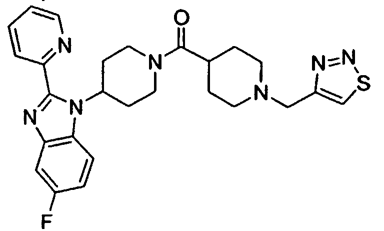 | 506 |
| | | (ESMS) |
| 422 | 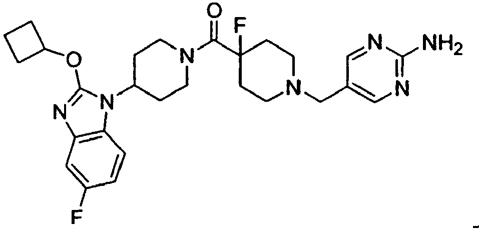 | 526 |
| | | (FAB) |
| 423 | 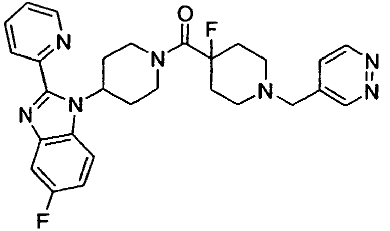 | 518 |
| | | (ESMS) |
| 424 | 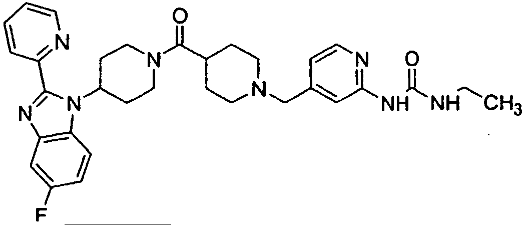 | 585 |
| | | (FAB) |
| 425 | 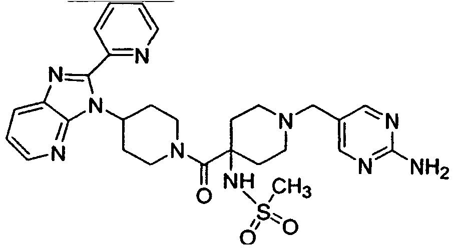 | 591 |
| | | (ESMS) |
| 426 | 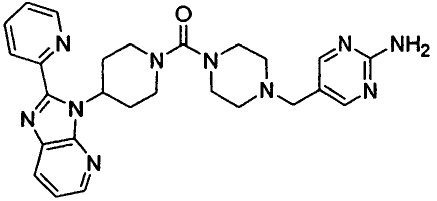 | 499 |
| | | (ESMS) |
| 427 | 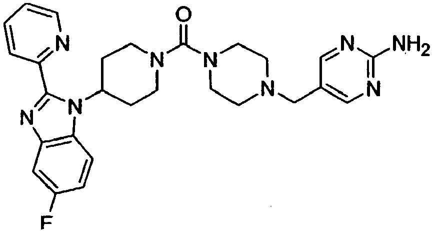 | 516 |
| | | (ESMS) |
| 428 | 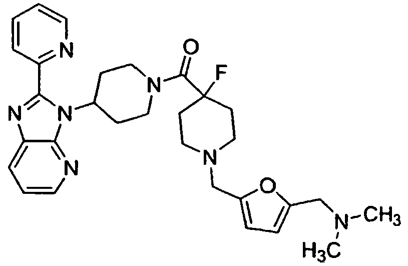 | 546 |
| | | (ESMS) |
| 429 | 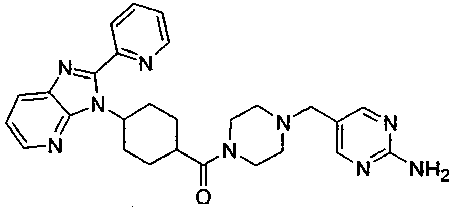 | 498 |
| | | (ESMS) |
| 430 | 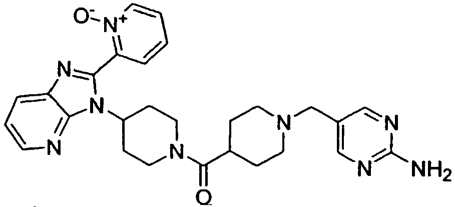 | 514 |
| | | (ESMS) |
| 431 | 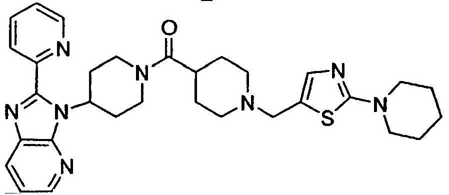 | 571 |
| | | (ESMS) |
| 432 | 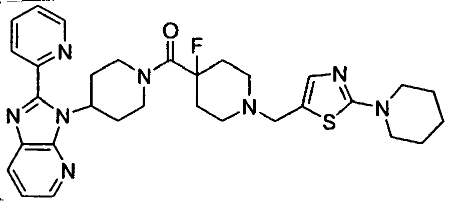 | 589 |
| | | (ESMS) |
| 433 | 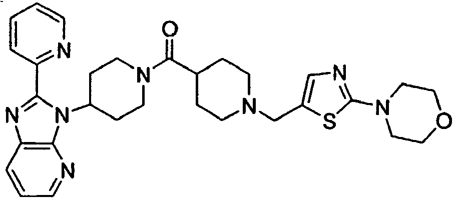 | 573 |
| | | (ESMS) |
| 434 | 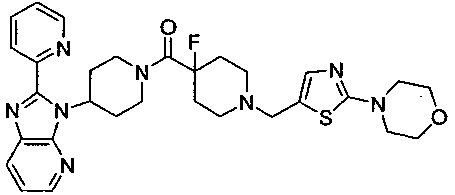 | 591 |
| | | (ESMS) |
| 435 | 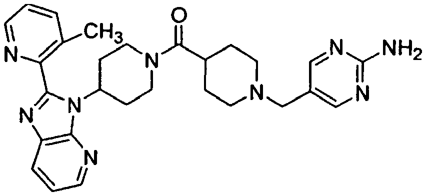 | 512 |
| | | (ESMS) |
| 436 | 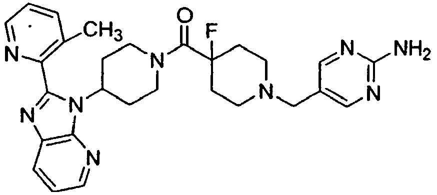 | 530 |
| | | (ESMS) |
| 437 | 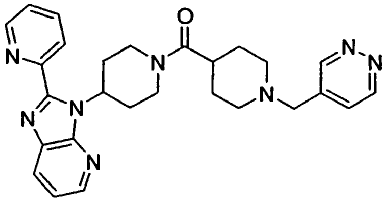 | 483 |
| | | (ESMS) |
| 438 | 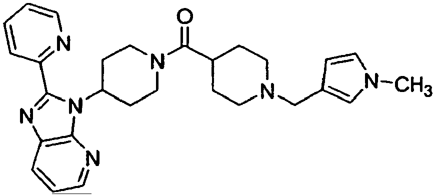 | 484 |
| | | (ESMS) |
| 439 | 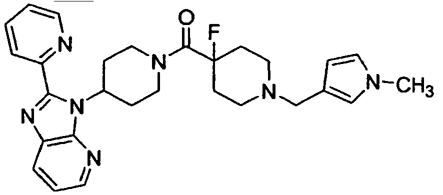 | 502 |
| | | (ESMS) |
| 440 | 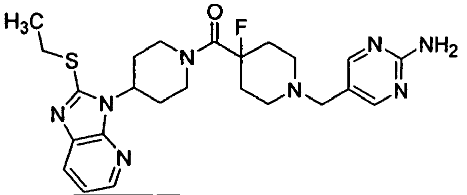 | 499 |
| | | (FAB) |
| 441 | 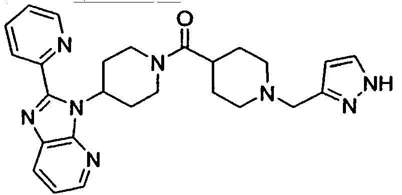 | 471 |
| | | (ESMS) |
| 442 | | 488 |
| | 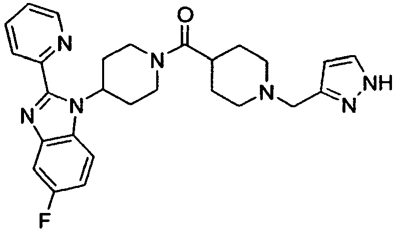 | (ESMS) |
| 443 | 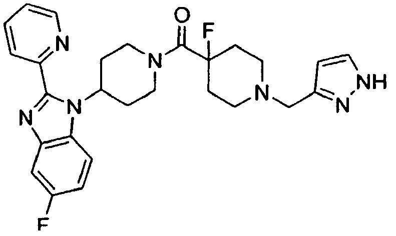 | 506 |
| | | (ESMS) |
| 444 | 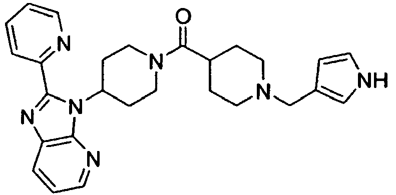 | 470 |
| | | (ESMS) |
| 445 | 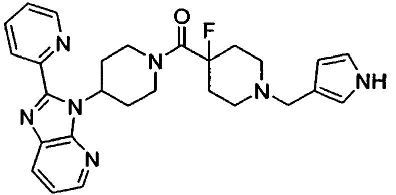 | 488 |
| | | (ESMS) |
| 446 | 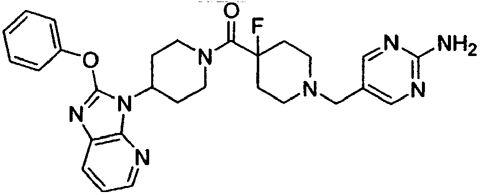 | 531 |
| | | (FAB) |
| 447 | 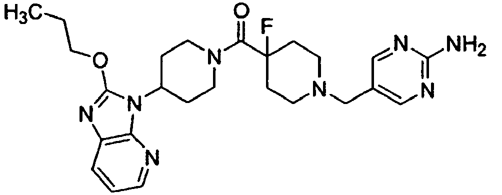 | 497 |
| | | (FAB) |
| 448 | 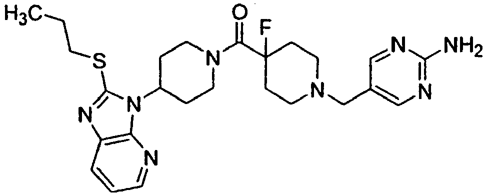 | 513 |
| | | (FAB) |
| 449 | 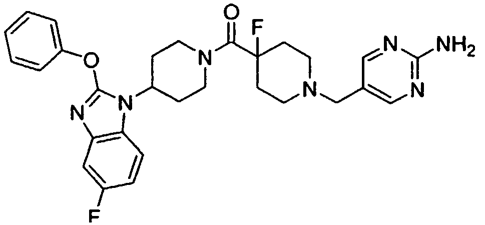 | 548 |
| | | (FAB) |
| 450 | 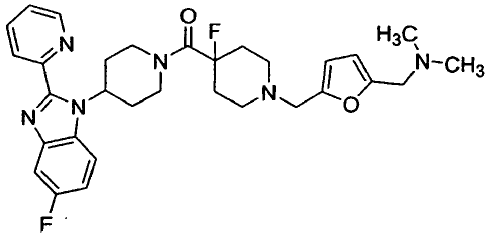 | 563 |
| | | (ESMS) |
| 451 | 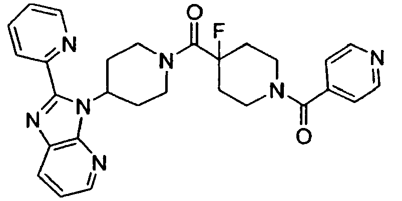 | 514(ESM S) |
| 452 | 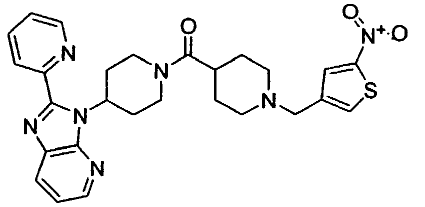 | 532 |
| | | (ESMS) |
| 453 | 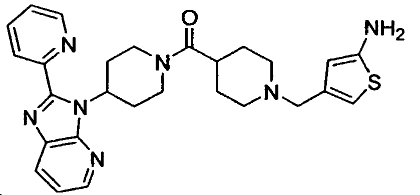 | 502 |
| | | (ESMS) |
| 454 | 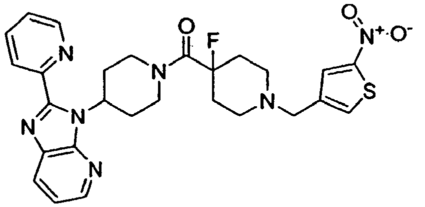 | 550 |
| | | (ESMS) |
| 455 | 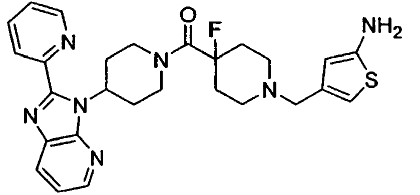 | 520 |
| | | (ESMS) |
| 456 | 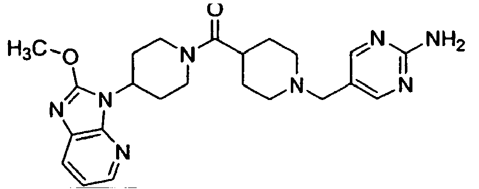 | 451 |
| | | (ESMA) |
| 457 | 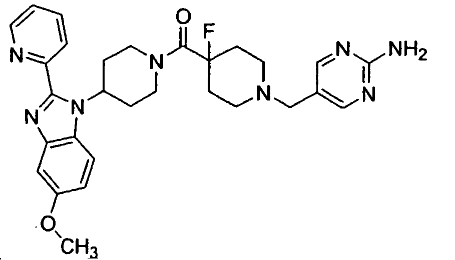 | 545 |
| | | (ESMS) |
| 458 | 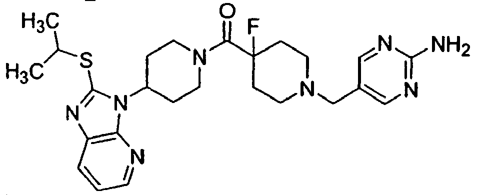 | 513 |
| | | (ESMS) |
| 459 | 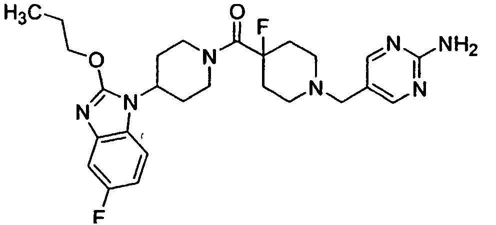 | 514 |
| | | (FAB) |
| 460 | 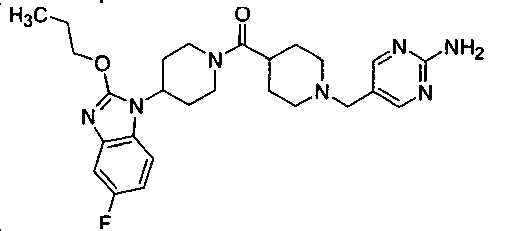 | 496 |
| | | (FAB) |
| 461 | 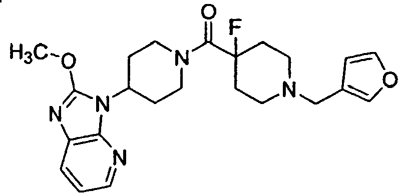 | 442 |
| | | (ESMS) |
| 462 | 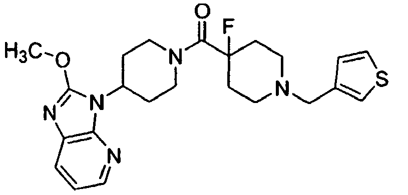 | 458 |
| | | (ESMS) |
| 463 | 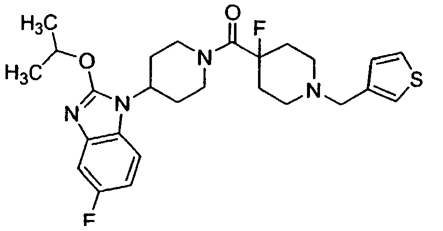 | 503 |
| | | (ESMS) |
| 464 | 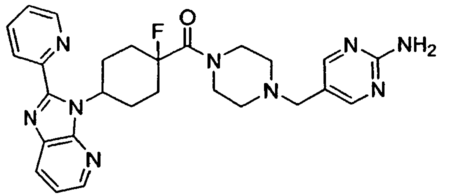 | 407 |
| | | (ESMS) |
| 465 | 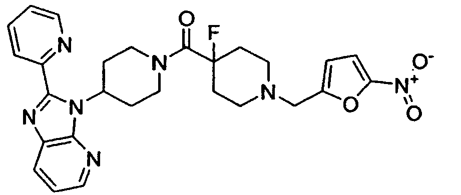 | 534 |
| | | (ESMS) |
| 466 | 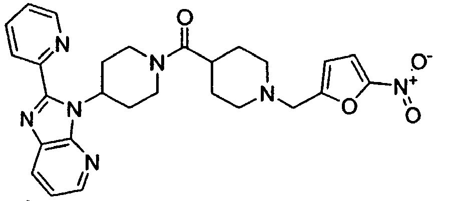 | 516 |
| | | (ESMS) |
| 467 | 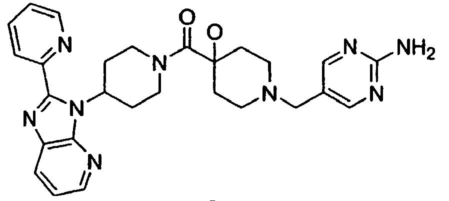 | 514 |
| | | (ESMS) |
| 468 | 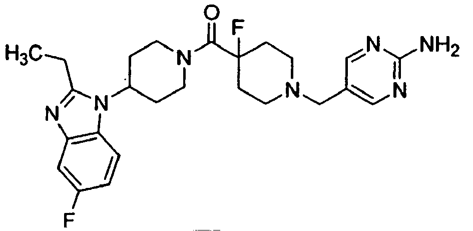 | 484 |
| | | (ESMS) |
| 469 | 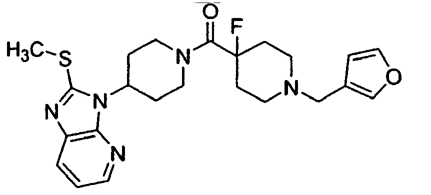 | 458 |
| | | (ESMS) |
| 470 | 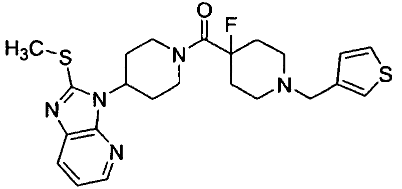 | 474 |
| | | (ESMS) |
| 471 | 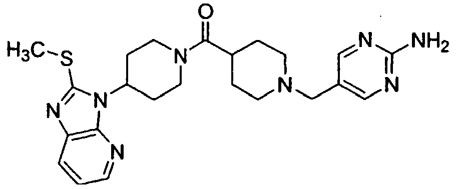 | 467 |
| | | (ESMA) |
| 472 | 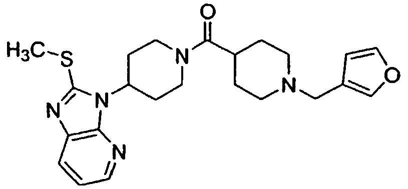 | 440 |
| | | (ESMS) |
| 473 | 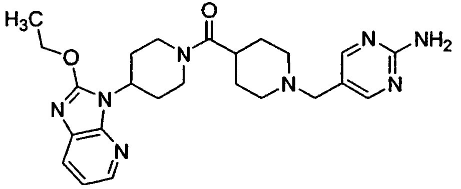 | 465 |
| | | (ESMS) |
| 474 | 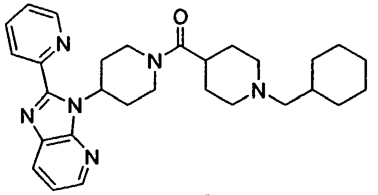 | 487 |
| | | (ESMS) |
| 475 | 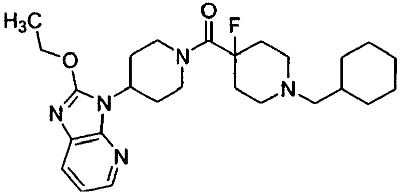 | 472 |
| | | (ESMS) |
| 476 | 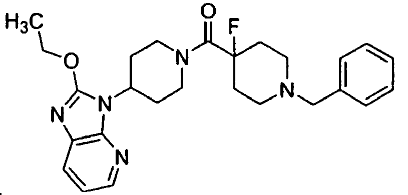 | 466 |
| | | (ESMS) |
| 477 | 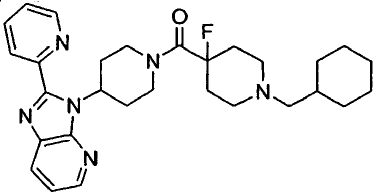 | 505 |
| | | (ESMS) |
| 478 | 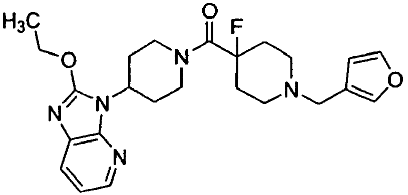 | 456 |
| | | (ESMS) |
| 479 | 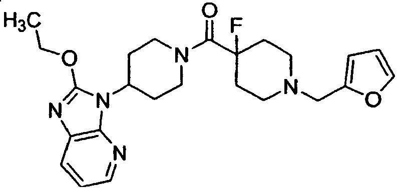 | 456 |
| | | (ESMS) |
| 480 | 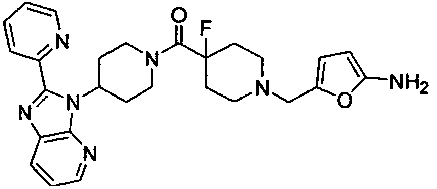 | 504 |
| | | (ESMS) |
| 481 | 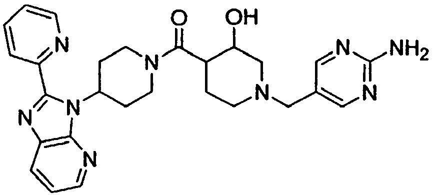 | 514 |
| | | (ESMS) |
| 482 | 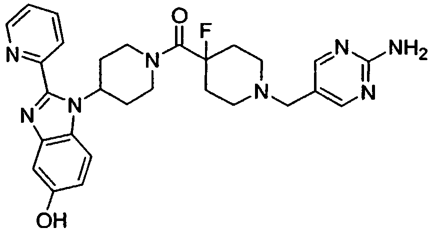 | 531 |
| | | (FAB) |
| 483 | 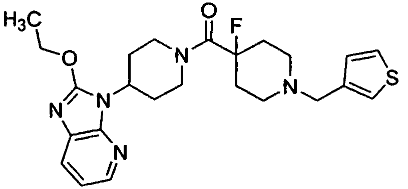 | 472 |
| | | (ESMS) |
| 484 | 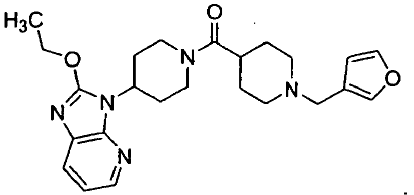 | 438 |
| | | (ESMS) |
| 485 | 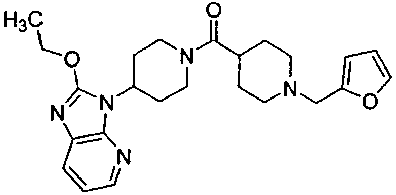 | 438 |
| | | (ESMS) |
| 486 | 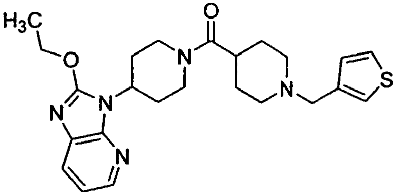 | 454 |
| | | (ESMS) |
| 487 | 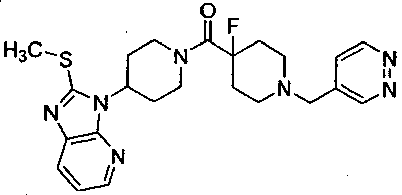 | 470 |
| | | (ESMS) |
| 488 | 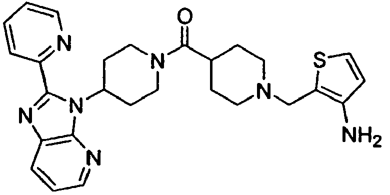 | 502 |
| | | (ESMS) |
| 489 | 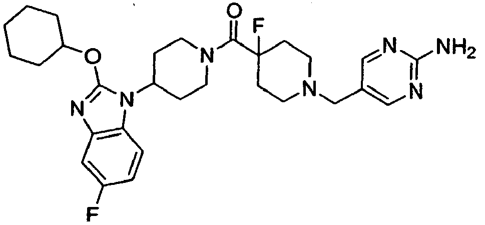 | 554 |
| | | (FAB) |
| 490 | 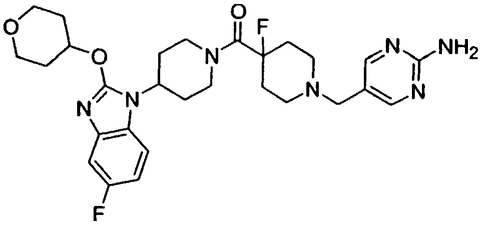 | 556 |
| | | (FAB) |
| 491 | 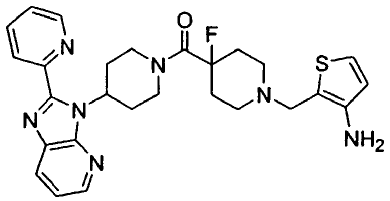 | 470 |
| | | (ESMS) |
| 492 | 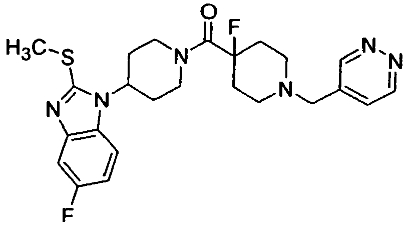 | 487 |
| | | (ESMS) |
| 493 | 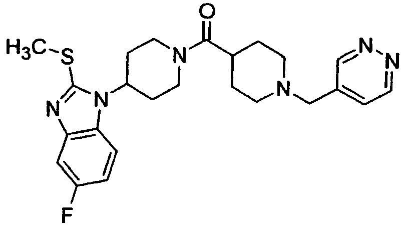 | 469 |
| | | (ESMS) |
| 44 | 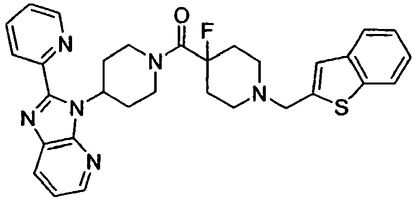 | 555 |
| | | (ESMS) |
| 495 | 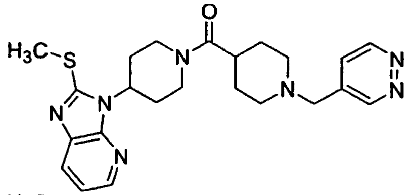 | 452 |
| | | (ESMS) |
| 496 | 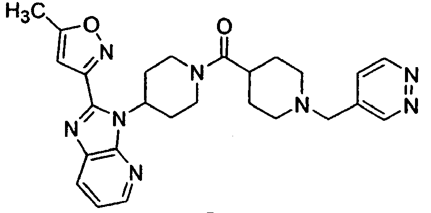 | 487 |
| | | (ESMS) |
| 497 | 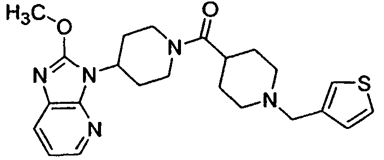 | 440 |
| | | (ESMS) |
| 498 | 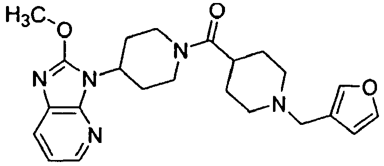 | 424 |
| | | (ESMS) |
| 499 | 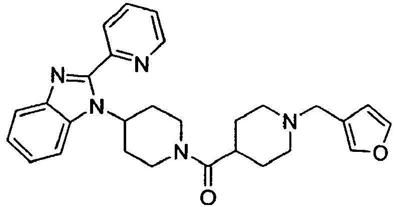 | 470 |
| | | (ESMS) |
| 500 | 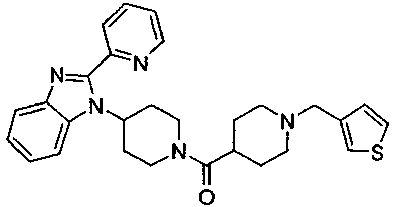 | 486 |
| | | (ESMS) |
| 501 | 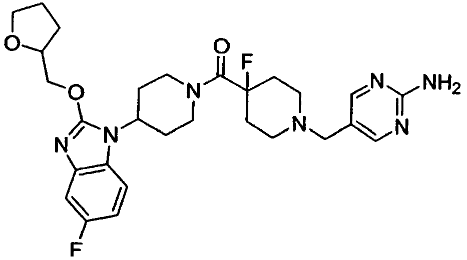 | 556 |
| | | (ESMS) |
| 502 | 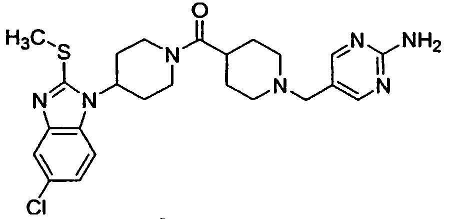 | 500 |
| | | (ESMS) |
| 503 | 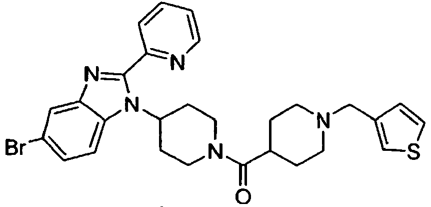 | 566 |
| | | (ESMS) |
| 504 | 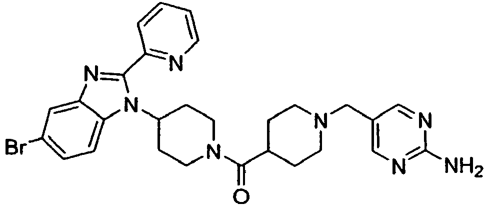 | 577 |
| | | (ESMS) |
| 505 | 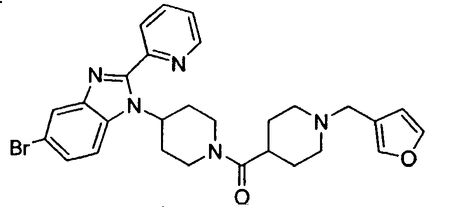 | 550 |
| | | (ESMS) |
| 506 | 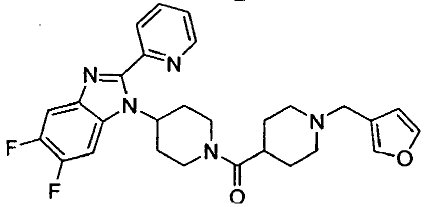 | 506 |
| | | (ESMS) |
| 507 | 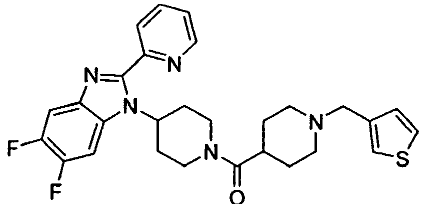 | 522 |
| | | (ESMS) |
| 508 | 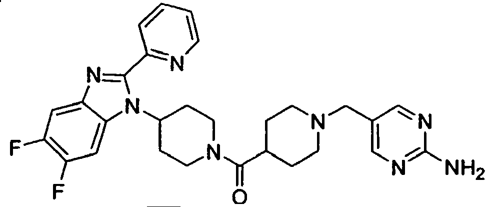 | 533 |
| | | (ESMS) |
| 509 | 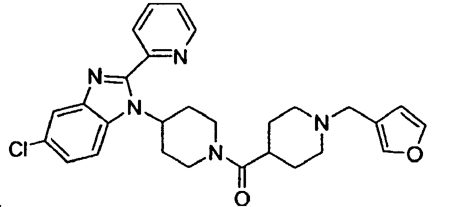 | 504 |
| | | (ESMS) |
| 510 | 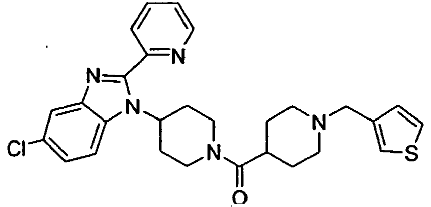 | 520 |
| | | (ESMS) |
| 511 | 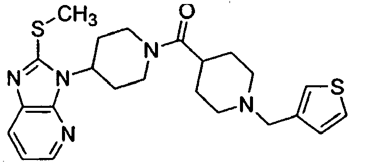 | 456 |
| | | (ESMS) |
| 512 | 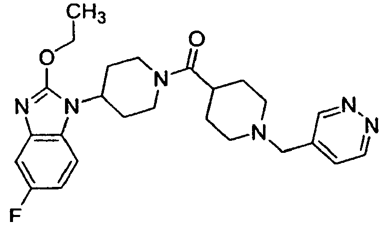 | 467 |
| | | (ESMS) |
| 513 | 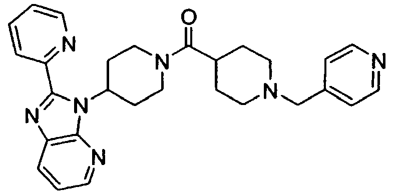 | 482 |
| | | (ESMS) |
| 514 | 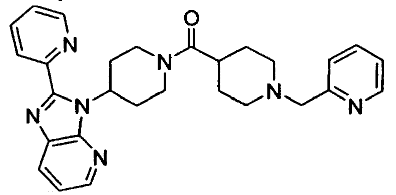 | 482 |
| | | (ESMS) |
| 515 | 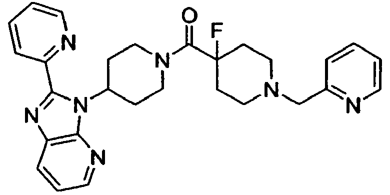 | 500 |
| | | (ESMS) |
| 516 | 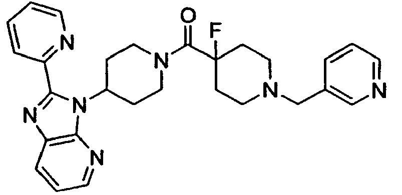 | 500 |
| | | (ESMS) |
| 517 | 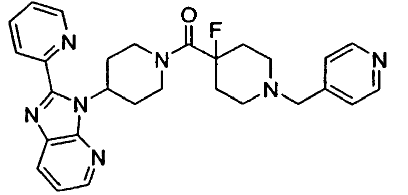 | 500 |
| | | (ESMS) |
| 518 | 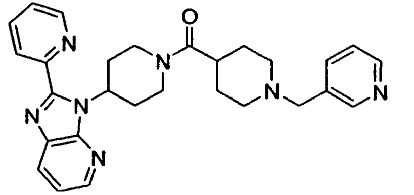 | 482 |
| | | (ESMS) |
| 519 | 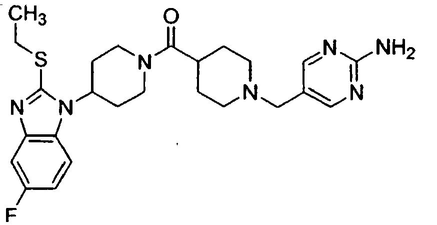 | 498 |
| | | (ESMS) |
| 520 | 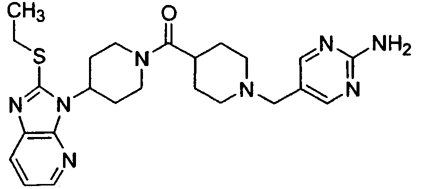 | 481 |
| | | (ESMS) |
| 521 | 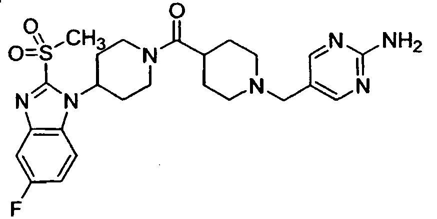 | 516 |
| | | (ESMS) |
| 522 | 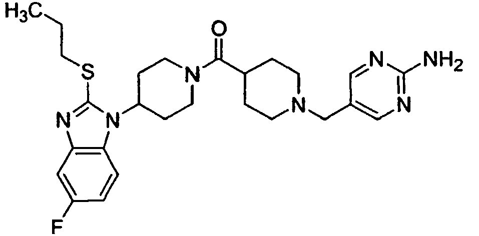 | 512 |
| | | (FAB) |
| 523 | 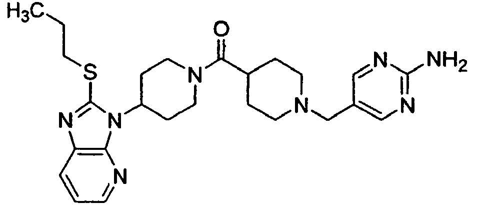 | 495 |
| | | (FAB) |
| 524 | 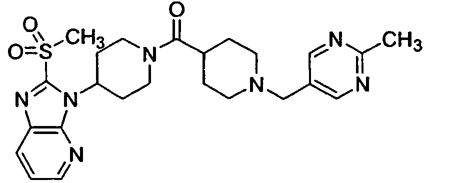 | 499 |
| | | (FAB) |
| 525 | 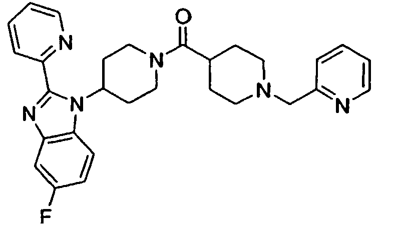 | 499 |
| | | (ESMS) |
| 526 | 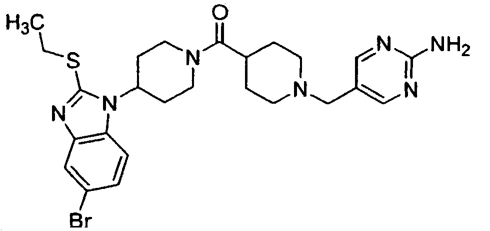 | 560 |
| | | (ESMS) |
| 527 | 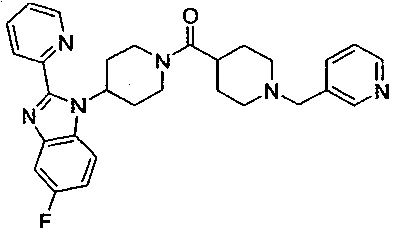 | 499 |
| | | (ESMS) |
| 528 | 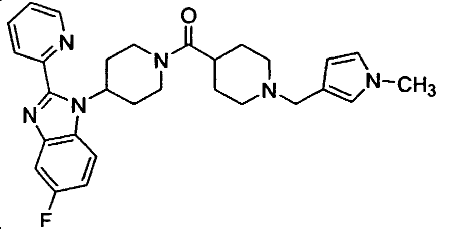 | 501(ESM S) |
| 529 | 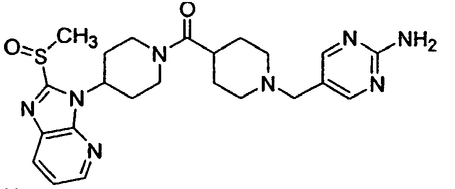 | 483 |
| | | (ESMS) |
| 530 | 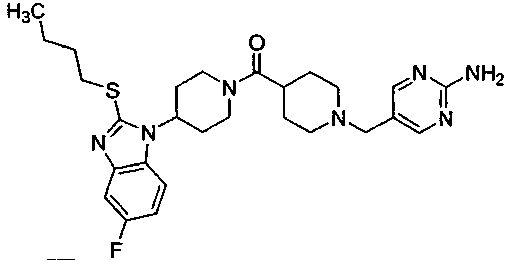 | 526 |
| | | (ESMS) |
| 531 | 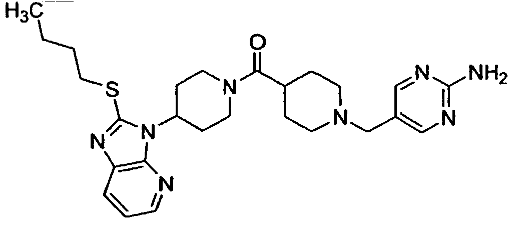 | 509 |
| | | (ESMS) |
| 532 | 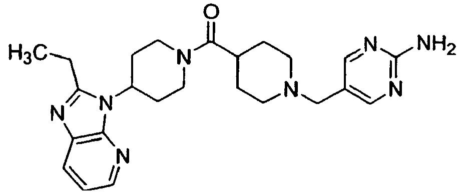 | 449 |
| | | (ESMS) |
| 533 | 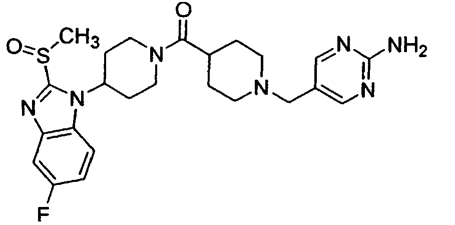 | 500 |
| | | (ESMS) |
| 534 | 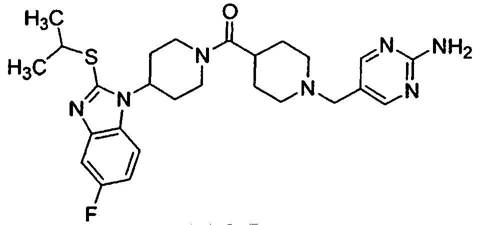 | 512 |
| | | (ESMS) |
| 535 | 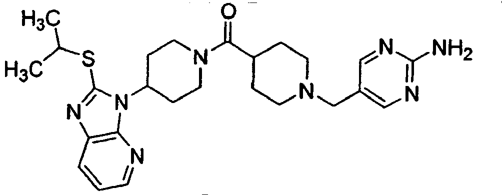 | 495 |
| | | (ESMS) |
| 536 | 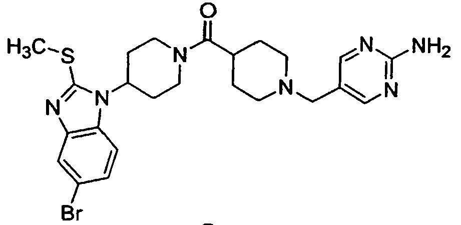 | 546 |
| | | (ESMS) |
| 537 | 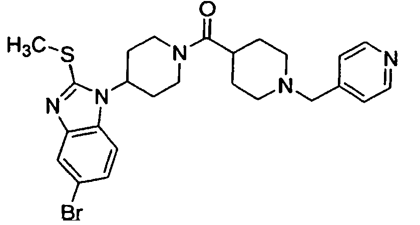 | 530 |
| | | (ESMS) |
| 538 | 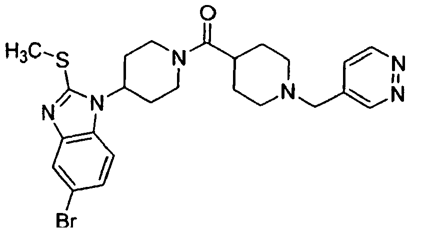 | 531 |
| | | (ESMS) |
| 539 | 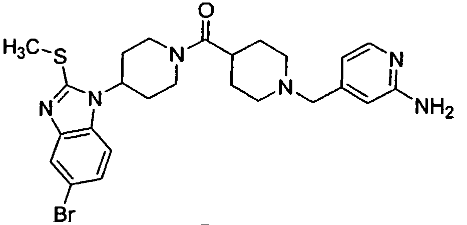 | 545 |
| | | (ESMS) |
| 540 | 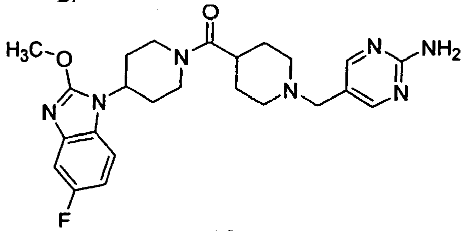 | 468 |
| | | (ESMS) |
| 541 | 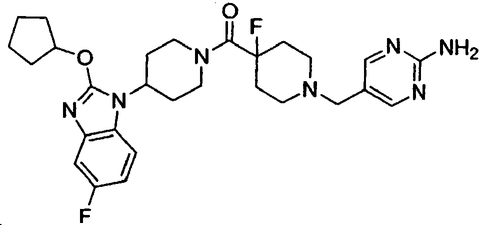 | 540 |
| | | (ESMS) |
| 542 | 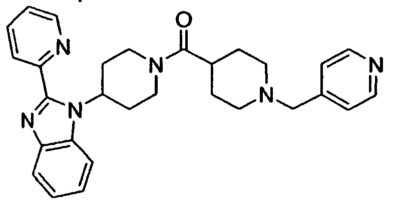 | 481 |
| | | (ESMS) |
| 543 | 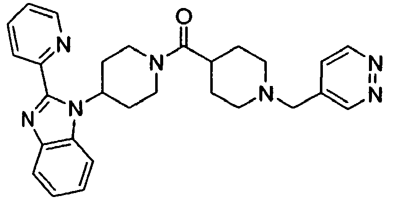 | 482 |
| | | (ESMS) |
| 544 | 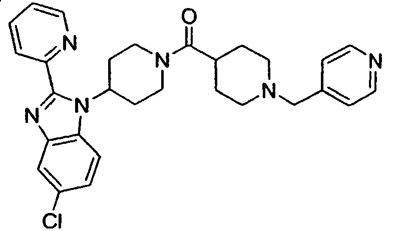 | 515 |
| | | (ESMS) |
| 545 | 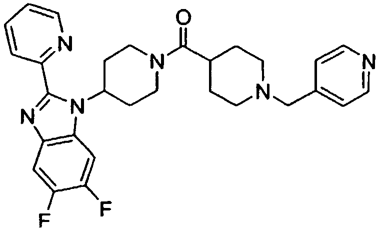 | 517 |
| | | (ESMS) |
| 546 | 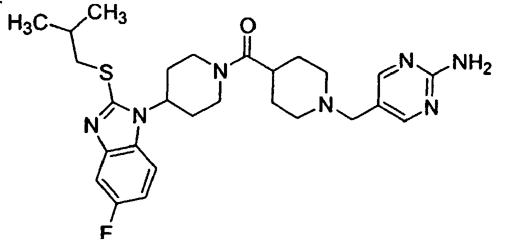 | 526 |
| | | (ESMS) |
| 547 | 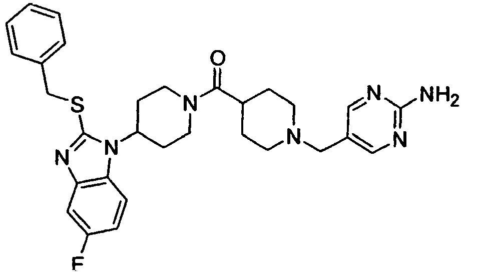 | 5560 |
| | | (ESMS) |
| 548 | 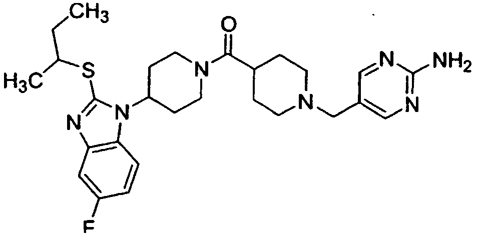 | 526 |
| | | (ESMS) |
| 549 | 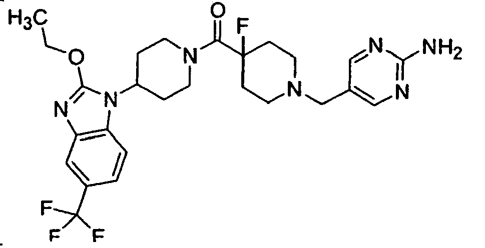 | 550 |
| | | (ESMS) |
| 550 | 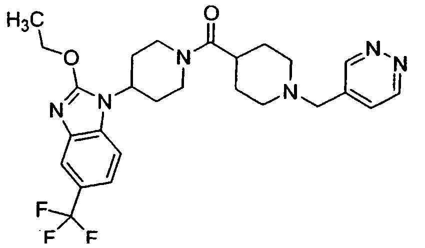 | 517 |
| | | (ESMS) |
| 551 | 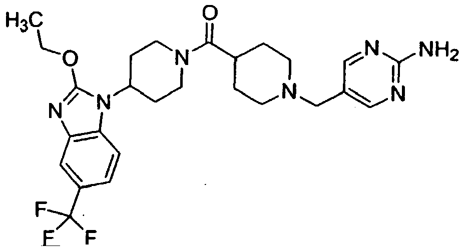 | 532 |
| | | (ESMS) |
| 552 | 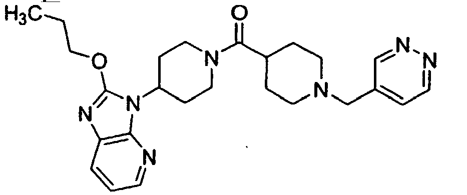 | 464 |
| | | (ESMS) |
| 553 | 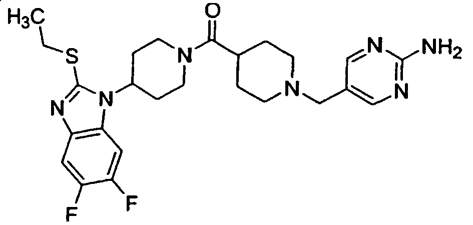 | 516 |
| | | (ESMS) |
| 554 | 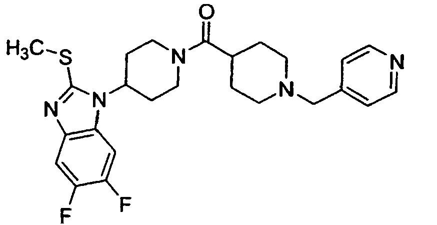 | 486 |
| | | (ESMS) |
| 555 | 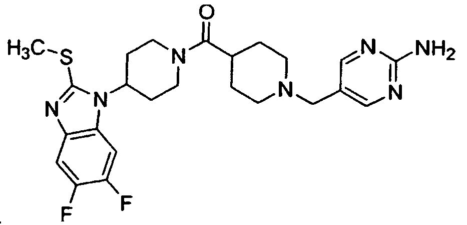 | 502 |
| | | (ESMS) |
| 556 | 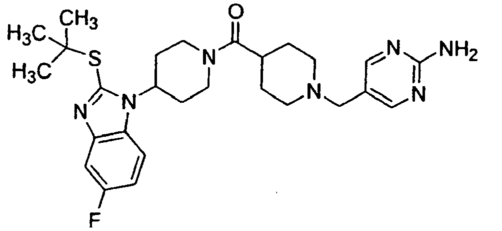 | 526 |
| | | (ESMS) |
| 557 | 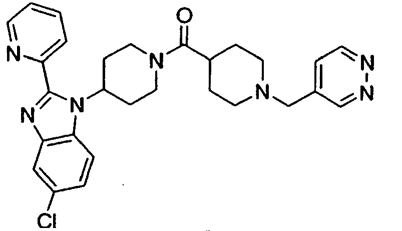 | 516 |
| | | (ESMS) |
| 558 | 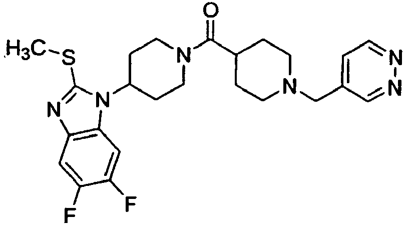 | 487 |
| | | (ESMS) |
| 559 | 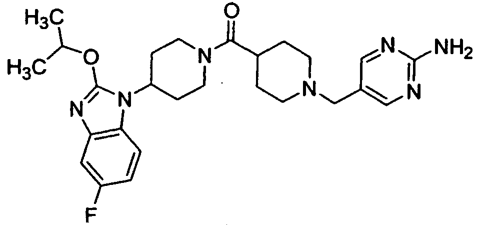 | 496 |
| | | (ESMS) |
| 560 | 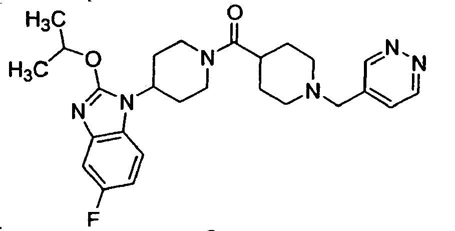 | 481 |
| | | (FAB) |
| 561 | 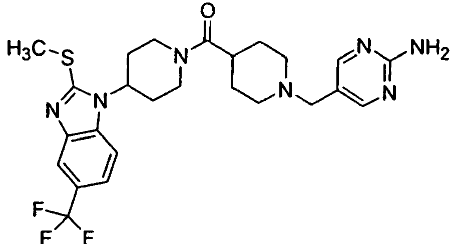 | 534 |
| | | (ESMS) |
| 562 | 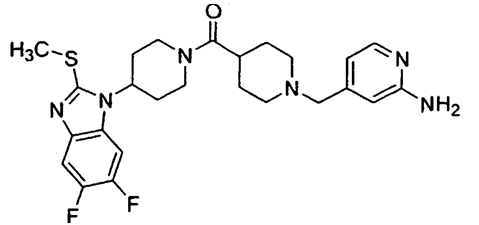 | 501 |
| | | (ESMS) |
| 563 | 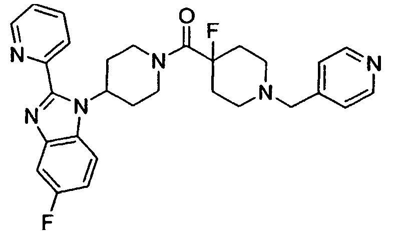 | 517 |
| | | (ESMS) |
| 564 | 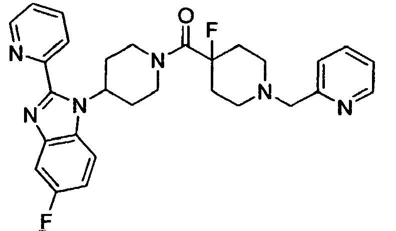 | 517 |
| | | (ESMS) |
| 565 | 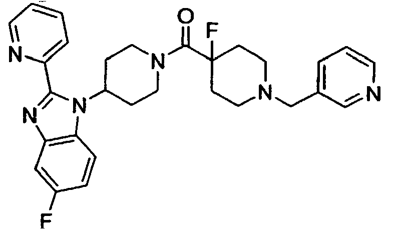 | 517 |
| | | (ESMS) |
| 566 | 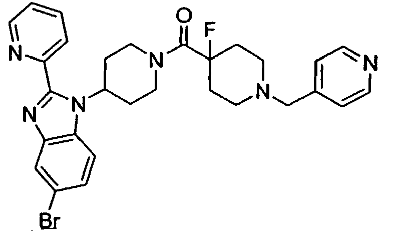 | 577 |
| | | (ESMS) |
| 567 | 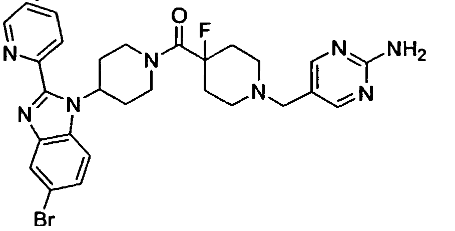 | 592 |
| | | (ESMS) |
| 568 | 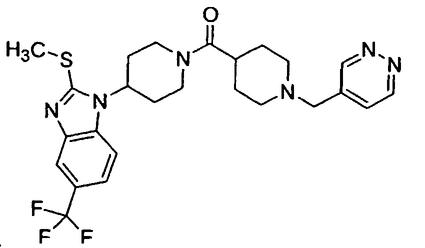 | 519 |
| | | (ESMS) |
| 569 | 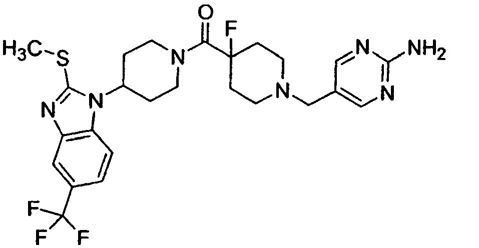 | 552 |
| | | (ESMS) |
| 570 | 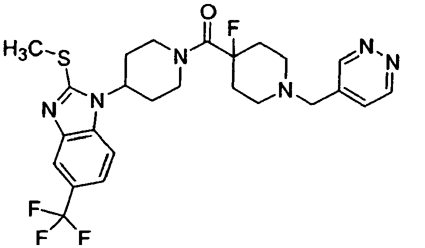 | 537 |
| | | (ESMS) |
| 571 | 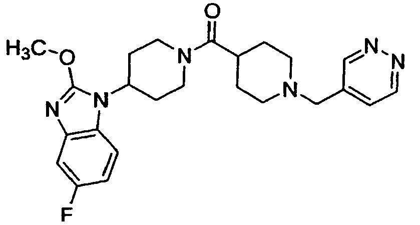 | 453 |
| | | (ESMS) |
| 572 | 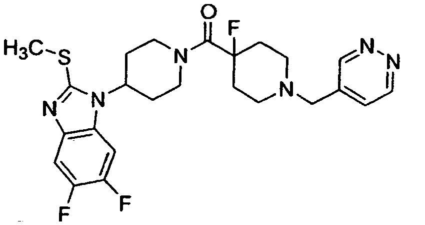 | 505 |
| | | (ESMS) |
| 573 | 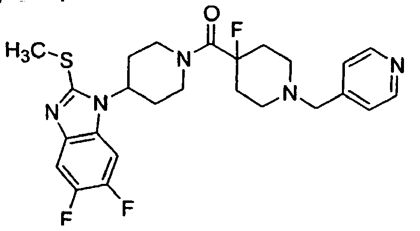 | 504 |
| | | (ESMS) |
| 574 | 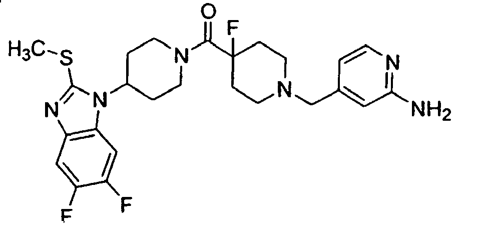 | 519 |
| | | (ESMS) |
| 575 | 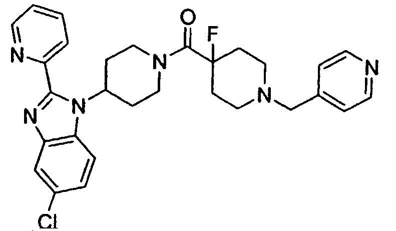 | 533 |
| | | (ESMS) |
| 576 | 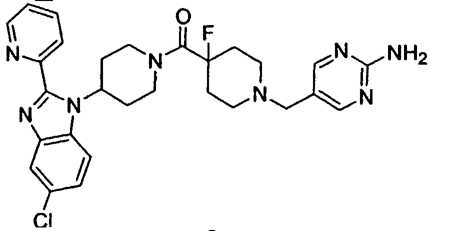 | 549 |
| | | (ESMS) |
| 577 | 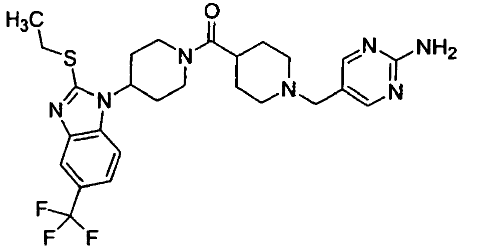 | 548 |
| | | (ESMS) |
| 578 | 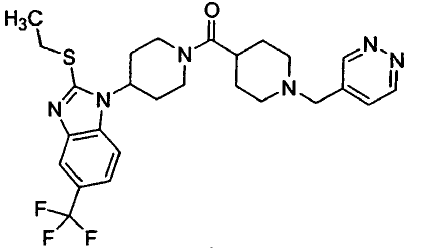 | 533 |
| | | (ESMS) |
| 579 | 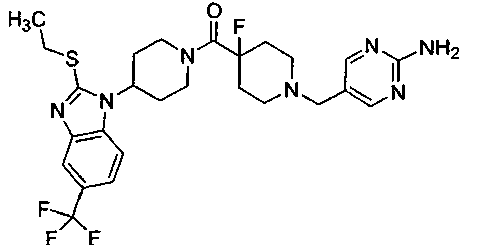 | 566 |
| | | (ESMS) |
| 580 | 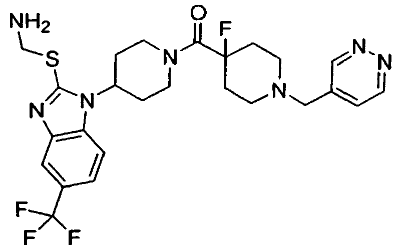 | 551 |
| | | (ESMS) |
| 581 | 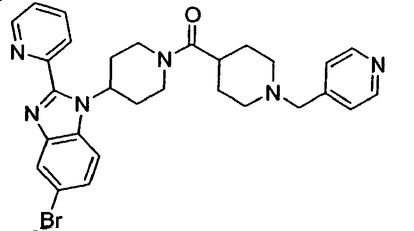 | 559 |
| | | (ESMS) |
| 582 | 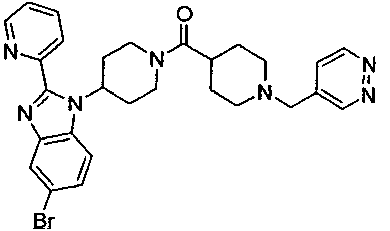 | 560 |
| | | (ESMS) |
| 583 | 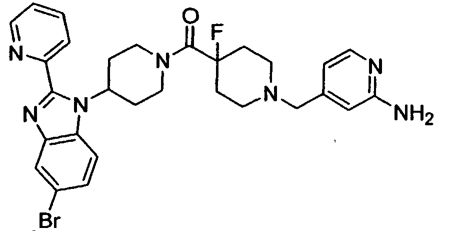 | 592 |
| | | (ESMS) |
| 584 | 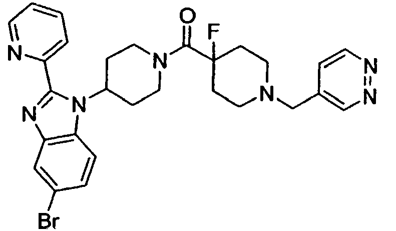 | 579 |
| | | (ESMS) |
| 585 | 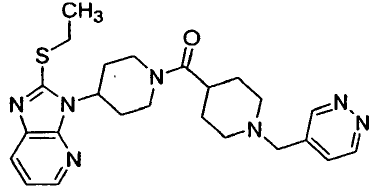 | 466 |
| | | (ESMS) |
| 586 | 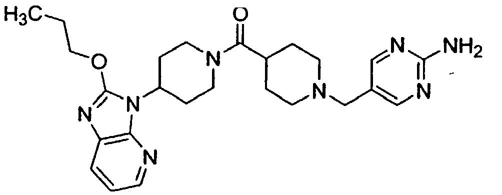 | 479 |
| | | (FAB) |
| 587 | 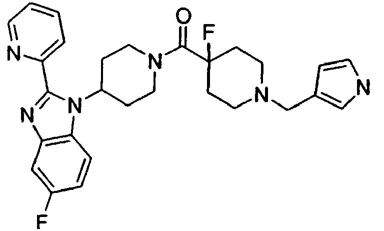 | 505 |
| | | (ESMS) |
| 588 | 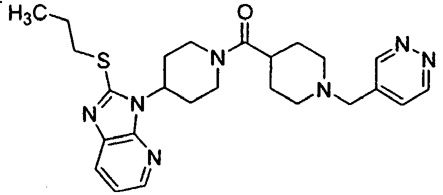 | 480 |
| | | (ESMS) |
| 589 | 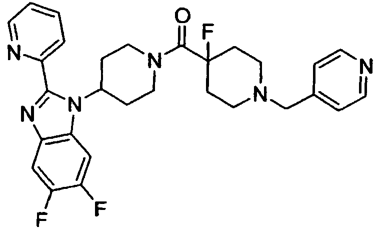 | 535 |
| | | (ESMS) |
| 590 | 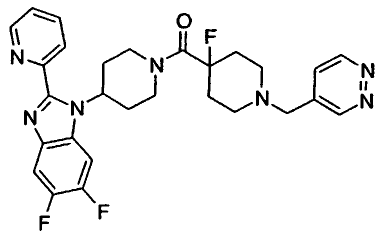 | 536 |
| | | (ESMS) |
| 591 | 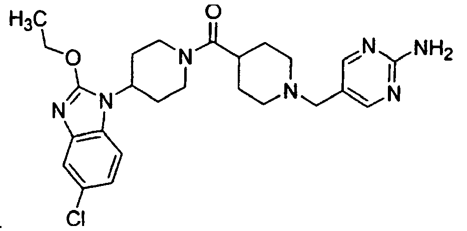 | 498 |
| | | (ESMS) |
| 592 | 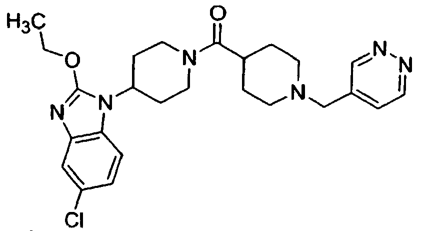 | 483 |
| | | (ESMS) |
| 593 | 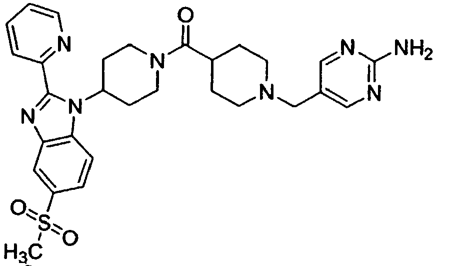 | 575 |
| | | (ESMS) |
| 594 | 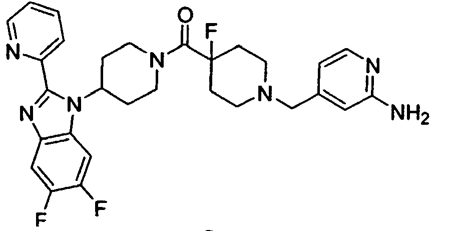 | 550 |
| | | (ESMS) |
| 595 | 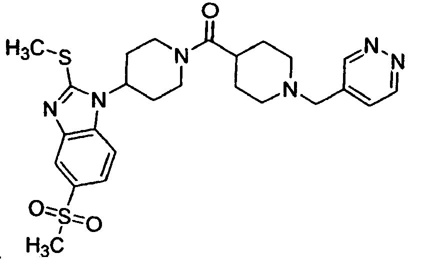 | 529 |
| | | (ESMS) |
| 596 | 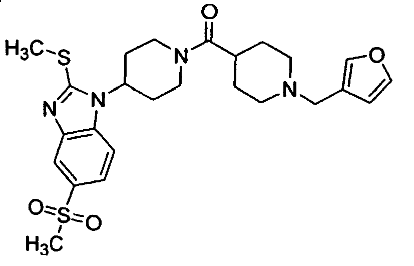 | 517 |
| | | (ESMS) |
| 597 | 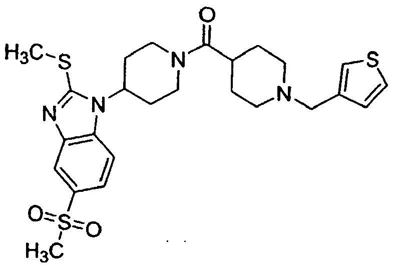 | 533 |
| | | (ESMS) |
| 598 | 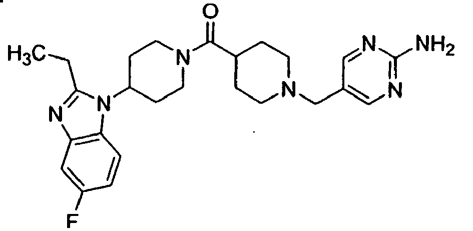 | 466 |
| | | (ESMS) |
| 599 | 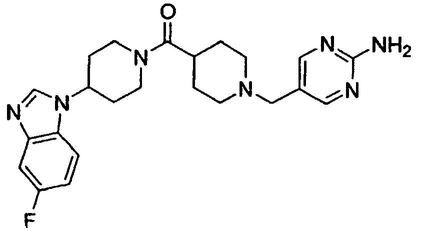 | 438 |
| | | (ESMS) |
| 600 | 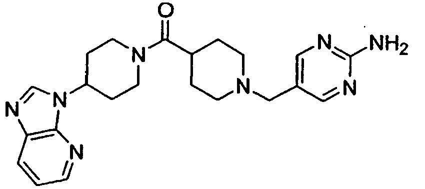 | 421 |
| | | (ESMS) |
| 601 | 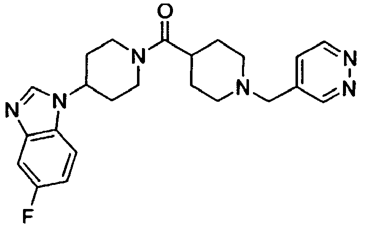 | 423 |
| | | (ESMS) |
| 602 | 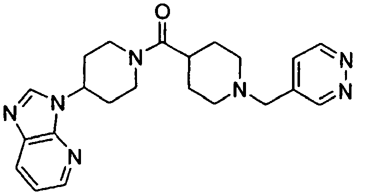 | 406 |
| | | (ESMS) |
| 603 | 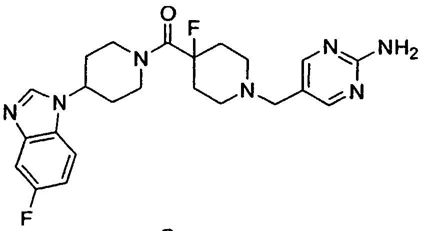 | 456 |
| | | (ESMS) |
| 604 | 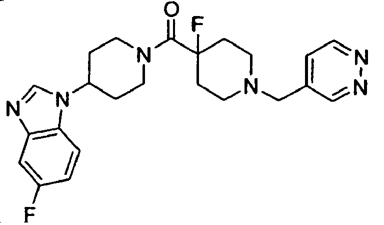 | 441 |
| | | (ESMS) |
| 605 | 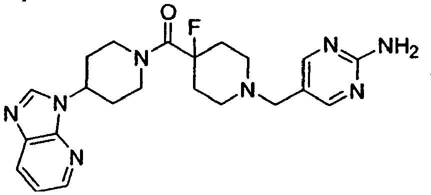 | 439 |
| | | (ESMS) |
| 606 | 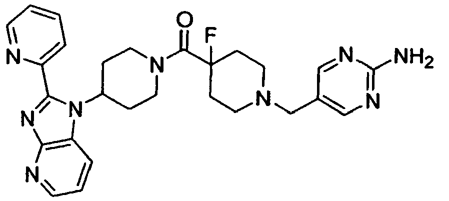 | 516 |
| | | (ESMS) |
| 607 | 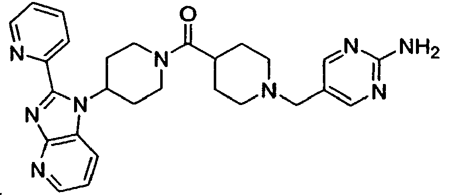 | 498 |
| | | (ESMS) |
| 608 | 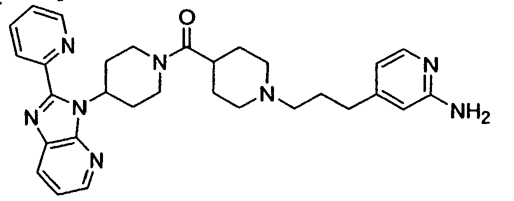 | 525 |
| | | (ESMS) |
| 609 | 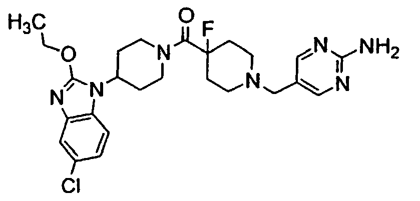 | 516 |
| | | (ESMS) |
| 610 | 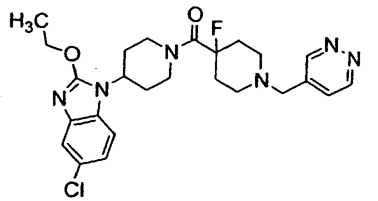 | 501 |
| | | (ESMS) |
| 611 | 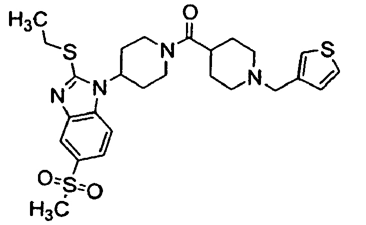 | 547 |
| | | (ESMS) |
| 612 | 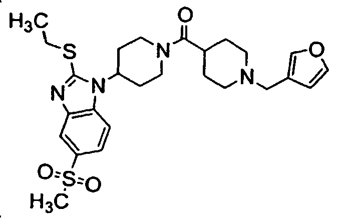 | 531 |
| | | (ESMS) |
| 613 | 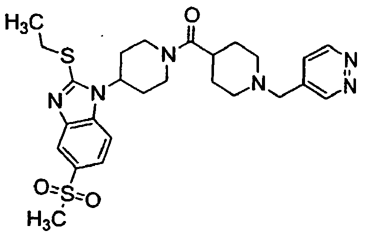 | 543 |
| | | (ESMS) |
| 614 | 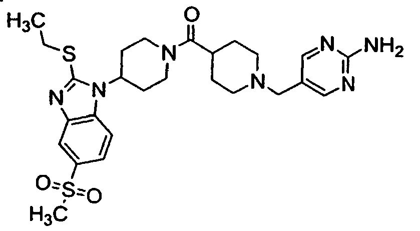 | 558 |
| | | (ESMS) |
| 615 | 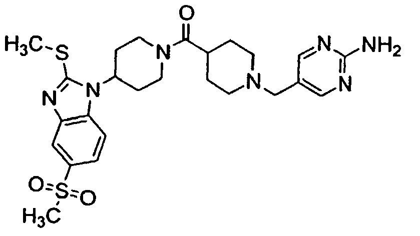 | 544 |
| | | (ESMS) |
| 616 | 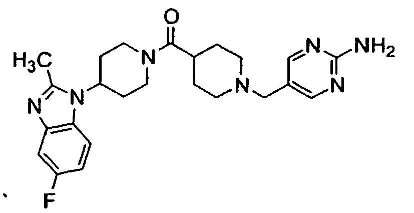 | 452 |
| | | (FAB) |
| 617 | 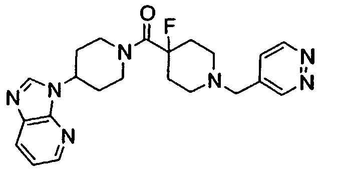 | 424 |
| | | (ESMS) |
| 618 | 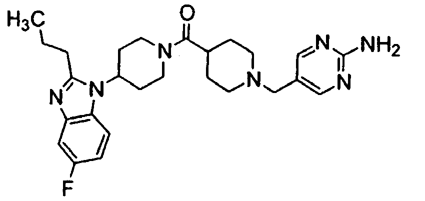 | 480 |
| | | (ESMS) |
| 619 | 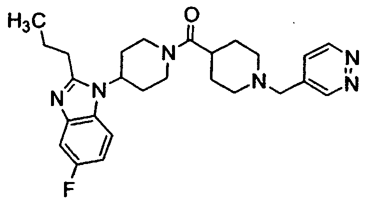 | 465 |
| | | (ESMS) |
| 620 | 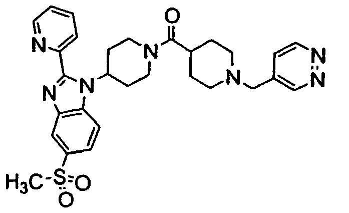 | 560 |
| | | (ESMS) |
| 621 | 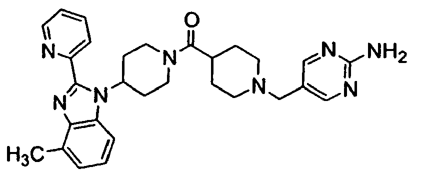 | 511 |
| | | (ESMS) |
| 622 | 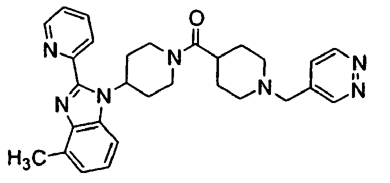 | 496 |
| | | (ESMS) |
| 623 | 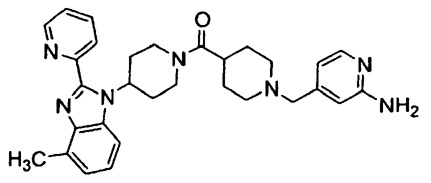 | 510 |
| | | (ESMS) |
| 624 | 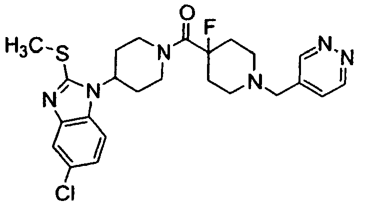 | 503 |
| | | (ESMS) |
| 625 | 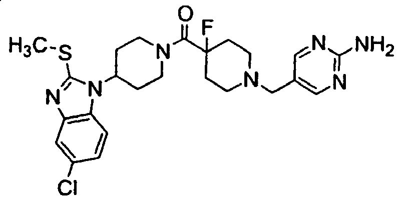 | 518 |
| | | (ESMS) |
| 626 | 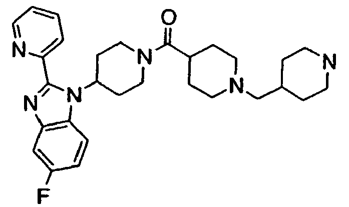 | 505 |
| | | (ESMS) |
| 627 | 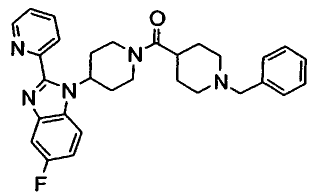 | 498 |
| | | (ESMS) |
| 628 | 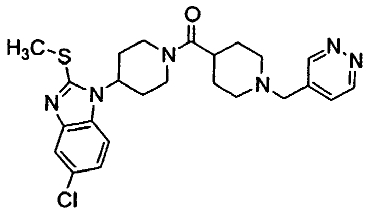 | 485 |
| | | (ESMS) |
| 629 | 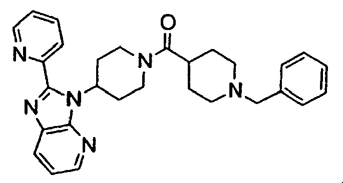 | 481 |
| | | (ESMS) |
| 630 | 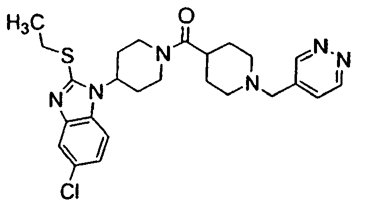 | 499 |
| | | (ESMS) |
| 631 | 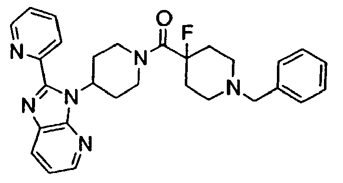 | 499 |
| | | (ESMS) |
| 632 | 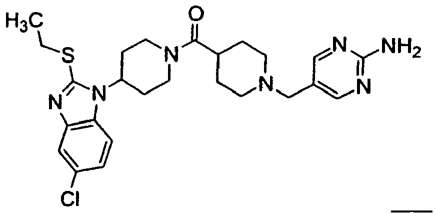 | 514 |
| | | (ESMS) |
| 633 | 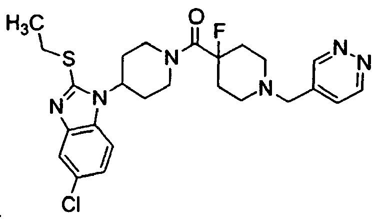 | 517 |
| | | (ESMS) |
| 634 | 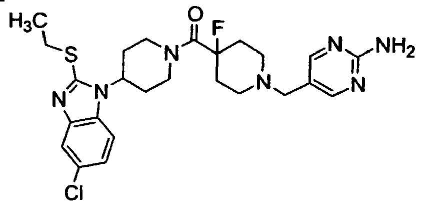 | 532 |
| | | (ESMS) |
| 635 | 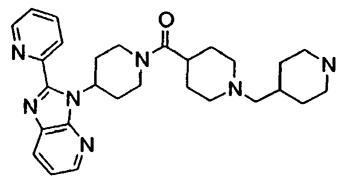 | 488 |
| | | (ESMS) |
| 636 | 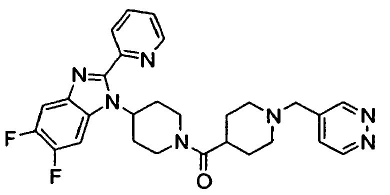 | 518 |
| | | (ESMS) |
| 637 | 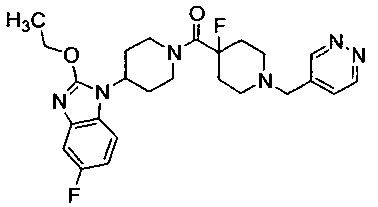 | 451 |
| | | (ESMS) |
| 638 | 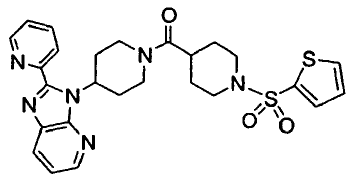 | 537 |
| | | (MH+) |
| 639 | 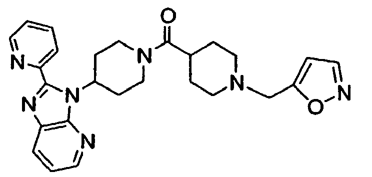 | 472 |
| | | (MH+) |
| 640 | 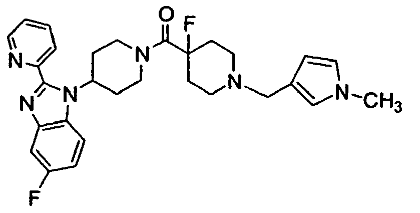 | 519 |
| | | (MH+) |
| 641 | 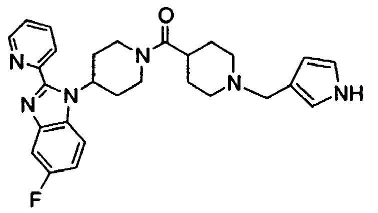 | 487 |
| | | (MH+) |
| 642 | 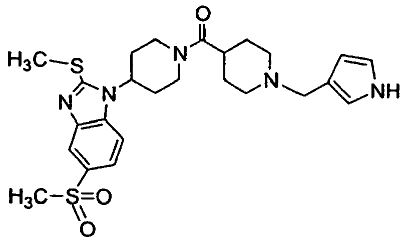 | 516 |
| | | (MH+) |
| 643 | 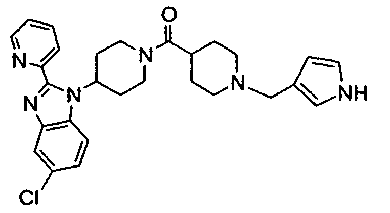 | 503 |
| | | (MH+) |
| 644 | 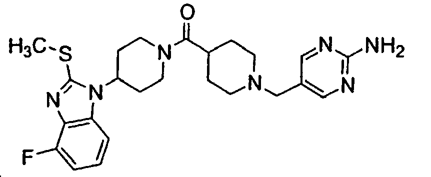 | 484 |
| | | (ESMS) |
| 645 | 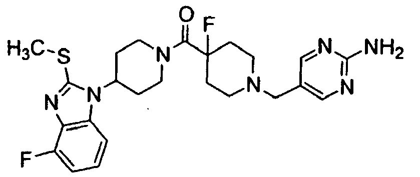 | 503 |
| | | (ESMS) |
| 646 | 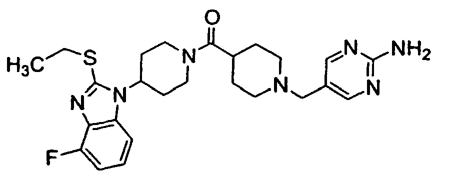 | 498 |
| | | (ESMS) |
| 647 | 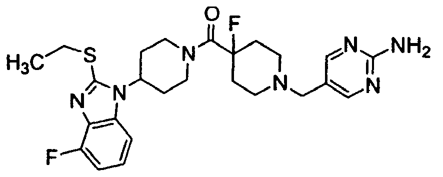 | 516 |
| | | (ESMS) |
| 648 | 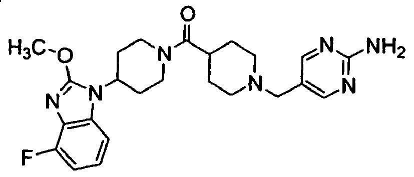 | 468 |
| | | (ESMS) |
| 649 | 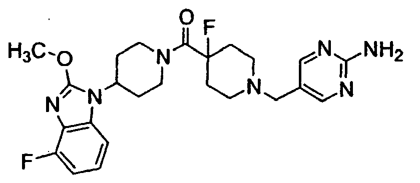 | 486 |
| | | (ESMS) |
| 650 | 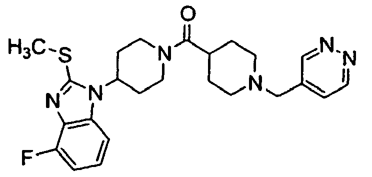 | 469 |
| | | (ESMS) |
| 651 | 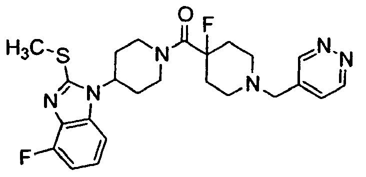 | 487 |
| | | (ESMS) |
| 652 | 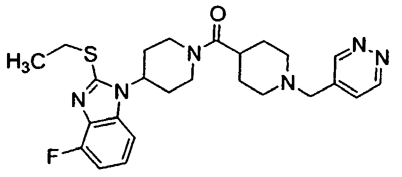 | 483 |
| | | (ESMS) |
| 653 | 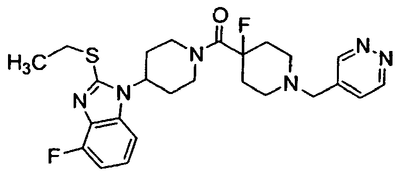 | 501 |
| | | (ESMS) |
| 654 | 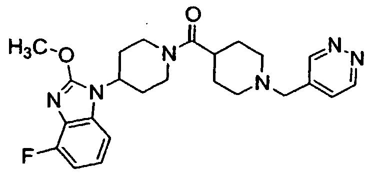 | 453 |
| | | (ESMS) |
| 655 | 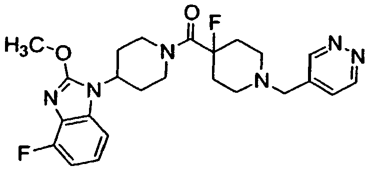 | 471 |
| | | (ESMS) |
| 656 | 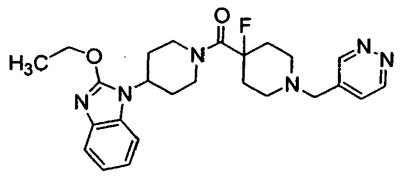 | 468 |
| | | (ESMS) |
| 657 | 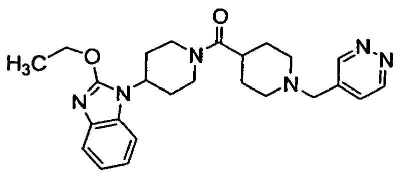 | 450 |
| | | (ESMS) |
| 658 | 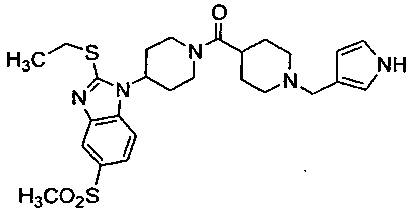 | 530 |
| | | (ESMS) |
| 659 | 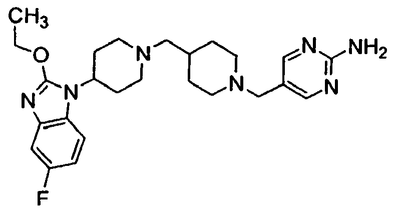 | |
| 660 | 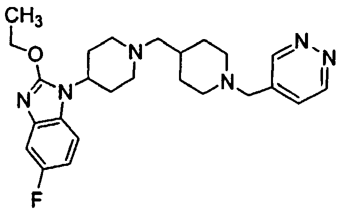 | 453 |
| | | (FAB) |
| 661 | 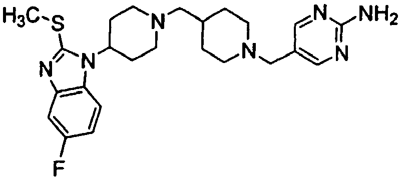 | 470 |
| | | (FAB) |
| 662 | 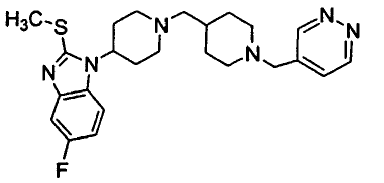 | 455 |
| | | (FAB) |
| 663 | 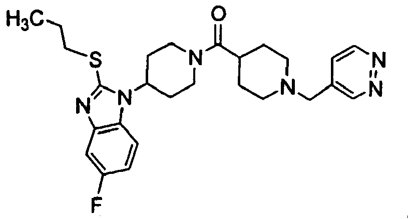 | 497 |
| | | (ESMS) |
| 664 | 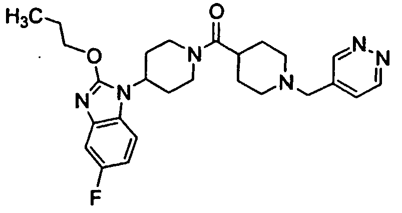 | 481 |
| | | (FAB) |
| 664A | 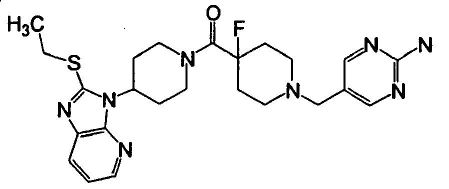 | 499 |
| | | (FAB) |

### Example 665

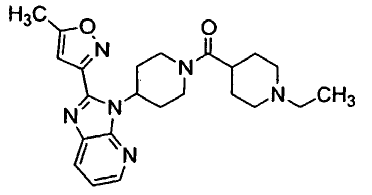

4-[[4-[2-(5-methyl-3-isoxazolyl)-3H-imidazo[4,5-b]pyridine-3-yl]-1-(4-piperidinylcarbonyl)piperidine (0.99 g, 2.51 mmoles) and pyridazine 4-carboxaldehyde (0.35 g, 3.26 mmoles) were stirred at RT in dry CH₂Cl₂ (25 ml) containing activated 3A molecular sieves (6.5 g). After 5 h, triacetoxy borohydride (3.2 g, 15 mmoles) was added and the mixture was stirred for 70 h. The mixture was diluted with CH₂Cl₂ and the solid filtered through a pad of Celite. The filtrate was stirred for 20 min. with saturated aqueous NaHCO₃, then separated, washed with brine, and dried over anahydrous Na₂SO₄. The reaction mixture was purified by preparative TLC. The plates were eluted with EtOAc:Hexanes:CH₃OH(NH₃) (75:20:5). Extraction of the bands with 13% CH₃OH(NH₃)/EtOAc gave a mixture of Example 665 and Example 496. Example 658: MS (M+H): 423.

In a similar manner, using 4-[[4-[2-(methylthio)-3H-imidazo[4,5-b]pyridine-3-yl]-1-(4-piperidinylcarbonyl)piperidine (0.88 gr.;2.44 mmoles), pyridazine 4-carboxaldehyde (0.34 g, 3.18 mmoles), and triacetoxy borohydride, a mixture of Example 666 and Example 495 was prepared:

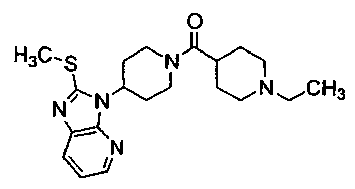

### Example 666: MS (M+H): 388

### General Procedure for H₃₋Receptor Binding Assay

The source of the H₃ receptors in this experiment was guinea pig brain. The animals weighed 400-600 g. The brain tissue was homogenized with a solution of 50 mM Tris, pH 7.5. The final concentration of tissue in the homogenization buffer was 10% w/v. The homogenates were centrifuged at 1,000 x g for 10 min. in order to remove clumps of tissue and debris. The resulting supernatants were then centrifuged at 50,000 x g for 20 min. in order to sediment the membranes, which were next washed three times in homogenization buffer (50,000 x g for 20 min. each). The membranes were frozen and stored at -70°C until needed.

All compounds to be tested were dissolved in DMSO and then diluted into the binding buffer (50 mM Tris, pH 7.5) such that the final concentration was 2 µg/ml with 0.1% DMSO. Membranes were then added (400 µg of protein) to the reaction tubes. The reaction was started by the addition of 3 nM [³H]R-α-methyl histamine (8.8 Ci/mmol) or 3 nM [³H]N^{α}-methyl histamine (80 Ci/mmol) and continued under incubation at 30°C for 30 min. Bound ligand was separated from unbound ligand by filtration, and the amount of radioactive ligand bound to the membranes was quantitated by liquid scintillation spectrometry. All incubations were performed in duplicate and the standard error was always less than 10%. Compounds that inhibited more than 70% of the specific binding of radioactive ligand to the receptor were serially diluted to determine a Kᵢ (nM).

### General Procedure for rHu H₃ Binding Assay

[3H]N^{∝}-methylhistamine (82 Ci/mmole) was obtained from Dupont NEN. Thioperamide was obtained from the Chemical Research Department, Schering-Plough Research Institute.

HEK-293 human embryonic kidney cells stably expressing the human histamine H₃ receptor were cultured in Dulbecco's modified Eagle's medium/10% fetal calf serum/penicillin (100 U/ml)/streptomycin (100 µg/ml)/Geneticin (0.5 mg/ml) at 37° C in a humidified 5% CO₂ atmosphere. Cells were harvested between passages five and twenty at 37° C in 5 mM EDTA/Hank's balanced salt solution and processed for membrane preparation. After low-speed centrifugation, ten min at 1000 xg, they were put into ten volumes of ice-cold buffer and disrupted with a Polytron (PTA 35/2 tip, 30 sec at setting 6). After subsequent low-speed centrifugation, supernatant was centrifuged ten min at 50,000 xg. The high-speed pellet was resuspended in the original volume of buffer, a sample was taken for protein assay (bicinchoninic acid, Pierce) and the suspension was centrifuged again at 50,000 xg. Membranes were resuspended at 1 mg of protein/ml of buffer and frozen at -80° C until use.

Membrane (15 µg of protein) was incubated with 1.2 nM [³H]N^{∝}-methyl-histamine, without or with inhibitor compounds, in a total volume of 200 µl of buffer. Nonspecific binding was determined in the presence of 10⁻⁵ M thioperamide. Assay mixtures were incubated for 30 min at 30° C in polypropylene, 96-well, deep-well plates, then filtered through 0.3% polyethylenimine-soaked GF/B filters. These were washed three times with 1.2 ml of 4° C buffer, dried in a microwave oven, impregnated with Meltilex wax scintillant and counted at 40% efficiency in a Betaplate scintillation counter (Wallac).

IC₅₀ values were interpolated from the data or were determined from curves fit to the data with Prism nonlinear least squares curve-fitting program (GraphPad Software, San Diego, CA). Kᵢ values were determined from IC₅₀ values according to the Cheng and Prusoff equation.

In these assays, compounds of formula I have a Kᵢ within the range of about 0.1 to about 600 nM. Preferred compounds of formula I have a Kᵢ within the range of about 0.1 to about 100 nM. More preferred compounds of formula I have a Kᵢ within the range of about 0.1 to about 20 nM.

Representative compounds of the present invention tested according to the above procedures have the following Ki values:

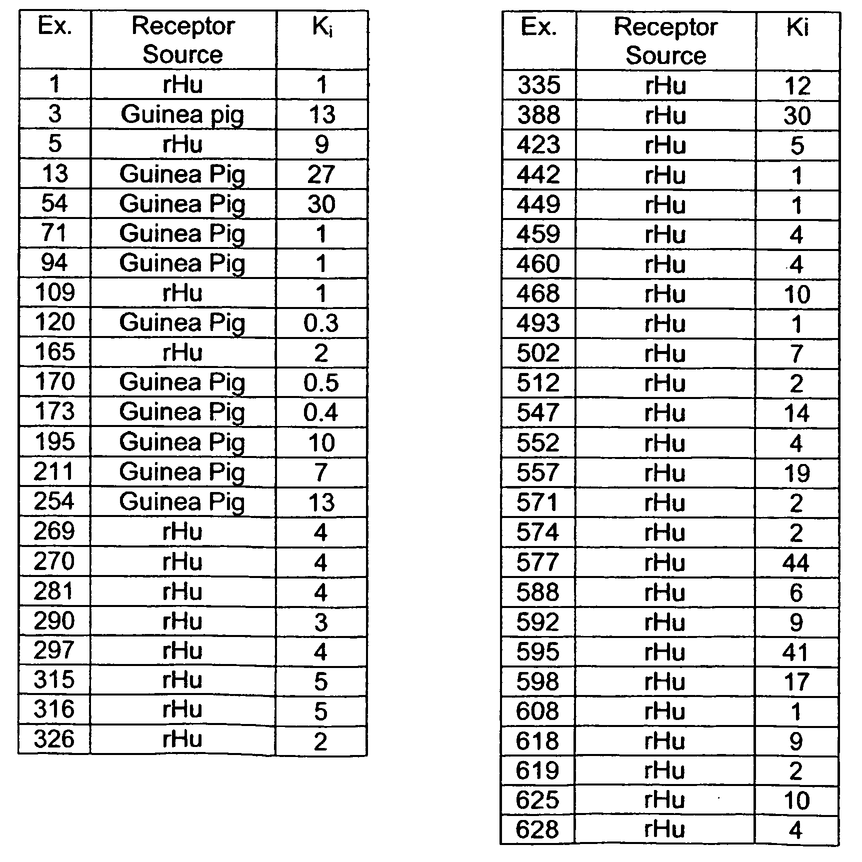

In this specification, the term "at least one compound of formula I" means that one to three different compounds of formula I may be used in a pharmaceutical composition or method of treatment. Preferably one compound of formula I is used. Similarly, "at least one H₁ receptor antagonist" means that one to three different H₁ antagonists may be used in a pharmaceutical composition or method of treatment. Preferably, one H₁ antagonist is used.

For preparing pharmaceutical compositions from the compounds described herein, e.g. from the compounds of the present invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g. magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), The Science and Practice of Pharmacy, 20th Edition, (2000), Lippincott Williams & Wilkins, Baltimore, MD.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compounds disclosed herein, including the compounds of the present invention, may also be deliverable transdermally. The transdermal compositions can take the form of creams, lotions, aerosols and/or emulsions and can be included in a transdermal patch of the matrix or reservoir type as are conventional in the art for this purposes.

Preferably the compound is administered orally.

Preferably, the pharmaceutical preparation is in a unit dosage form. In such form, the preparation is subdivided into suitably sized unit doses containing appropriate quantities of the active component, e.g., an effective amount to achieve the desired purpose.

The quantity of active compound in a unit dose of preparation may be varied or adjusted from about 1 mg to about 350 mg, preferably from about 1 mg to about 150 mg, more preferably from about 1 mg to about 50 mg, according to the particular application.

The actual dosage employed may be varied depending upon the requirements of the patient and the severity of the condition being treated. Determination of the proper dosage regimen for a particular situation is within the skill of the art. For convenience, the total daily dosage may be divided and administered in portions during the day as required.

The amount and frequency of administration of the compounds described herein, including those of the invention, and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 1 mg/day to about 300 mg/day, preferably 1 mg/day to 75 mg/day, in two to four divided doses.

When a combination of H₃ antagonist and H₁ antagonist compounds is to be used, the two active components may be co-administered simultaneously or sequentially, or a single pharmaceutical composition comprising a H₃ antagonist and an H₁ antagonist in a pharmaceutically acceptable carrier can be administered. The components of the combination can be administered individually or together in any conventional dosage form such as capsule, tablet, powder, cachet, suspension, solution, suppository, nasal spray, etc. The dosage of the H₁ antagonist can be determined from published material, and may range from 1 to 1000 mg per dose.

When separate H₃ and H₁ antagonist pharmaceutical compositions are to be administered, they can be provided in a kit comprising in a single package, one container comprising an H₃ antagonist in a pharmaceutically acceptable carrier, and a separate container comprising an H₁ antagonist in a pharmaceutically acceptable carrier, with the H₃ and H₁ antagonists being present in amounts such that the combination is therapeutically effective. A kit is advantageous for administering a combination when, for example, the components must be administered at different time intervals or when they are in different dosage forms.

While the present invention has been described in conjunction with the specific embodiments set forth above, many alternatives, modifications and variations thereof will be apparent to those of ordinary skill in the art.

## Claims

1. A compound represented by the structural formula 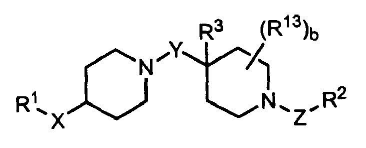 or a pharmaceutically acceptable salt or solvate thereof, wherein:
b is 0 to 2;
X is a bond or C₁-alkylene;
Y is -C(O)-, -C(S)-, -CH₂- or -SO₂-;
Z is a bond, C₁-C₆ alkylene, C₁-C₆ alkenylene, -C(O)-, -CH(CN)-, -SO₂- or -CH₂C(O)NR⁴-;
R¹ is 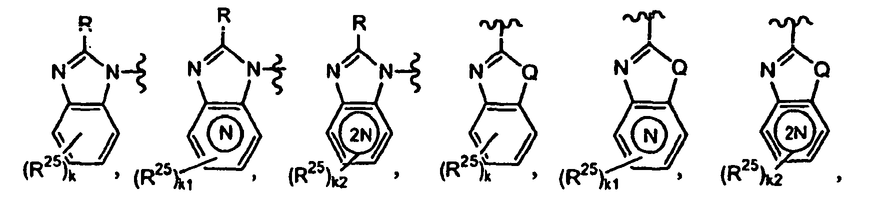 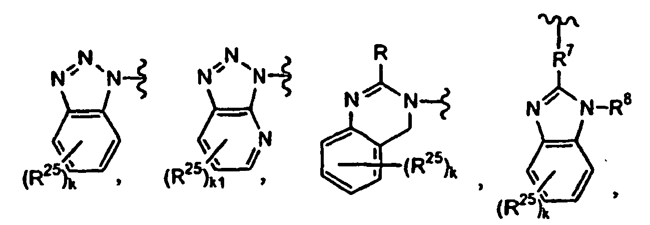 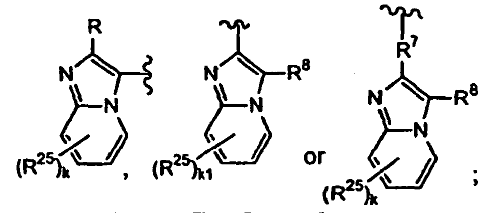
Q is -N(R⁸)-, -S- or -O-;
k is 0, 1, 2, 3 or 4;
k1 is 0, 1, 2 or 3;
k2 is 0, 1 or 2;
R is H, C₁-C₆ alkyl, halo(C₁-C₆)alkyl-, C₁-C₆ alkoxy, (C₁-C₆)alkoxy-(C₁-C₆)alkyl-, (C₁-C₆)-alkoxy-(C₁-C₆)alkoxy, (C₁-C₆)alkoxy-(C₁-C6)alkyl-SO₀₋₂, R³²-aryl(C₁-C₆)alkoxy-, R³²-aryl(C₁-C₆)alkyl-, R³²-aryl, R³²-aryloxy, R³²-heteroaryl, (C₁-C₆)cycloalkyl, (C₃-C₆)cycloalkyl-(C₁-C₆)alkyl, (C₃-C₆)cycloalkyl-(C₁-C₆)alkoxy, (C₃-C₆)cycloalkyl-oxy-, R³⁷-heterocycloalkyl, R³⁷-heterocycloalkyl-oxy-, R³⁷-heterocycloalkyl-(C₁-C₆)alkoxy, N(R³⁰)(R³¹)-(C₁-C₆)alkyl-, -N(R³⁰)(R³¹), -NH-(C₁-C₆)alkyl-O-(C₁-C₆)alkyl, -NHC(O)NH(R²⁹); R²⁹-S(O)₀₋₂-, halo(C₁-C₆)alkyl-S(O)₀₋₂-, N(R³⁰)(R³¹)-(C₁-C₆)alkyl-S(O)₀₋₂- or benzoyl;
R⁸ is H, C₁-C₆ alkyl, halo(C₁-C₆)alkyl-, (C₁-C₆)alkoxy-(C₁-C₆)alkyl-, R³²-aryl(C₁-C₆)alkyl-, R³²-aryl, R³²-heteroaryl, (C₃-C₆)cycloalkyl, (C₃-C₆)cycloalkyl-(C₁-C₆)alkyl, R³⁷-heterocycloalkyl, N(R³⁰)(R³¹)-(C₁-C₆)alkyl-, R²⁹-S(O)₂-, halo(C₁-C₆)alkyl-S(O)₂-, R²⁹-S(O)₀₋₁-(C₂-C₆)alkyl-, halo(C₁-C₆)alkyl-S(O)₀₋₁-(C₂-C₆)alkyl-;
R² is a six-membered heteroaryl ring having 1 or 2 heteroatoms independently selected from N or N-O, with the remaining ring atoms being carbon; a five-membered heteroaryl ring having 1, 2, 3 or 4 heteroatoms independently selected from N, O or S, with the remaining ring atoms being carbon; R³²-quinolyl; heterocycloalkyl; (C₃-C₆)cycloalkyl: C₁-C₆ alkyl; hydrogen; thianaphthenyl; 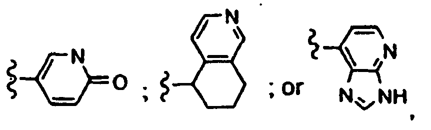 wherein said six-membered heteroaryl ring or said five-membered heteroaryl ring is optionally substituted by R⁶;
R³ is H, halogen, C₁-C₆ alkyl, -OH, (C₁-C₆)alkoxy ou -NHSO₂-(C₁-C₆)alkyl;
R⁴ is independently selected from the group consisting of hydrogen, C₁-C₆ alkyl, C₃-C₆ cycloalkyl, (C₃-C₆)cycloalkyl(C₁-C₆)alkyl, R³³-aryl, R³³-aryl(C₁-C₆)alkyl, and R³²-heeroaryl;
R⁵ is hydrogen, C₁C₆ alkyl, -C(O)R²⁰, -C(O)₂R²⁰, -C(O)N(R²⁰)₂, (C₁-C₆)alkyl-SO₂, or (C₁-C₆)alkyl-SO₂-NH-;
or R⁴ and R⁵, together with the nitrogen to which they are attached, form an azetidinyl, pyrrolidinyl, piperidinyl, piperazinyl or morpholinyl ring;
R⁶ is 1 to 3 substituents independently selected from the group consisting of -OH, halogen, C₁-C₆ alkyl, C₁-C₆ alkoxy, C₁-C₆ alkylthio, -CF₃, -NR⁴R⁵n -CH₂-NR⁴R⁵, -NHSO₂R²², -N(SO₂R²²)₂, phenyl, R³³-phenyl, NO₂, -CO₂R⁴, -CON(R⁴)₂, 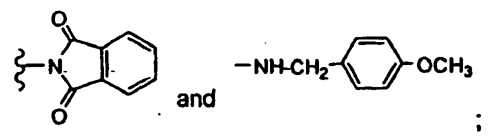
R⁷ is -N(R²⁹)-, -O- or -S(O)₀₋₂-;
R¹³ is independently selected from the group consisting of C₁-C₆ alkyl, hydroxyl, C₁-C₆ alkoxy, or fluoro, provided that when R¹³ is hydroxy or fluoro then R¹³ is not bound to a carbon adjacent to a nitrogen; or two R¹³ substituents form a C₁ to C₂ alkyl bridge from one ring carbon to another non-adjacent ring carbon; or R¹³ is =O;
R²⁰ is independently selected from the group consisting of hydrogen, C₁-C₆ alkyl, or aryl, wherein said aryl group is optionally substituted with from 1 to 3 groups independently selected from halogen, -CF₃, -OCF₃, hydroxyl, or methoxy; or when two R²⁰ groups are present, said two R²⁰ groups taken together with the nitrogen to which they are bound can form a five or six membered heterocyclic ring;
R²² is C₁-C₆ alkyl, R³⁴-aryl or heterocycloalkyl;
R²⁴ is H, C₁-C₆ alkyl, -SO₂R²² or R³⁴-aryl;
R²⁵ is independently selected from the group consisting of C₁-C₆ alkyl, halogen, -CN, -NO₂, -CF₃, -OH, C₁-C₆ alkoxy, (C₁-C₆)alkyl-C(O)-, aryl-C(O)-, -C(O)OR²⁹, -N(R⁴)(R⁵), N(R⁴)(R⁵)-C(O)-, N(R⁴)(R⁵)-S(O)₁₋₂-, R²²-S(O)₀₋₂-, halo-(C₁-C₆)alkyl- or halo-(C₁-C₆)alkoxy-(C₁-C₆)alkyl-;
R²⁹ is H, C₁-C₆ alkyl, C₃-C₆ cycloalkyl, R³⁵-aryl or R³⁵-aryl(C₁-C₆)alkyl-;
R³⁰ is H, C₁-C₆ alkyl-, R³⁵-aryl or R³⁵-aryl(C₁-C₆)alkyl-;
R³¹ is H, C₁-C₆ alkyl-, R³⁵-aryl, R³⁵-aryl(C₁-C₆)alkyl-, R³⁵-heteroaryl, (C₁-C₆)alkyl-C(O)-, R³⁵-aryl-C(O)-, N(R⁴)(R⁵)-C(O)-, (C₁-C₆)alkyl-S(O)₂- or R³⁵-aryl-S(O)₂-;
or R³⁰ and R³¹ together are -(CH₂)₄₋₅-, -(CH₂)₂-O-(CH₂)₂- or -(CH₂)₂-N(R³⁸)-(CH₂)₂- and form a ring with the nitrogen to which they are attached;
R³² is 1 to 3 substituents independently selected from the group consisting of H, -OH, halogen, C₁-C₆ alkyl, C₁-C₆ alkoxy, R³⁵-aryl-O-, -SR²², -CF₃, -OCF₃, -OCHF₂, -NR³⁹R⁴⁰, phenyl, R³³-phenyl, NO₂, -CO₂R³⁹, -CON(R³⁹)₂, -S(O)₂R²², -S(O)₂N(R²⁰)₂, -N(R²⁴)S(O)₂R²², -CN, hydroxy-(C₁-C₆)alkyl-, -OCH₂CH₂OR²², and
R³⁵-aryl(C₁-C₆)alkyl-O-, or two R³² groups on adjacent carbon atoms together form a -OCH₂O- or -O(CH₂)₂O- group;
R³³ is 1 to 3 substituents independently selected from the group consisting of C₁-C₆ alkyl, halogen, -CN, -NO₂, -CF₃, -OCF₃, -OCHF₂ and -O(C₁-C₆)alkyl;
R³⁴ is 1 to 3 substituents independently selected from the group consisting of H, halogen, -CF₃, -OCF₃, -OH and -OCH₃;
R³⁵ is 1 to 3 substituents independently selected from hydrogen, halo, C₁-C₆ alkyl, hydroxy, C₁-C₆ alkoxy, phenoxy, -CF₃, -N(R³⁶)₂, -COOR²⁰ and -NO₂;
R³⁶ is independently selected form the group consisting of H and C₁-C₆ alkyl;
R³⁷ is 1 to 3 substituents independently selected from hydrogen, halo, C₁-C₆ alkyl, hydroxy, C₁-C₆ alkoxy, phenoxy, -CF₃, -N(R³⁶)₂, -COOR²⁰, -C(O)N(R²⁹)₂ and -NO₂, or R³⁷ is one or two =O groups;
R³⁸ is H, C₁-C₆ alkyl, R³⁵-aryl, R³⁵-aryl(C₁-C₆)alkyl-, (C₁-C₆)alkyl-SO₂ or halo(C₁-C₆)alkyl-SO₂-;
R³⁹ is independently selected from the group consisting of hydrogen, C₁-C₆ alkyl, C₃-C₆ cycloalkyl, (C₃-C₆)cycloalkyl(C₁-C₆)alkyl, R³³-aryl, R³³-aryl(C₁-C₆)alkyl, and R³²-heteroaryl; and
R⁴⁰ is hydrogen, C₁-C₆ alkyl, -C(O)R²⁰, -C(O)₂R²⁰, -C(O)N(R²⁰)₂, (C₁-C₆)alkyl-SO₂-, or (C₁-C₆)alkyl-SO₂-NH-;
or R³⁹ and R⁴⁰, together with the nitrogen to which they are attached, form an azetidinyl, pyrrolidinyl, piperidinyl, piperazinyl or morpholinyl ring;
aryl represents a carbocyclic group containing from 6 to 14 carbon atoms and having at least one aromatic ring;
heteroaryl represents a cyclic group, having 1 to 4 heteroatoms selected from O, S and N, said heteroatom interrupting a carbocyclic ring structure and having a sufficient number of delocalized pi electrons to provide aromatic character, the aromatic heterocyclic group containing from 2 to 14 carbon atoms; and
heterocycloalkyl represents a group selected from 2-tetrahydrofuranyl, 3-tetrahydrofuranyl, 2-tetrahydrothienyl, 3-tetrahydrothienyl, 2-piperidinyl, 3-piperidinyl, 4-piperidinyl, 2-pyrrolidinyl, 3-pyrrolidinyl, 2-piperazinyl, 3-piperazinyl, 2-dioxanyl, 4-dioxanyl, 1,3-dioxolanyl, 1,3,5-trithianyl, pentamethylene sulfide, perhydroisoquinolinyl, decahydroquinolinyl, trimethylene oxide, azetidinyl, 1-azacycloheptanyl, 1,3-dithianyl, 1,3,5-trioxanyl, morpholinyl, thiomorpholinyl, 1,4-thioxanyl, 1,3,5-hexahydrotriazinyl, thiazolidinyl and tetrahydropyranyl.

2. A compound of claim 1 wherein R³ is hydrogen or fluorine, and b is 0.

3. A compound of claim 2 wherein X is a bond.

4. A compound of claim 3 wherein Y is -C(O)-.

5. A compound of claim 4 wherein Z is straight or branched C₁-C₃ alkyl.

6. A compound of claim 5 wherein R² is a six-membered heteroaryl ring, optionally substituted with one R⁶ substituent.

7. A compound of claim 6 wherein R² is pyrimidyl, R⁶⁻pyrimidyl, pyridyl, R⁶⁻pyridyl or pyridazinyl and R⁶ is -NH₂.

8. A compound of claim 7 wherein R² is

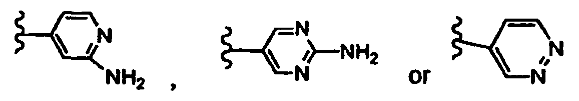

9. A compound of claim 1 wherein R¹ is

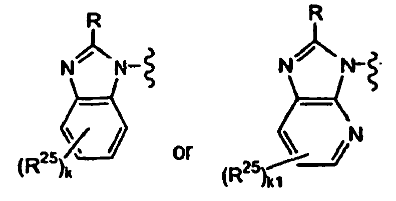

10. A compound of claim 9 wherein R is (C₁-C₆)alkyl, (C₁-C₆)alkoxy, (C₁-C₆)alkoxy(C₁-C₆)alkoxy, (C₁-C₆)alkylthio, heteroaryl or R³²-aryl; R²⁵ is halogen or - CF₃; and k and k1 are 0 or 1.

11. A compound of claim 10 wherein R is -CH₃, -CH₂CH₃, -OCH₃, -OCH₂CH₃,-OCH₂CH₂CH₃, -OCH((CH₃)₂, -CH₂CH₃, -SCH₃, -SCH₂CH₃, pyridyl, pyrimidyl, pyrazinyl, furanyl, oxazolyl or R³²-phenyl.

12. A compound of claim 11 wherein R² is

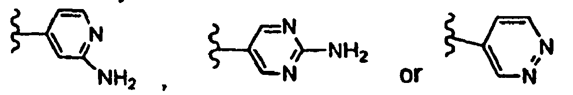

13. A compound of claim 1 selected from the group consisting of 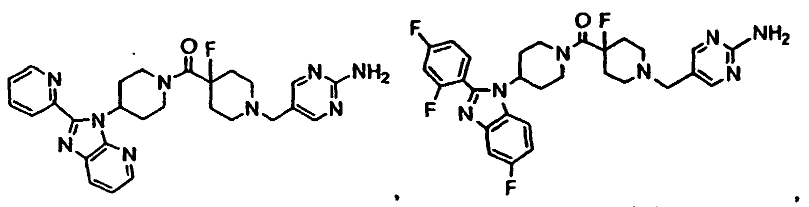 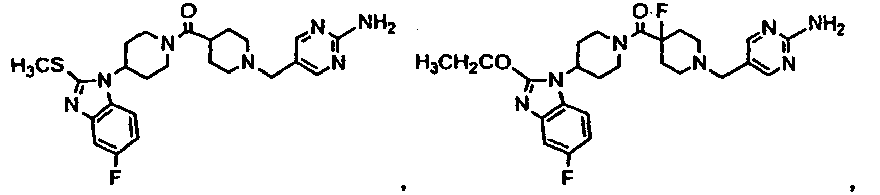 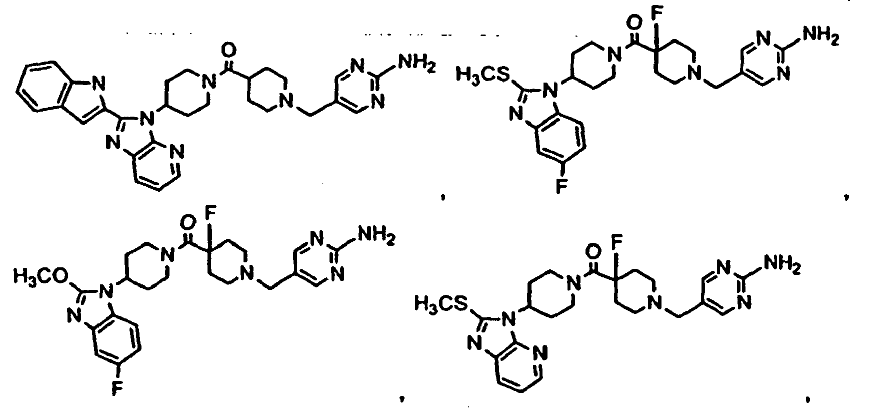 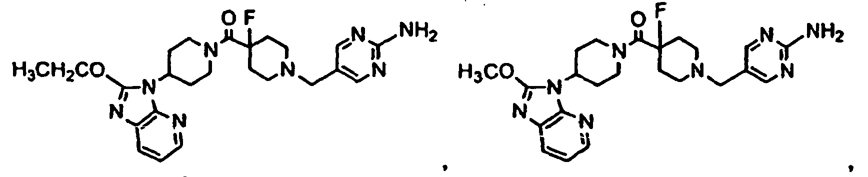 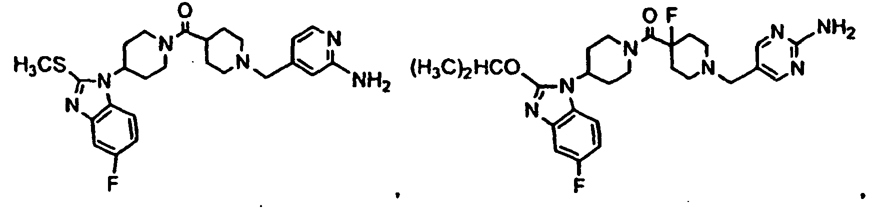 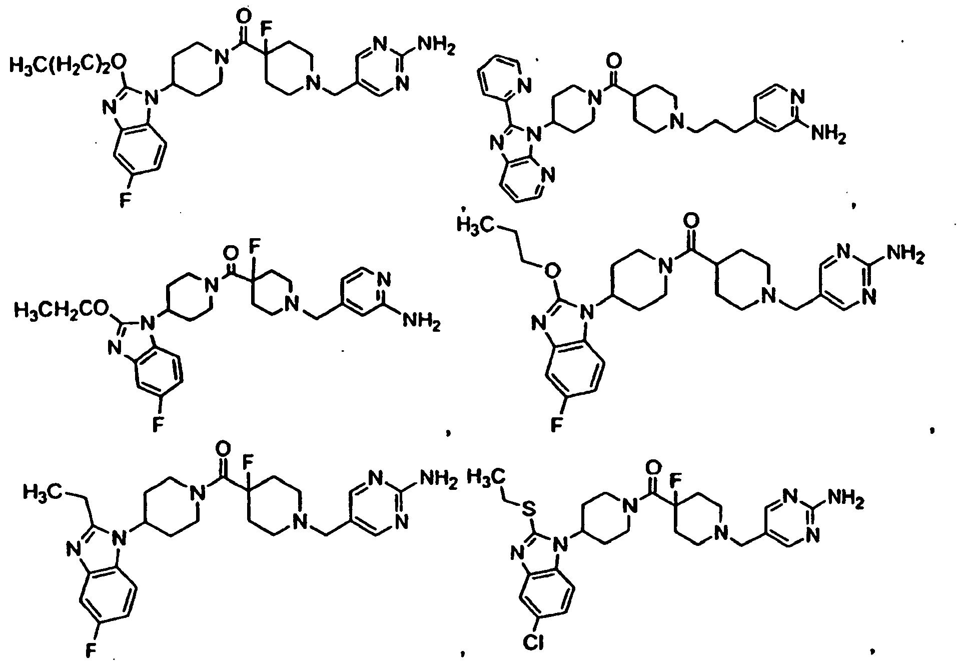 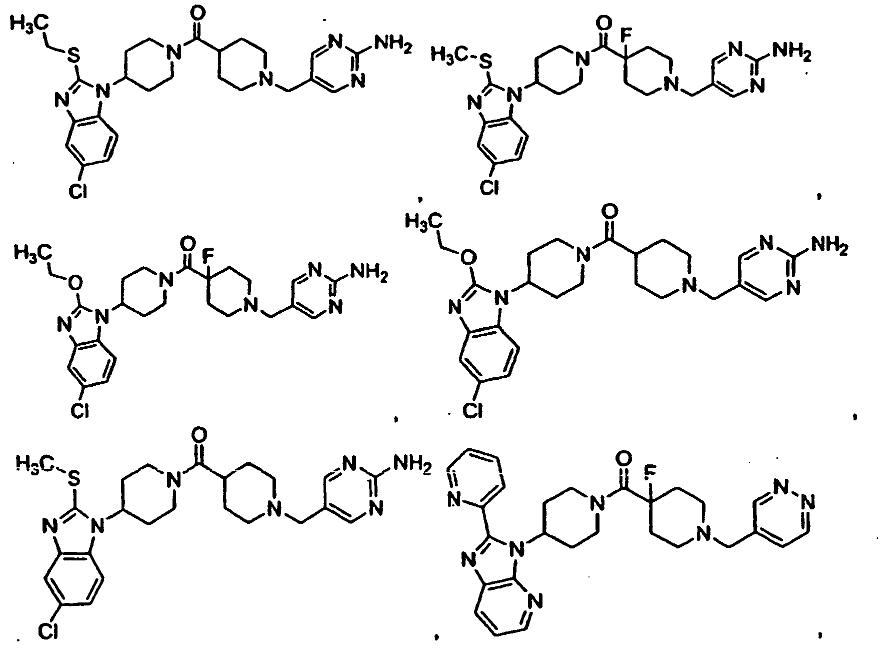 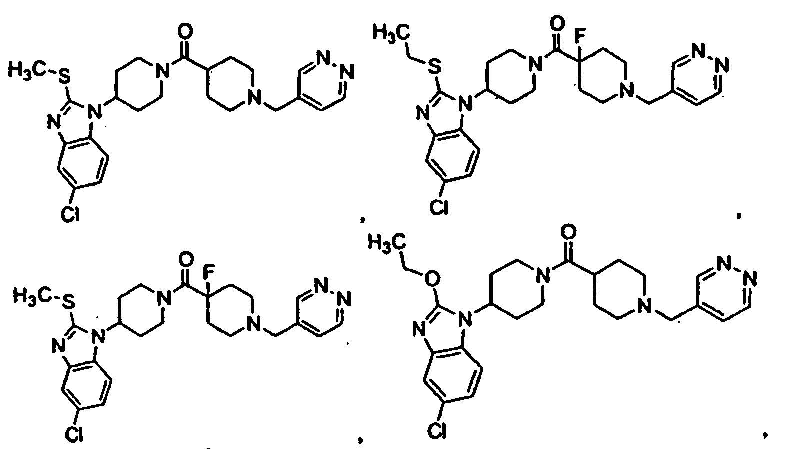 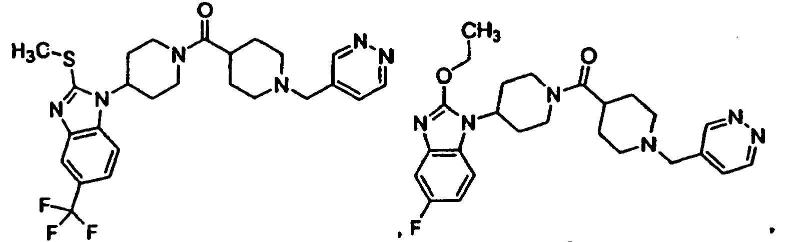 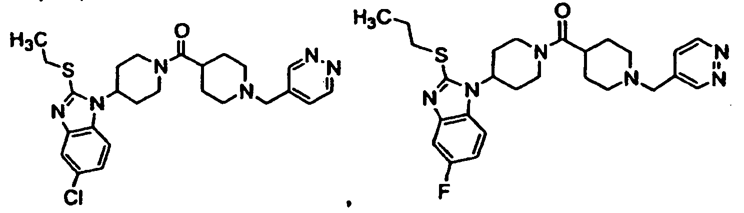 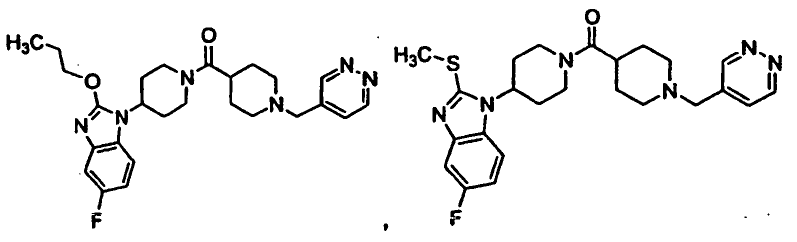 and

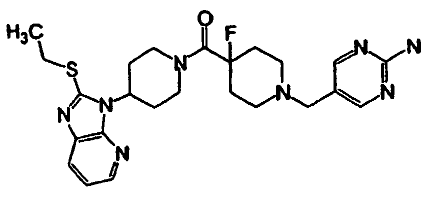

14. A compound having the structure: 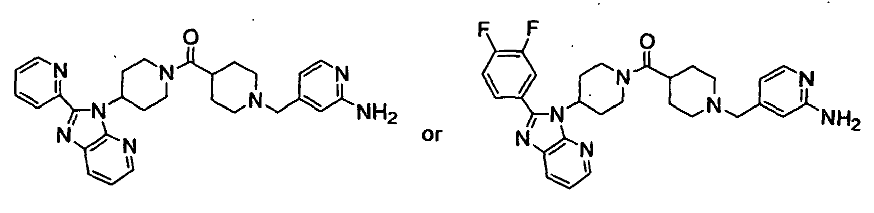 or a pharmaceutically acceptable salt or solvate thereof.

15. A pharmaceutical composition comprising an effective amount of a compound of claim 1 or claim 14 and a pharmaceutically effective carrier.

16. The use of a compound of claim 1 or claim 14 for the preparation of a medicament for treating allergy, allergy-induced airway responses, congestion, hypotension, cardiovascular disease, diseases of the GI tract, hyper and hypo motility and acidic secretion of the gastro-intestinal tract, obesity, sleeping disorders, disturbances of the central nervous system, attention deficit hyperactivity disorder, hypo and hyperactivity of the central nervous system, Alzheimer's disease, schizophrenia, or migraine.

17. A pharmaceutical composition comprising an effective amount of a compound of claim 1 or claim 14, and an effective amount of an H₁ receptor antagonist, and a pharmaceutically effective carrier.

18. The use of claim 16, wherein the medicament is to be used in combination with an H₁ receptor antagonist.

19. The use or claim 18 wherein the medicament is for treating allergy, allergy-induced airway responses, and congestion.

20. The pharmaceutical composition of claim 17 or the use of claim 18 wherein said H, receptor antagonist is selected from: astemizole, azatadine, azelastine, acrivastine, brompheniramine, cetirizine, chlorpheniramine, clemastine, cyclizine, carebastine, cyproheptadine, carbinoxamine, descarboethoxyloratadine, diphenhydramine, doxylamine, dimethindene, ebastine, epinastine, efletirizine, fexofenadine, hydroxyzine, ketotifen, loratadine, levocabastine, meclizine, mizolastine, mequitazine, mianserin, noberastine, norastemizole, picumast, pyrilamine, promethazine, terfenadine, tripelennamine, temelastine, trimeprazine or triprolidine.

21. The pharmaceutical composition of claim 17 or the use of claim 18 wherein the H₁ receptor antagonist is loratadine or descarboethoxyloratadine.

## Patentansprüche

1. Verbindung mit der Strukturformel 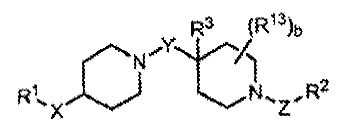 oder ein pharmazeutisch annehmbares Salz oder Solvat davon, worin
b 0 bis 2 ist;
X eine Bindung oder C₁-Alkylen ist;
Y -C(O)-, -C(S)-, -(CH₂)- oder -SO₂- ist;
Z eine Bindung, C₁-C₆-Alkylen, C₁-C₆-Alkenylen, -C(O)-, -CH(CN)-, -SO₂- oder -CH₂C(O)NR⁴- ist;
R¹ 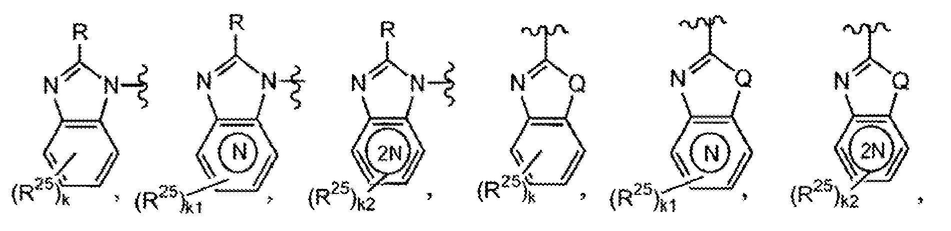 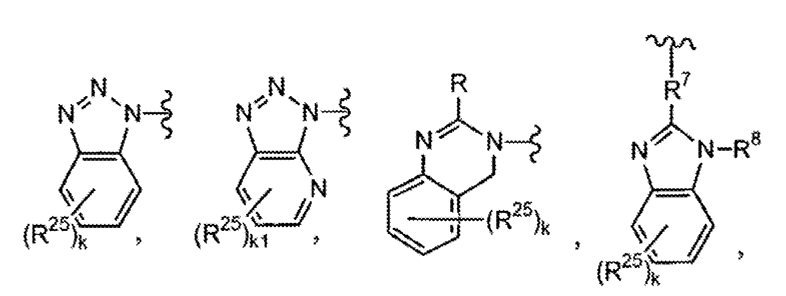 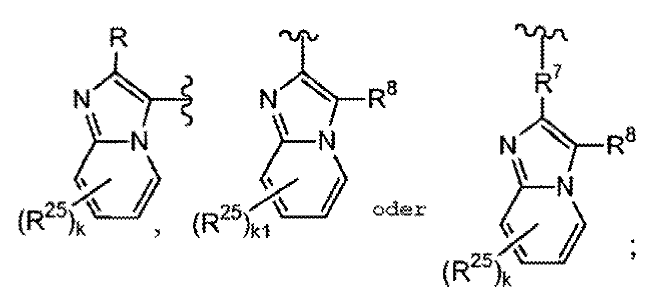 ist,
Q -N(R⁸)-, -S- oder -O- ist;
k 0, 1, 2, 3 oder 4 ist,
k1 0, 1, 2 oder 3 ist,
k2 0, 1 oder 2 ist,
R H, C₁-C₆-Alkyl, Halogen (C₁-C₆) alkyl-, C₁-C₆-Alkoxy, (C₁-C₆)-Alkoxy-(C₁-C₆)alkyl-, (C₁-C₆)-Alkoxy-(C₁-C₆)alkoxy, (C₁-C₆)-Alkoxy-(C₁-C₆)alkyl-SO₀₋₂, R³²-Aryl(C₁-C₆)alkoxy-, R³²⁻Aryl(C₁-C₆)alkyl-, R³²-Aryl, R³²-Aryloxy_{,} R³²-Heteroaryl, (C₃-C₆)-Cycloalkyl, (C₃-C₆)-Cycloalkyl(C₁-C₆)alkyl, (C₃-C₆)-Cycloalkyl(C₁-C₆)alkoxy, (C₃-C₆)-Cycloalkyloxy-, R³⁷-Heterocycloalkyl, R³⁷-Heterocycloalkyloxy-, R³⁷-Heterocycloalkyl-(C₁-C₆)alkoxy, N(R³⁰)(R³¹)-(C₁-C₆)-alkyl-, -N(R³⁰)(R³¹), -NH-(C₁-C₆)-Alkyl-O-(C₁-C₆)alkyl, -NHC(O)NH(R²⁹); R²⁹-S(O)₀₋₂-, Halogen(C₁-C₆)alkyl-S(O)₀₋₂, N (R³⁰)(R³¹)(C₁-C₆)-Alkyl-S(O)₀₋₂- oder Benzoyl ist;
R⁸ H, C₁-C₆-Alkyl, Halogen (C₁-C₆)alkyl-, (C₁-C₆)-Alkoxy-(C₁-C₆)alkyl-, R³²-Aryl(C₁-C₆)alkyl-, R³²-Aryl, R³²-Heteroaryl, (C₃-C₆)-Cycloalkyl, (C₃-C₆)-Cycloalkyl-(C₁-C₆)alkyl, R³⁷-Heterocycloalkyl, N(R³⁰)(R³¹)-(C₁-C₆)-Alkyl-, R²⁹-S(O)₂, Halogen(C₁-C₆)alkyl-S(O)₂-, R²⁹-S(O)₀₋₁-(C₂-C₆)-Alkyl, Halogen (C₁-C₆)alkyl-S(O)₀₋₁-(C₂-C₆)alkyl- ist;
R² ein sechsgliedriger Heteroarylring mit 1 oder 2 Heteroatomen, die unabhängig aus N oder N-O ausgewählt sind, wobei die restlichen Ringatome Kohlenstoff sind; ein fünfgliedriger Heteroarylring mit 1, 2, 3 oder 4 Heteroatomen, die unabhängig aus N, 0 oder S ausgewählt sind, wobei die restlichen Ringatome Kohlenstoff sind; R³²-Chinolyl; Heterocycloalkyl; (C₃-C₆)-Cycloalkyl; C₁-C₆-Alkyl; Wasserstoff, Thianaphtenyl; 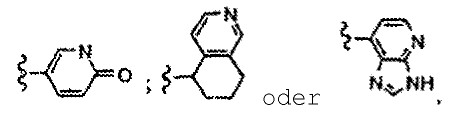 ist,
wobei der sechsgliedrige Heteroarylring oder der fünfgliedrige Heteroarylring gegebenenfalls mit R⁶ substituiert ist;
R³ H, Halogen, C₁-C₆-Alkyl, -OH, (C₁-C₆)-Alkoxy oder -NHSO₂-(C₁-C₆)alkyl ist;
R⁴ unabhängig ausgewählt ist aus der Gruppe bestehend aus Wasserstoff, C₁-C₆-Alkyl, C₃-C₆-Cycloalkyl, (C₃-C₆)-Cycloalkyl (C₁-C₆) alkyl, R³³-Aryl, R³³-Aryl (C₁-C₆)alkyl und R³²-Heteroaryl;
R⁵ Wasserstoff, C₁-C₆-Alkyl, -C(O)R²⁰, -C(O)₂R²⁰, -C(O)N(R²⁰)₂, (C₁-C₆)-Alkyl-SO₂- oder (C₁-C₆)-Alkyl-SO₂-NH- ist;
oder R⁴ und R⁵ zusammen mit dem Stickstoff, an den sie gebunden sind, einen Azetidinyl-, Pyrrolidinyl-, Piperidinyl-, Piperazinyl- oder Morpholinylring bilden;
R⁶ 1 bis 3 Substituenten ist, die unabhängig ausgewählt sind aus der Gruppe bestehend aus -OH, Halogen, C₁-C₆-Alkyl-, C₁-C₆-Alkoxy, C₁-C₆-Alkylthio, -CF₃, -NR⁴R⁵, -CH₂NR⁴R⁵, -NHSO₂R²², -N(SO₂R²²)₂, Phenyl, R³³-Phenyl, NO₂, -CO₂R⁴, -CON(R⁴)₂ 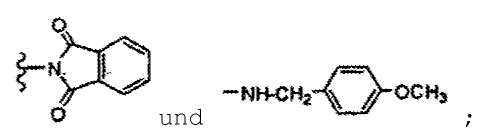
R⁷ -N(R²⁹)-, -O- oder -S(O)₀₋₂ ist;
R¹³ unabhängig ausgewählt ist aus der Gruppe bestehend aus C₁-C₆-Alkyl, Hydroxyl, C₁-C₆-Alkoxy oder Fluor mit der Maßgabe, dass, wenn R¹³ Hydroxy oder Fluor ist, dann ist R¹³ nicht an einen Kohlenstoff neben einem Stickstoff gebunden; oder zwei R¹³-Substituenten eine C₁- bis C₂-Alkylbrücke von einem Ringkohlenstoff zu einem anderen, nicht benachbarten Ringkohlenstoff bilden, oder R¹³ =O ist;
R²⁰ unabhängig ausgewählt ist aus der Gruppe bestehend aus Wasserstoff, C₁-C₆-Alkyl oder Aryl, wobei die Arylgruppe gegebenenfalls mit 1 bis 3 Gruppen substituiert ist, die unabhängig ausgewählt sind aus Halogen, -CF₃, -OCF₃, Hydroxyl oder Methoxy; oder, wenn zwei R²⁰-Gruppen vorhanden sind, die beiden R²⁰-Gruppen zusammen mit dem Stickstoff, an den sie gebunden sind, einen fünf- oder sechsgliedrigen heterocyclischen Ring bilden können;
R²² C₁-C₆-Alkyl, R³⁴-Aryl oder Heterocycloalkyl ist;
R²⁴ H, C₁-C₆-Alkyl, -SO₂R²² oder R³⁴-Aryl ist;
R²⁵ unabhängig ausgewählt ist aus der Gruppe bestehend aus C₁-C₆-Alkyl, Halogen, -CN, -NO₂, -CF₃, -OH, C₁-C₆-Alkoxy, (C₁-C₆)-Alkyl-C(O)-, Aryl-C(O)-, -C(O)OR²⁹, -N(R⁴)(R⁵), N(R⁴)(R⁵)-C(O)-, N (R⁴)(R⁵)-S(O)₁₋₂-, R²²-S(O)₀₋₂-, Halogen- (C₁-C₆)alkyl- oder Halogen-(C₁-C₆)alkoxy-(C₁-C₆)alkyl-;
R²⁹ H, C₁-C₆-Alkyl, C₃-C₆-Cycloalkyl, R³⁵-Aryl oder R³⁵-Aryl-(C₁-C₆)alkyl ist;
R³⁰ H, C₁-C₆-Alkyl, R³⁵-Aryl oder R³⁵-Aryl-(C₁-C₆)alkyl ist;
R³¹ H, (C₁-C₆)-Alkyl-, R³⁵-Aryl, R³⁵-Aryl(C₁-C₆)alkyl-, R³⁵-Heteroaryl, (C₁-C₆)-Alkyl-C(O)-, R³⁵-Aryl-C(O)-, N(R⁴)(R⁵)-C(O)-, (C₁-C₆)-Alkyl-S(O)₂- oder R³⁵-Aryl-S(O)₂- ist;
oder R³⁰ und R³¹ zusammen -(CH₂)₄₋₅-_{,} -(CH₂)₂-O-(CH₂)₂- oder -(CH₂)₂-N(R³⁸)-(CH₂)₂- sind und mit dem Stickstoff, an den sie gebunden sind, einen Ring bilden;
R³² 1 bis 3 Substituenten ist, die unabhängig ausgewählt sind aus der Gruppe bestehend aus H, -OH, Halogen, C₁-C₆-Alkyl, C₁-C₆-Alkoxy, R³⁵-Aryl-O-, -SR²², -CF₃, -OCF₃, -OCHF₂, -NR³⁹R⁴⁰, Phenyl, R³³-Phenyl, -NO₂, -CO₂R³⁹, -CON(R³⁹)₂, -S(O)₂R²², -S(O)₂N(R²⁰)₂, -N(R²⁴)S(O)₂R²², -CN, Hydroxy-(C₁-C₆) alkyl-, -OCH₂CH₂OR²² und R³⁵-Aryl (C₁-C₆) - alkyl-O-, oder zwei R³²-Gruppen an benachbarten Kohlenstoffatomen zusammen eine -OCH₂O- oder -O(CH₂)₂O- Gruppe bilden;
R³³ 1 bis 3 Substituenten ist, die unabhängig ausgewählt sind aus der Gruppe bestehend aus C₁-C₆-Alkyl, Halogen, -CN, -NO₂, -CF₃, -OCF₃, -OCHF₂ und -O-(C₁-C₆)Alkyl;
R³⁴ 1 bis 3 Substituenten ist, die unabhängig ausgewählt sind aus der Gruppe bestehend aus H, Halogen, -CF₃, - OCF₃, -OH and -OCH₃;
R³⁵ 1 bis 3 Substituenten ist, die unabhängig ausgewählt sind aus der Gruppe bestehend aus Wasserstoff, Halogen, C₁-C₆-Alkyl, Hydroxy, C₁-C₆-Alkoxy, Phenoxy, -CF₃, -N(R³⁶)₂, -COOR²⁰ und -NO₂;
R³⁶ unabhängig ausgewählt ist aus der Gruppe bestehend aus H und C₁-C₆-Alkyl;
R³⁷ 1 bis 3 Substituenten ist, die unabhängig ausgewählt sind aus der Gruppe bestehend aus Wasserstoff, Halogen, C₁-C₆-Alkyl, Hydroxy, C₁-C₆-Alkoxy, Phenoxy, -CF₃, -N(R³⁶)₂, -COOR²⁰, -C(O)N(R²⁹)₂ und -NO₂, oder R³⁷ eine oder zwei =0 Gruppen ist;
R³⁸ H, C₁-C₆-Alkyl, R³⁵-Aryl, R³⁵-Aryl(C₁-C₆)alkyl-, (C₁-C₆)-Alkyl-SO₂ oder Halogen(C₁-C₆)alkyl-SO₂- ist;
R³⁹ unabhängig ausgewählt ist aus der Gruppe bestehend aus Wasserstoff, C₁-C₆-Alkyl, C₃-C₆-Cycloalkyl, (C₃-C₆) - Cycloalkyl(C₁-C₆)alkyl, R³³-Aryl, R³³-Aryl(C₁-C₆)alkyl und R³²-Heteroaryl; und
R⁴⁰ Wasserstoff, C₁-C₆-Alkyl, -C(O)R²⁰, -C(O)₂R²⁰, -C(O)N(R²⁰)₂, (C₁-C₆)-Alkyl-SO₂- oder (C₁-C₆)-Alkyl-SO₂-NH- ist;
oder R³⁹ und R⁴⁰ zusammen mit dem Stickstoff, an den sie gebunden sind, einen Azetidinyl-, Pyrrolidinyl-, Piperidinyl-, Piperazinyl- oder Morpholinylring bilden;
Aryl für eine carbocyclische Gruppe steht, die 6 bis 14 Kohlenstoffatome enthält und mindestens einen aromatischen Ring aufweist;
Heteroaryl für eine cyclische Gruppe mit 1 bis 4 Heteroatomen ausgewählt aus 0, S oder N steht, wobei das Heteroatom eine carbocyclische Ringstruktur unterbricht und eine ausreichende Anzahl delokalisierter π-Elektronen aufweist, um aromatischen Charakter zu liefern, wobei die aromatische heterocyclische Gruppe 2 bis 14 Kohlenstoffatome enthält; und
Heterocycloalkyl für eine Gruppe ausgewählt aus 2-Tetrahydrofuranyl, 3-Tetrahydrofuranyl, 2-Tetrahydrothienyl, 3-Tetrahydrothienyl, 2-Piperidinyl, 3-Piperidinyl, 4-Piperidinyl, 2-Pyrrolidinyl, 3-Pyrrolidinyl, 2-Piperazinyl, 3-Piperazinyl, 2-Dioxanyl, 4-Dioxanyl, 1,3-Dioxolanyl, 1,3,5-Trithianyl, Pentamethylensulfid, Perhydroisochinolinyl, Decahydrochinolinyl, Trimethylenoxide, Azetidinyl, 1-Azacycloheptanyl, 1,3-Dithianyl, 1,3,5-Trioxanyl, Morpholinyl, Thiomorpholinyl, 1,4-Thioxanyl, 1,3,5-Hexahydrotriazinyl, Thiazolidinyl und Tetrahydropyranyl steht.

2. Verbindung nach Anspruch 1, bei der R³ Wasserstoff oder Fluor und b 0 ist.

3. Verbindung nach Anspruch 2, bei der X eine Bindung ist.

4. Verbindung nach Anspruch 3, bei der Y -C(O)- ist.

5. Verbindung nach Anspruch 4, bei der Z geradkettiges oder verzweigtes C₁-C₃-Alkyl ist.

6. Verbindung nach Anspruch 5, bei der R² ein sechsgliedriger Heteroarylring ist, der gegebenenfalls mit einem R⁶-Substituenten substituiert ist.

7. Verbindung nach Anspruch 6, bei der R² Pyrimidyl, R⁶-Pyrimidyl, Pyridyl, R⁶-Pyridyl oder Pyridazinyl ist und R⁶ -NH₂ ist.

8. Verbindung nach Anspruch 7, bei der R² 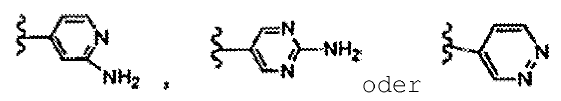 ist.

9. Verbindung nach Anspruch 1, bei der R¹ 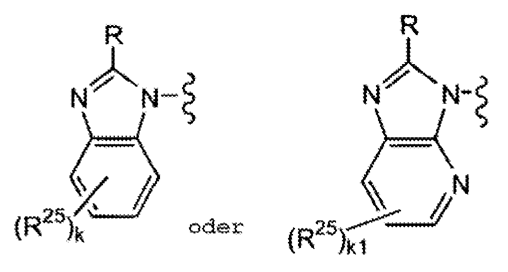 ist.

10. Verbindung nach Anspruch 9, bei der R (C₁-C₆) -Alkyl, (C₁-C₆)-Alkoxy, (C₁-C₆)-Alkoxy(C₁-C₆)alkoxy, (C₁-C₆)-Alkylthio, Heteroaryl oder R³²-Aryl ist; R²⁵ Halogen oder -CF₃ ist sowie k und k1 0 oder 1 ist.

11. Verbindung nach Anspruch 10, bei der R -CH₃, -CH₂CH₃, -OCH₃, -OCH₂CH₃, -OCH₂CH_{Z}CH₃, -OCH((CH₃)₂, -CH₂CH₃, -SCH₃, -SCH₂CH₃, Pyridyl, Pyrimidyl, Pyrazinyl, Furanyl, Oxazolyl oder R³²-Phenyl ist.

12. Verbindung nach Anspruch 11, bei der R² 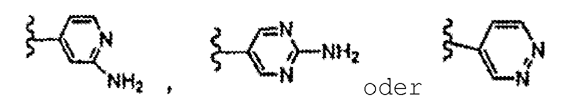 ist.

13. Verbindung nach Anspruch 1 ausgewählt aus der Gruppe bestehend aus 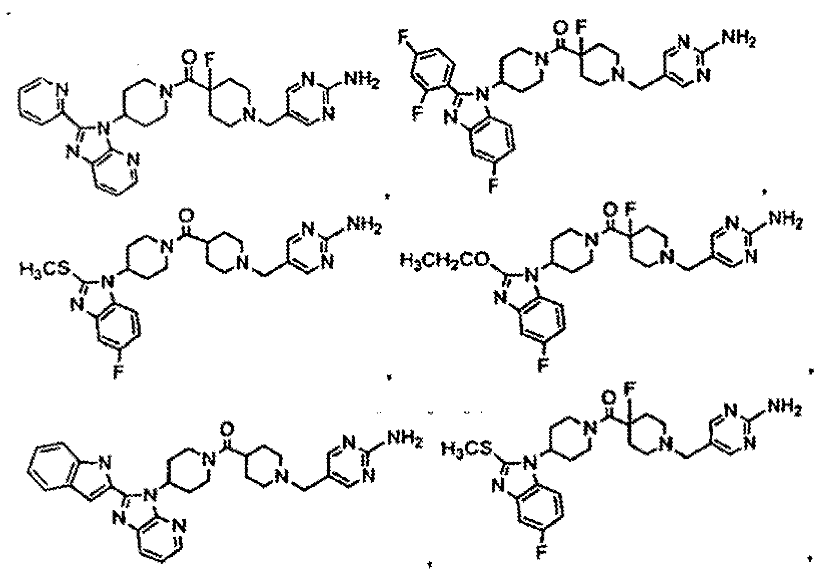 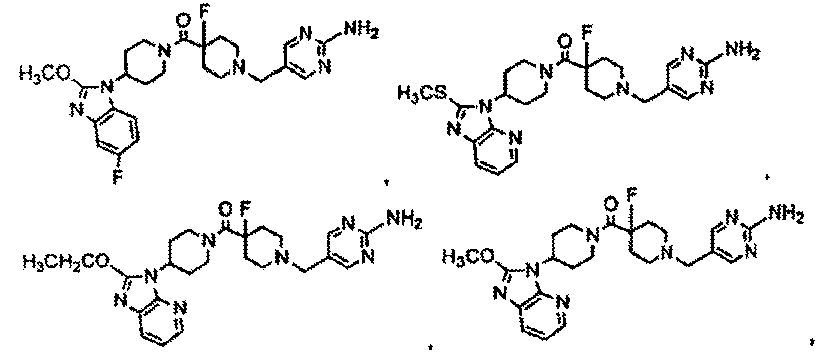 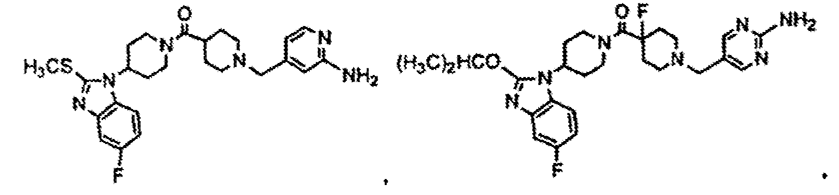 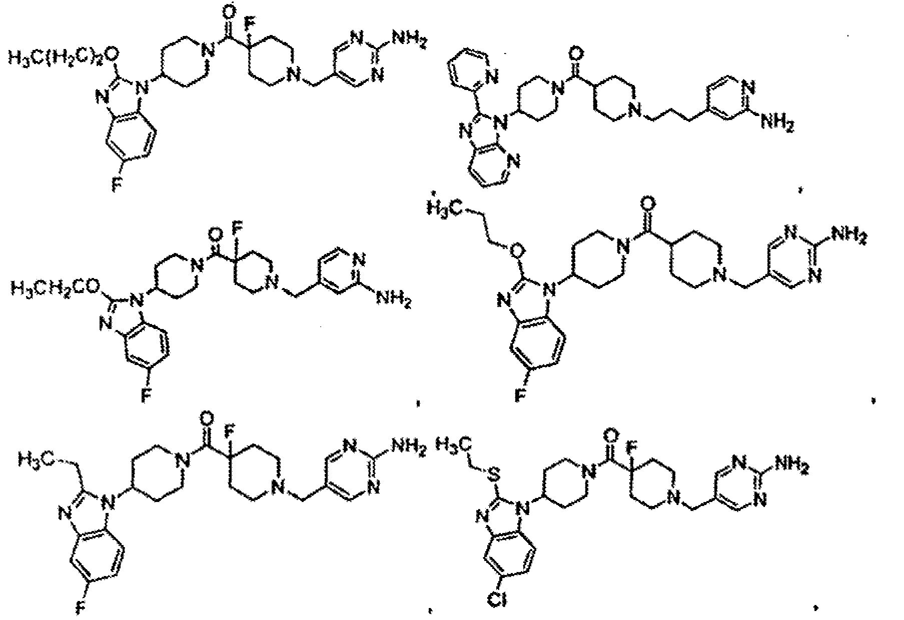 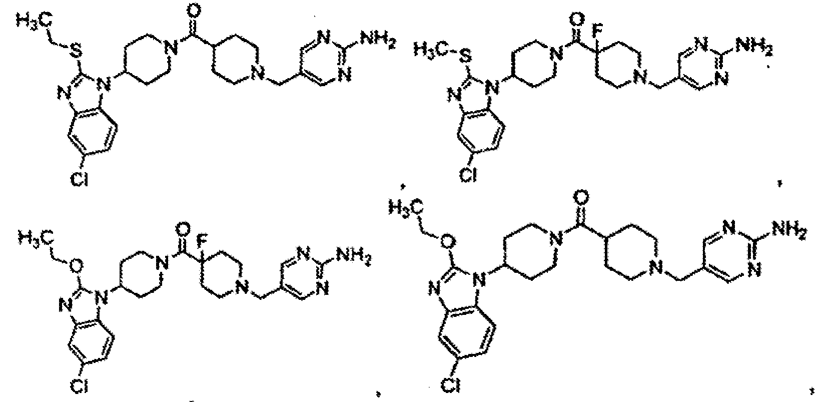 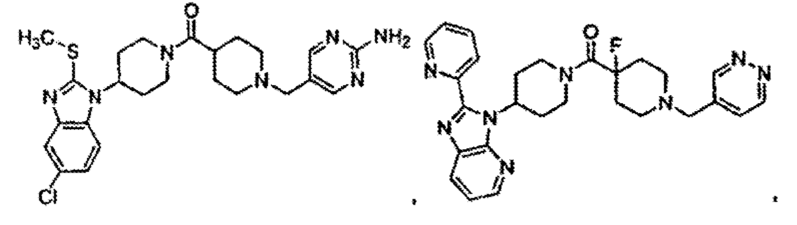 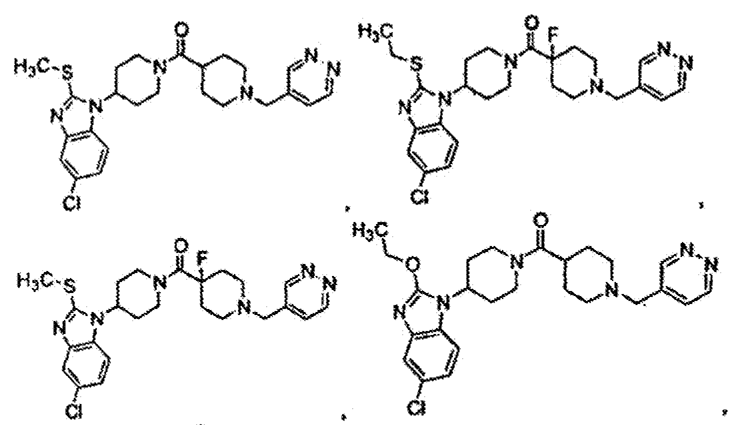 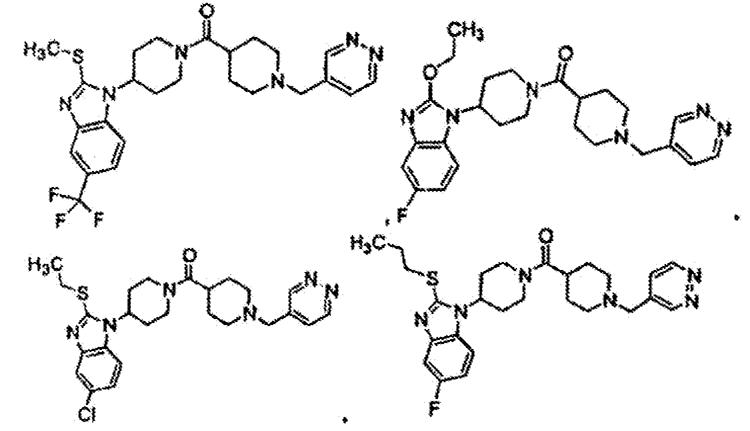 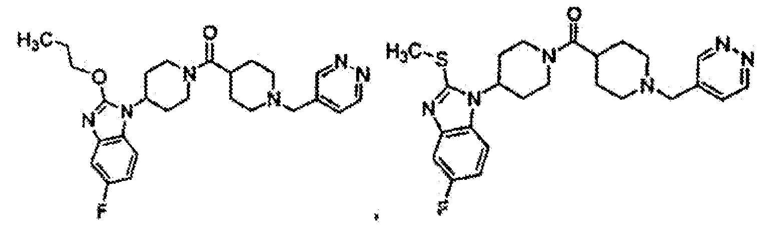 und

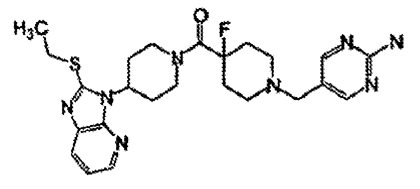

14. Verbindung mit der Struktur: 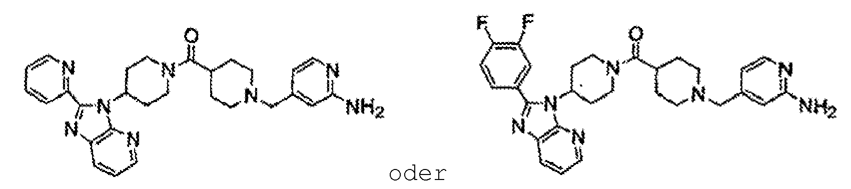 oder ein pharmazeutisch annehmbares Salz oder Solvat davon.

15. Pharmazeutische Zusammensetzung, die eine wirksame Menge einer Verbindung gemäß Anspruch 1 oder Anspruch 14 und einen pharmazeutisch wirksamen Träger enthält.

16. Verwendung einer Verbindung nach Anspruch 1 oder Anspruch 14 zur Herstellung eines Medikaments zur Behandlung von Allergie, allergisch induzierten Reaktionen der Luftwege, Schwellungen, Hypotonie, Herz-Kreislauf-Erkrankung, Erkrankungen des Gastrointestinaltrakts, Hyper- und Hypomotilität und Säuresekretion des Gastrointestinaltrakts, Fettleibigkeit, Schlafstörungen, Störungen des zentralen Nervensystems, Aufmerksamkeits-Wahrnehmungsdefizit-Hyperaktivitäts-Syndrom, Hypo- und Hyperaktivität des zentralen Nervensystems, Morbus Alzheimer, Schizophrenie oder Migräne.

17. Pharmazeutische Zusammensetzung, die eine wirksame Menge einer Verbindung gemäß Anspruch 1 oder Anspruch 14 und eine wirksame Menge eines H₁-Rezeptorantagonisten und einen pharmazeutisch wirksamen Träger enthält.

18. Verwendung nach Anspruch 16, bei der das Medikament in Kombination mit einem H₁-Rezeptorantagonisten verwendet werden soll.

19. Verwendung nach Anspruch 18, bei der das Medikament zur Behandlung von Allergie, allergisch induzierten Reaktionen der Luftwege und Schwellungen dient.

20. Pharmazeutische Zusammensetzung nach Anspruch 17 oder Verwendung nach Anspruch 18, wobei der H₁-Rezeptorantagonist ausgewählt ist aus: Astemizol, Azatadin, Azelastin, Acrivastin, Brompheniramin, Cetirizin, Chlorpheniramin, Clemastin, Cyclizin, Carebastin, Cyproheptadin, Carbinoxamin, Descarboethoxyloratadin, Diphenhydramin, Doxylamin, Dimethinden, Ebastin, Epinastin, Efletirizin, Fexofenadin, Hydroxyzin, Ketotifen, Loratadin, Levocabastin, Meclizin, Mizolastin, Mequitazin, Mianserin, Noberastin, Norastemizol, Picumast, Pyrilamin, Promethazin, Terfenadin, Tripelennamin, Temelastin, Trimeprazin oder Triprolidin.

21. Pharmazeutische Zusammensetzung nach Anspruch 17 oder Verwendung nach Anspruch 18, wobei der H₁-Rezeptorantagonist Loratadin oder Descarboethoxyloratadin ist.

## Revendications

1. Composé représenté par la formule structurale : 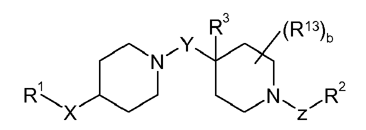 ou sel ou solvat pharmacologiquement admissible d'un tel composé,
dans laquelle formule :
- l'indice b vaut de 0 à 2 ;
- X représente une liaison ou un groupe alkylène en C₁ ;
- Y représente un chaînon symbolisé par -C(O)-, -C(S)-, -CH₂- ou -SO₂- ;
- Z représente une liaison, un groupe alkylène en C₁₋₆ ou alcénylène en C₁₋₆, un chaînon symbolisé par -C(O)-, -CH(CN)- ou -SO₂-, ou un fragment représenté par -CH₂C(O)NR⁴- ;
- R¹ représente un groupe de formule : 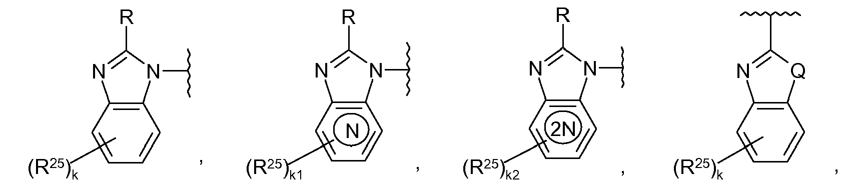 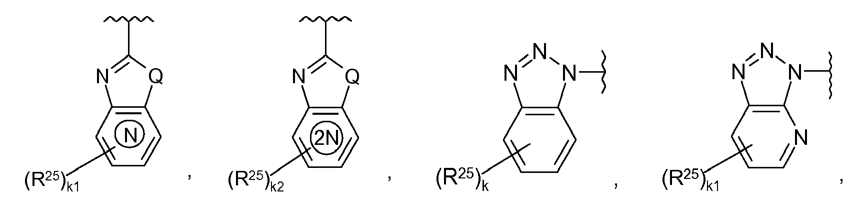 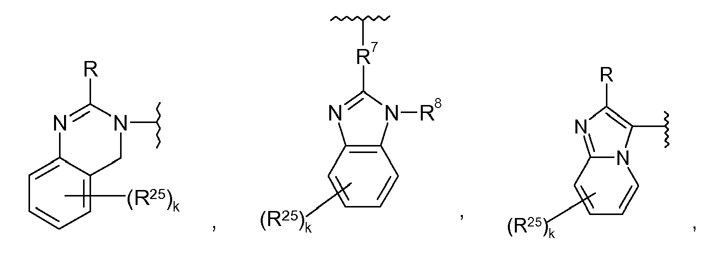 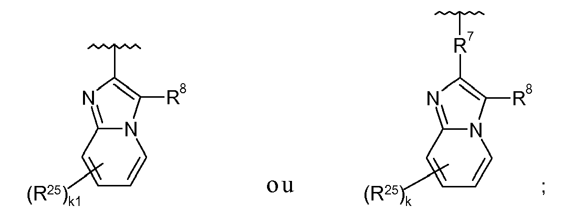
- Q représente un chaînon symbolisé par -N(R⁸)-, -S- ou -O- ;
- l'indice k vaut 0, 1, 2, 3 ou 4 ;
- l'indice k1 vaut 0, 1, 2 ou 3 ;
- l'indice k2 vaut 0, 1 ou 2 ;
- R représente un atome d'hydrogène, ou un groupe alkyle en C₁₋₆, halogénoalkyle en C₁₋₆, alcoxy en C₁₋₆, (alcoxy en C₁₋₆)-alkyle en C₁₋₆, (alcoxy en C₁₋₆)-alcoxy en C₁₋₆, (alcoxy en C₁₋₆)-(alkyle en C₁₋₆)-S(O)₀₂-, R³²-aryl-alcoxy en C₁₋₆, R³²-aryl-alkyle en C₁₋₆, R³²-aryle, R³²-aryloxy, R³²-hétéroaryle, cycloalkyle en C₃₋₆, (cycloalkyle en C₃₋₆)-alkyle en C₁₋₆, (cycloalkyle en C₃₋₆)-alcoxy en C₁₋₆, cycloalkyl-oxy en C₃₋₆, R³⁷-hétéro-cycloalkyle, R³⁷-hétérocycloalkyl-oxy, R³⁷-hétérocycloalkyl-alcoxy
en C₁₋₆, (R³⁰)(R³¹)N-alkyle en C₁₋₆, (R³⁰)(R³¹)N-, (alcoxy en C₁₋₆)-(alkyle en C₁₋₆)-NH-, (R²⁹)NHC(O)NH-, R²⁹-S(O)₀₋₂-, (halogénoalkyle en C₁₋₆)-S(O)₀₋₂-, (R³⁰)(R³¹)N-(alkyle en C₁₋₆)-S(O)₀₋₂-, ou benzoyle ;
- R⁸ représente un atome d'hydrogène, ou un groupe alkyle en C₁₋₆, halogénoalkyle en C₁₋₆, (alcoxy en C₁₋₆)-alkyle en C₁₋₆, R³²-aryl-alkyle en C₁₋₆, R³²-aryle, R³²-hétéroaryle, cycloalkyle en C₃₋₆, (cycloalkyle en C₃₋₆)-alkyle en C₁₋₆, R³⁷-hétérocycloalkyle, (R³⁰)(R³¹)N-alkyle en C₁₋₆, R²⁹-SO₂-, (halogénoalkyle en C₁₋₆)-SO₂-, R²⁹-S(O)₀₋₁-alkyle en C₂₋₆, ou (halogénoalkyle en C₁₋₆)-S(O)₀₋₁-alkyle en C₂₋₆ ;
- R² représente
- un atome d'hydrogène,
- un groupe cyclique hétéroaryle à six chaînons, comportant un ou deux chaînons hétéroatomiques choisis indépendamment parmi un atome d'azote et un groupe N-oxyde, le reste des atomes du cycle étant des atomes de carbone,
- un groupe cyclique hétéroaryle à cinq chaînons, comportant 1, 2, 3 ou 4 chaînons hétéroatomiques choisis indépendamment parmi des atomes d'azote, d'oxygène ou de soufre, le reste des atomes du cycle étant des atomes de carbone,
- un groupe R³²-quinolyle, thianaphtényle, hétérocycloalkyle, cycloalkyle en C₃₋₆, ou alkyle en C₁₋₆,
- ou un groupe de formule 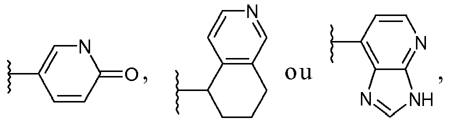 étant entendu que lesdits groupes cycliques hétéroaryle à six chaînons et hétéroaryle à cinq chaînons peuvent porter, en option, un ou des substituants symbolisés par R⁶ ;
- R³ représente un atome d'hydrogène ou d'halogène, ou un groupe alkyle en C₁₋₆, hydroxyle, alcoxy en C₁₋₆ ou (alkyle en C₁₋₆)-sulfonylamino ;
- R⁴ représente une entité choisie indépendamment dans l'ensemble formé par un atome d'hydrogène et les groupes alkyle en C₁₋₆, cycloalkyle en C₃₋₆, (cycloalkyle en C₃₋₆)-alkyle en C₁₋₆, R³³-aryle, R³³-aryl-alkyle en C₁₋₆ et R³²-hétéroaryle ;
- R⁵ représente un atome d'hydrogène, un groupe alkyle en C₁₋₆, (alkyle en C₁₋₆)-sulfonyle ou (alkyle en C₁₋₆)-sulfonylamino, ou un groupe symbolisé par -C(O)R²⁰, -C(O)OR²⁰ ou -C(O)N(R²⁰)₂ ;
- ou R⁴ et R⁵ représentent des entités qui forment, conjointement avec l'atome d'azote auquel elles sont liées, un groupe cyclique azétidinyle, pyrrolidinyle, pipéridinyle, pipérazinyle ou morpholinyle ;
- R⁶ représente 1 à 3 substituants choisis indépendamment dans l'ensemble formé par les atomes d'halogène, les groupes alkyle en C₁₋₆, alcoxy en C₁₋₆, alkylthio en C₁₋₆, phényle et R³³-phényle et les groupes de formule -OH, -CF₃, -NR⁴R⁵, -CH₂-NR⁴R⁵, -NHSO₂R²², -N(SO₂R²²)₂, -NO₂, -CO₂R⁴, -CON(R⁴)₂, 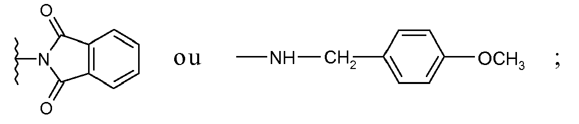
- R⁷ représente un chaînon symbolisé par -N(R²⁹)-, -O- ou -S(O)₀₋₂- ;
- R¹³ représente une entité choisie indépendamment dans l'ensemble formé par un atome de fluor et les groupes alkyle en C₁₋₆, hydroxyle et alcoxy en C₁₋₆, sous réserve que, si R¹³ représente un atome de fluor ou un groupe hydroxyle, ce substituant R¹³ n'est pas lié à un atome de carbone adjacent à un atome d'azote, ou bien deux substituants R¹³ forment un pont alkylène en C₁₋₂ allant d'un atome de carbone du cycle à un autre atome de carbone, non adjacent, du cycle, ou encore R¹³ représente un substituant oxo, de formule =O ;
- R²⁰ représente une entité choisie indépendamment dans l'ensemble formé par un atome d'hydrogène, un groupe alkyle en C₁₋₆ et un groupe aryle, lequel groupe aryle peut, en option, porter 1 à 3 substituants choisis indépendamment parmi les atomes d'halogène et les groupes hydroxyle, méthoxy, trifluorométhyle et trifluorométhoxy, ou bien, s'il y a deux substituants représentés par R²⁰, ces deux substituants R²⁰ peuvent, conjointement avec l'atome d'azote auquel ils sont liés, former un groupe hétérocyclique à cinq ou six chaînons ;
- R²² représente un groupe alkyle en C₁₋₆, R³⁴-aryle ou hétérocycloalkyle ;
- R²⁴ représente un atome d'hydrogène, un groupe alkyle en C₁₋₆ ou R³⁴-aryle, ou un groupe symbolisé par -SO₂R²² ;
- R²⁵ représente une entité choisie indépendamment dans l'ensemble formé par les atomes d'halogène, les groupes de formule -CN, -NO₂, - OH ou -CF₃, les groupes alkyle en C₁₋₆, alcoxy en C₁₋₆, (alkyle en C₁₋₆)-carbonyle et aryl-carbonyle, les groupes symbolisés par -C(O)OR²⁹, - NR⁴R⁵, -C(O)-NR⁴R⁵, -S(O)₁₋₂-NR⁴R⁵ ou -S(O)₀₋₂R²², et les groupes halogénoalkyle en C₁₋₆ et halogéno-(alcoxy en C₁₋₆)-alkyle en C₁₋₆ ;
- R²⁹ représente un atome d'hydrogène ou un groupe alkyle en C₁₋₆, cycloalkyle en C₃₋₆, R³⁵-aryle ou R³⁵-aryl-alkyle en C₁₋₆ ;
- R³⁰ représente un atome d'hydrogène ou un groupe alkyle en C₁₋₆, R³⁵-aryle ou R³⁵-aryl-alkyle en C₁₋₆ ;
- R³¹ représente un atome d'hydrogène, un groupe alkyle en C₁₋₆, R³⁵-aryle, R³⁵-aryl-alkyle en C₁₋₆, R³⁵-hétéroaryle, (alkyle en C₁₋₆)-carbonyle,
R³⁵-aryl-carbonyle, (alkyle en C₁₋₆)-sulfonyle ou R³⁵-aryl-sulfonyle, ou un groupe symbolisé par -C(O)-NR⁴R⁵ ;
- ou R³⁰ et R³¹ représentent ensemble un groupe de formule -(CH₂)₄₋₅-, - (CH₂)₂-O-(CH₂)₂- ou -(CH₂)₂-N(R³⁸)-(CH₂)₂-, qui forme un cycle avec l'atome d'azote auquel il est lié par ses deux extrémités ;
- R³² représente 1 à 3 substituants choisis indépendamment dans l'ensemble formé par les atomes d'hydrogène et d'halogène, les groupes alkyle en C₁₋₆, alcoxy en C₁₋₆, R³⁵-aryl-oxy, phényle, R³³-phényle, hydroxyalkyle en C₁₋₆ et R³⁵-aryl-alcoxy en C₁₋₆, et les groupes de formule - OH, -SR²², -CF₃, -OCF₃, -OCHF₂, -NR³⁹R⁴⁰, -NO₂, -CO₂R³⁹,-C(O)N(R³⁹)₂, -SO₂R²², -SO₂N(R²⁰)₂, -N(R²⁴)SO₂R²², -CN et - OCH₂CH₂OR²², ou deux substituants R³² placés sur des atomes de carbone adjacents forment ensemble un groupe de formule -OCH₂O- ou -O(CH₂)₂O- ;
- R³³ représente 1 à 3 substituants choisis indépendamment dans l'ensemble constitué par les atomes d'halogène, les groupes alkyle en C₁₋₆ et alcoxy en C₁₋₆, et les groupes de formule -CN, -NO₂, -CF₃, -OCF₃ et -OCHF₂ ;
- R³⁴ représente 1 à 3 substituants choisis indépendamment dans l'ensemble constitué par les atomes d'hydrogène et d'halogène et les groupes de formule -CF₃, -OCF₃ -OH et -OCH₃ ;
- R³⁵ représente 1 à 3 substituants choisis indépendamment dans l'ensemble constitué par les atomes d'hydrogène et d'halogène, les groupes alkyle en C₁₋₆, hydroxyle, alcoxy en C₁₋₆ et phénoxy, et les groupes de formule -CF₃, -N(R³⁶)₂, -COOR²⁰ et -NO₂ ;
- R³⁶ représente une entité choisie indépendamment dans l'ensemble formé par un atome d'hydrogène et les groupes alkyle en C₁₋₆ ;
- R³⁷ représente 1 à 3 substituants choisis indépendamment dans l'ensemble formé par les atomes d'hydrogène et d'halogène, les groupes alkyle en C₁₋₆, hydroxyle, alcoxy en C₁₋₆ et phénoxy, et les groupes de formule -CF₃, -N(R³⁶)₂,-COOR²⁰, -C(O)N(R²⁹)₂ ou -NO₂, ou R³⁷ représente 1 ou 2 substituants oxo, de formule =O ;
- R³⁸ représente un atome d'hydrogène ou un groupe alkyle en C₁₋₆, R³⁵-aryle, R³⁵-aryl-alkyle en C₁₋₆, alkylsulfonyle en C₁₋₆ ou halogénoalkylsulfonyle en C₁₋₆ ;
- R³⁹ représente une entité choisie indépendamment dans l'ensemble formé par un atome d'hydrogène et les groupes alkyle en C₁₋₆, cycloalkyle en C₃₋₆, (cycloalkyle en C₃₋₆)-alkyle en C₁₋₆, R³³-aryle, R³³-aryl-alkyle en C₁₋₆ et R³²-hétéroaryle ;
- et R⁴⁰ représente un atome d'hydrogène, un groupe alkyle en C₁₋₆, (alkyle en C₁₋₆)-sulfonyle ou (alkyle en C₁₋₆)-sulfonylamino, ou un groupe sym-bolisé par -C(O)R²⁰, -C(O)OR²⁰ ou -C(O)N(R²⁰)₂ ;
- ou R³⁹ et R⁴⁰ représentent des entités qui forment, conjointement avec l'atome d'azote auquel elles sont liées, un groupe cyclique azétidinyle, pyrrolidinyle, pipéridinyle, pipérazinyle ou morpholinyle ;
étant entendu que :
- le terme "aryle" désigne un groupe carbocyclique comportant de 6 à 14 atomes de carbone et au moins un cycle aromatique ;
- le terme "hétéroaryle" désigne un groupe cyclique comportant de 1 à 4 hétéroatomes choisis parmi des atomes d'oxygène, de soufre et d'azote, lequel ou lesquels hétéroatomes s'insére(nt) dans une structure carbocyclique et apporte(nt) un nombre d'électrons π délocalisés suffisant pour conférer un caractère aromatique audit groupe, lequel groupe hétérocyclique aromatique comporte de 2 à 14 atomes de carbone ;
- et le terme hétérocycloalkyle désigne un groupe choisi parmi les suivants : 2-tétrahydrofuranyle, 3-tétrahydrofuranyle, 2-tétrahydrothiényle, 3-tétrahydrothiényle, 2-pipéridinyle, 3-pipéridinyle, 4-pipéridinyle, 2-pyrrolidinyle, 3-pyrrolidinyle, 2-pipérazinyle, 3-pipérazinyle, 2-dioxanyle, 4-dioxanyle, 1,3-dioxolanyle, 1,3,5-trithianyle, thianyle, perhydroisoquinolyle, décahydroquinolyle, oxétanyle, azétidinyle, 1-azacycloheptanyle, 1,3-dithianyle, 1,3,5-trioxanyle, morpholinyle, thiomorpholinyle, 1,4-thioxanyle, 1,3,5-hexahydrotriazinyle, thiazolidinyle et tétrahydropyranyle.

2. Composé conforme à la revendication 1, dans lequel R³ représente un atome d'hydrogène ou de fluor et l'indice b vaut 0.

3. Composé conforme à la revendication 2, dans lequel X représente une liaison.

4. Composé conforme à la revendication 3, dans lequel Y représente un chaînon -C(O)-.

5. Composé conforme à la revendication 4, dans lequel Z représente un groupe alkyle en C₁₋₃, linéaire ou ramifié.

6. Composé conforme à la revendication 5, dans lequel R² représente un groupe cyclique hétéroaryle à six chaînons, qui peut, en option, porter un substituant unique représenté par R⁶.

7. Composé conforme à la revendication 6, dans lequel R² représente un groupe pyrimidinyle, R⁶-pyrimidinyle, pyridyle, R⁶-pyridyle ou pyrida-zinyle, et R⁶ représente un groupe amino.

8. Composé conforme à la revendication 7, dans lequel R² représente
un groupe de formule

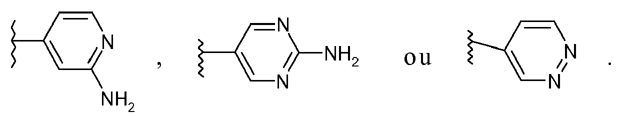

9. Composé conforme à la revendication 1, dans lequel R représente
un groupe de formule

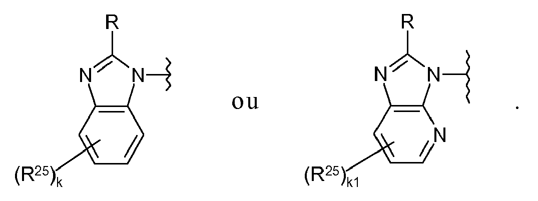

10. Composé conforme à la revendication 9, dans lequel R représente un groupe alkyle en C₁₋₆, alcoxy en C₁₋₆, (alcoxy en C₁₋₆)-alcoxy en C₁₋₆, alkylthio en C₁₋₆, hétéroaryl ou R³²-aryle, R²⁵ représente un atome d'halogène ou un groupe de formule -CF₃, et les indices k et k1 valent chacun 0 ou 1.

11. Composé conforme à la revendication 10, dans lequel R représente un groupe de formule -CH₃, -CH₂CH₃, -OCH₃, -OCH₂CH₃, - OCH₂CH₂CH₃, -OCH(CH₃)₂, -SCH₃ ou -SCH₂CH₃, ou un groupe pyridyle, pyrimidyle, pyrazinyle, furanyle, oxazolyle ou R³²-phényle.

12. Composé conforme à la revendication 11, dans lequel R² représente un groupe de formule

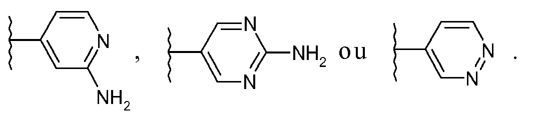

13. Composé conforme à la revendication 1, choisi dans l'ensemble formé par les suivants : 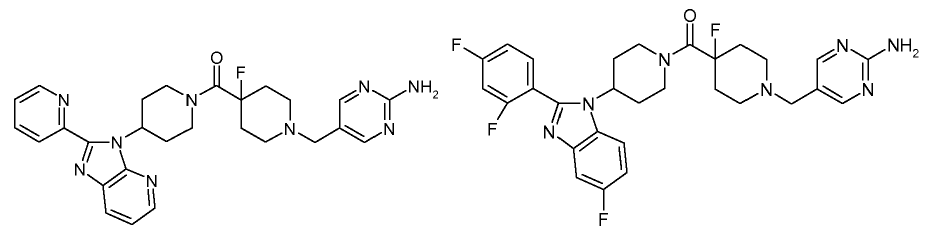 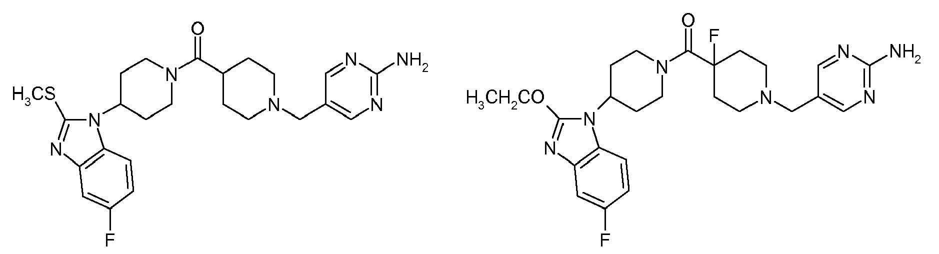 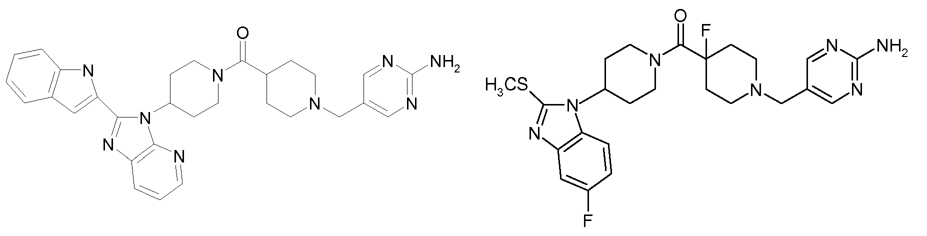 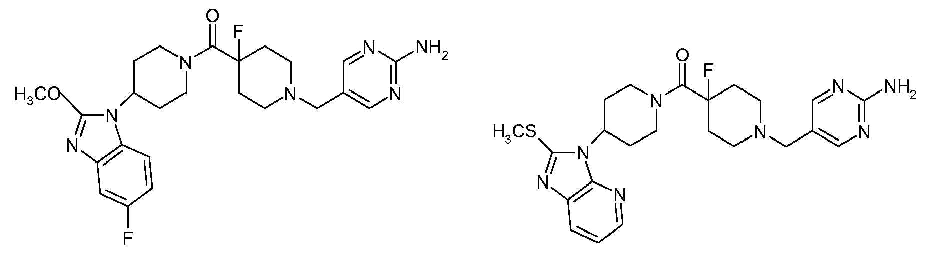 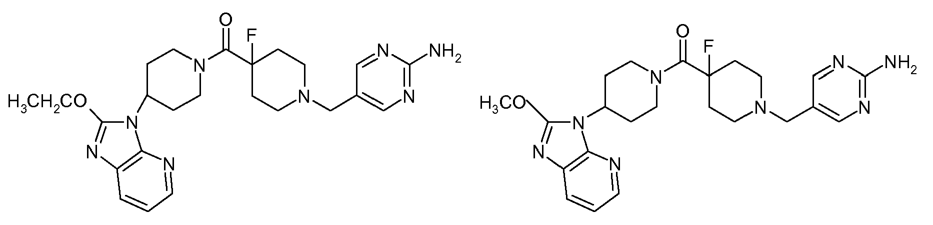 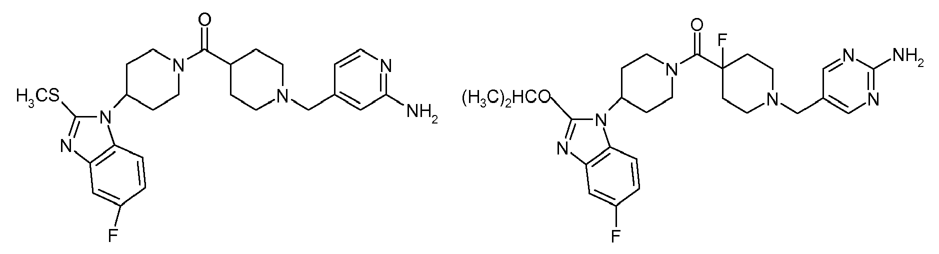 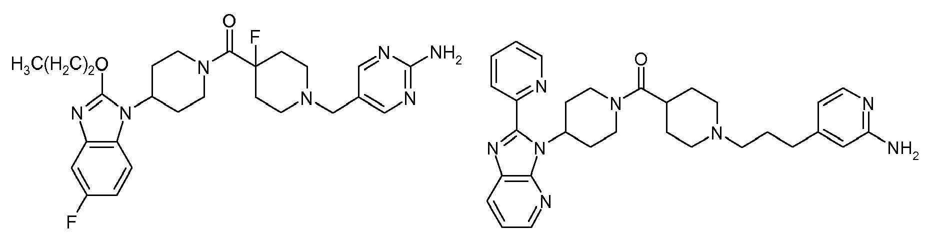 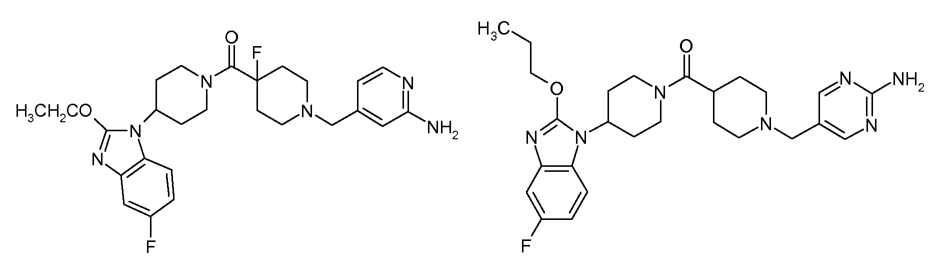 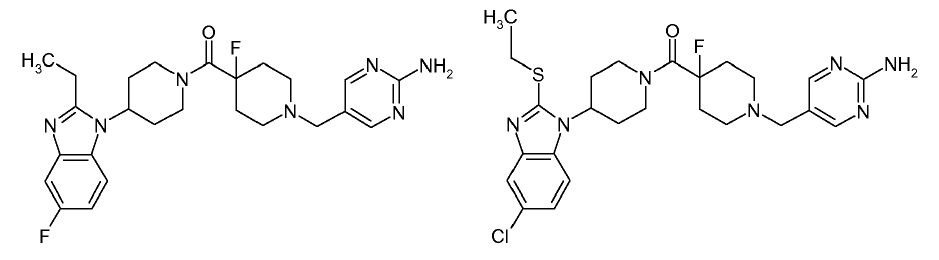 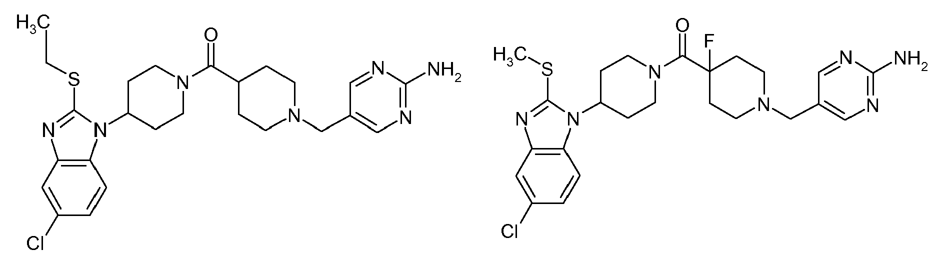 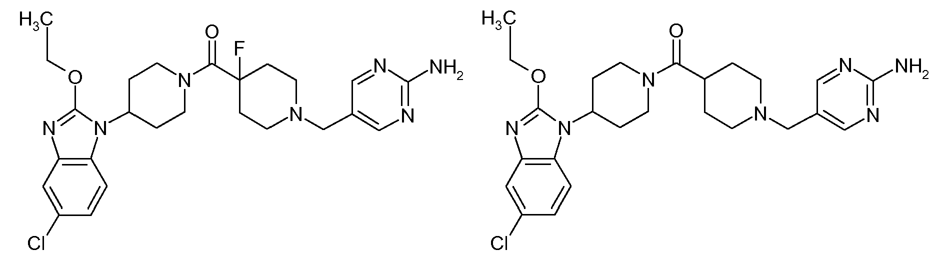 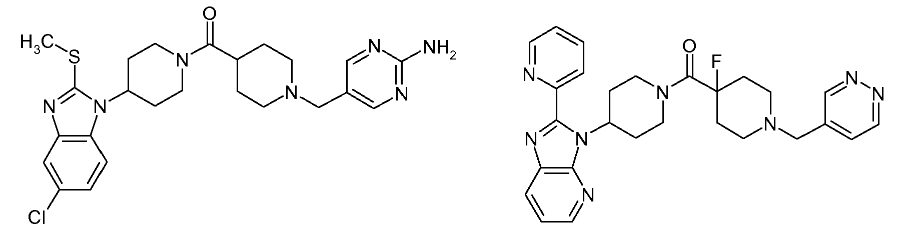 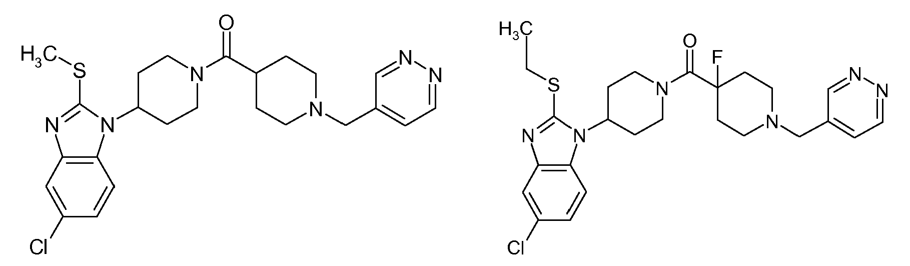 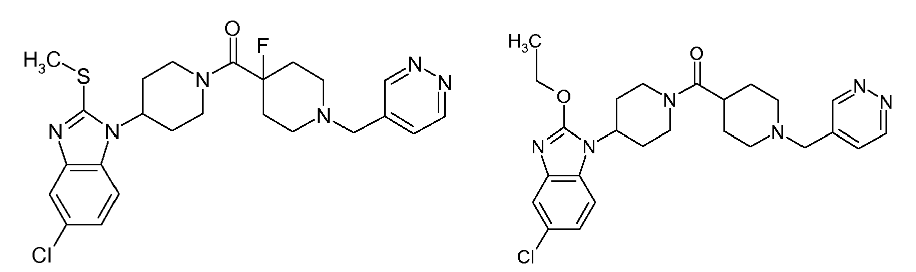 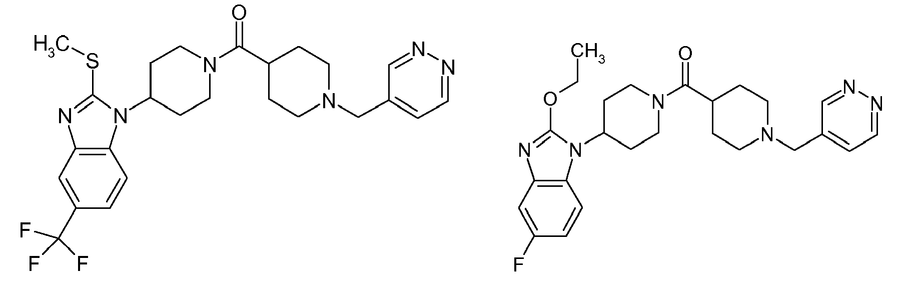 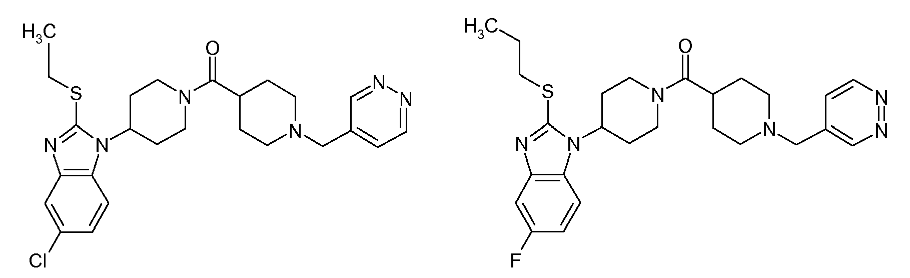 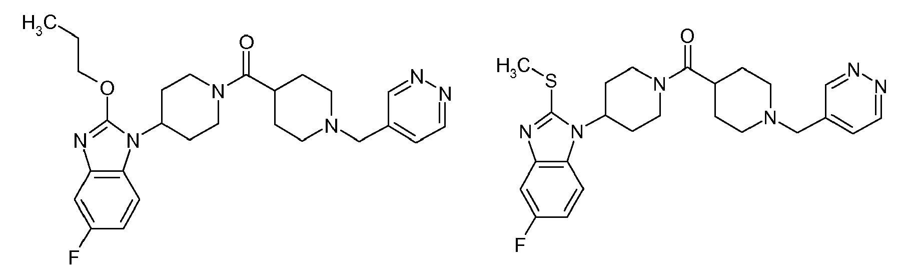 et

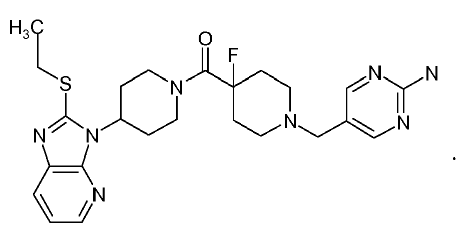

14. Composé présentant la structure : 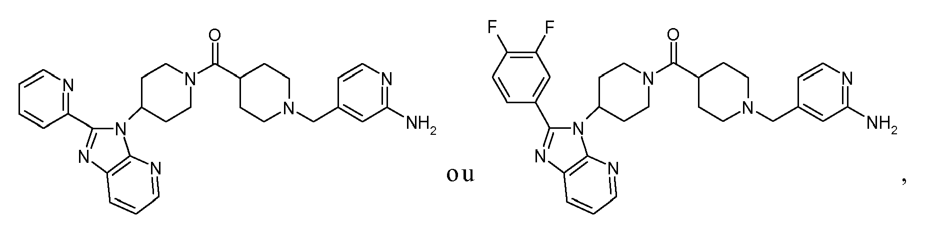 ou sel ou solvat pharmacologiquement admissible d'un tel composé.

15. Composition pharmaceutique comprenant un composé conforme à la revendication 1 ou 14, en quantité efficace, et un véhicule pharmacologiquement efficace.

16. Emploi d'un composé conforme à la revendication 1 ou 14 dans la préparation d'un médicament conçu pour traiter une allergie, des réponses des voies aériennes à une allergie, une congestion, l'hypotension, une maladie cardio-vasculaire, des maladies du tractus gastro-intestinal, une hypermotilité ou une hypomotilité du tractus gastro-intestinal, une hyperacidité ou une hypoacidité des sécrétions du tractus gastro-intestinal, l'obésité, des troubles du sommeil, des troubles du système nerveux central, les troubles d'hyperactivité avec déficit d'attention, une hyperactivité ou une hypoactivité du système nerveux central, la maladie d'Alzheimer, la schizophrénie ou une migraine.

17. Composition pharmaceutique comprenant un composé conforme à la revendication 1 ou 14, en quantité efficace et un antagoniste des récepteurs H₁, en quantité efficace, ainsi qu'un véhicule pharmacologiquement efficace.

18. Emploi conforme à la revendication 16, ledit médicament étant conçu pour être utilisé en association avec un antagoniste des récepteurs H₁.

19. Emploi conforme à la revendication 18, ledit médicament étant conçu pour traiter une allergie, des réponses des voies aériennes à une allergie, ou une congestion.

20. Composition pharmaceutique conforme à la revendication 17, ou emploi conforme à la revendication 18, ledit antagoniste des récepteurs H₁ étant choisi parmi les suivants : astémizole, azatadine, azélastine, acrivastine, bromphéniramine, cétirizine, chlorphéniramine, clémastine, cyclizine, carébastine, cyproheptadine, carbinoxamine, descarboéthoxyloratadine, diphénhydramine, doxylamine, diméthindène, ébastine, épinastine, eflétirizine, fexofénadine, hydroxyzine, kétotifène, loratadine, lévocabastine, méclizine, mizolastine, méquitazine, miansérine, nobérastine, norastémizole, picumast, pyrilamine, prométhazine, terfénadine, tripélennamine, témélastine, triméprazine et tiprolidine.

21. Composition pharmaceutique conforme à la revendication 17, ou emploi conforme à la revendication 18, ledit antagoniste des récepteurs H₁ étant de la loratadine ou de la descarboéthoxyloratadine.
